# EUROPÄISCHE PATENTSCHRIFT

(11) **EP 2 588 457 B1**
(45) Veröffentlichungstag und Bekanntmachung des Hinweises auf die Patenterteilung: **05.11.2014**
(21) Anmeldenummer: 11729377.9
(22) Anmeldetag: 24.06.2011
(51) Int. Cl.: C07D 215/38, C07D 471/04, A61K 31/4745, A61P 35/00

(54) **PYRAZOLOCHINOLINDERIVATE ALS DNA-PK-INHIBITOREN**
PYRAZOLOQUINOLINE DERIVATIVES AS DNA-PK INHIBITORS
DÉRIVÉS DE PYRAZOLOQUINOLINE EN TANT QU'INHIBITEURS DE DNA-PK

(30) Priorität: 01.07.2010 DE 102010025786
(43) Veröffentlichungstag der Anmeldung: 08.05.2013
(73) Patentinhaber: Merck Patent GmbH, 64293 Darmstadt (DE)
(72) Erfinder: FUCHSS, Thomas, 64625 Bensheim-Auerbach (DE); MEDERSKI, Werner, 64673 Zwingenberg (DE); ZENKE, Frank, 64291 Darmstadt (DE)
(86) Internationale Anmeldenummer: PCT/EP2011/003127
(87) Internationale Veröffentlichungsnummer: WO 2012/000632

(56) Entgegenhaltungen:
- WO-A2-2006/122806
- WO-A2-2009/155527

## Beschreibung

Die Erfindung betrifft Verbindungen der Formeln (I), (II) und (III) 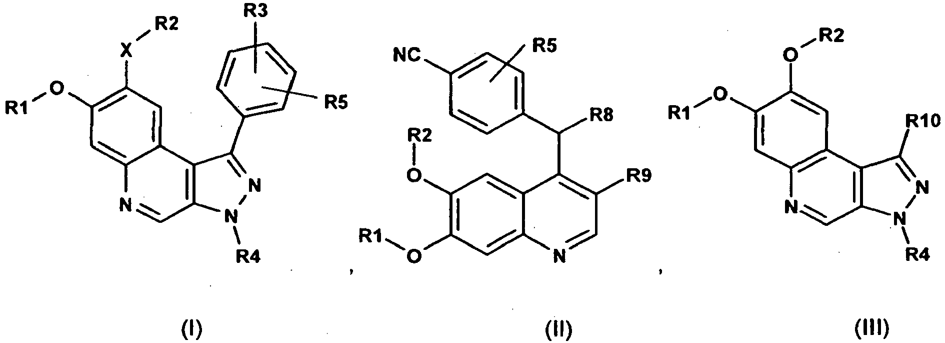 worin R1, R2, R3, R4, R5, R8, R9, R10 und X die in den Ansprüchen angegebene Bedeutung aufweisen, und/oder ihre physiologisch unbedenklichen Salze, Tautomere und Stereoisomere, einschließlich deren Mischungen in allen Verhältnissen. Die Verbindungen der Formel (I) können zur Hemmung von Serin-Threonin-Proteinkinasen sowie zur Sensibilisierung von Krebszellen gegenüber Antikrebsmitteln und/oder ionisierender Strahlung verwendet werden. Gegenstand der Erfindung ist auch die Verwendung der Verbindungen der Formel (I) in der Prophylaxe, Therapie oder Verlaufskontrolle von Krebs, Tumoren, Metastasen oder Störungen der Angiogenese, in Kombination mit Radiotherapie und/oder einem Antikrebsmittel. Die Erfindung betrifft ferner ein Verfahren zum Herstellen der Verbindungen der Formel (I) durch Umsetzen von Verbindungen der Formel (II) oder (III) und gegebenenfalls Umwandeln einer Base oder Säure der Verbindungen der Formel (I) in eines ihrer Salze.

Die DNA-abhängige Proteinkinase (DNA-PK) ist eine Serin/Threonin-Proteinkinase, die in Verbindung mit DNA aktiviert wird. Biochemische und genetische Daten zeigen, dass DNA-PK (a) aus einer katalytischen Untereinheit, die man DNA-PKcs nennt, und (b) zwei regulatorischen Komponenten (Ku70 und Ku80) besteht. Funktional ist DNA-PK ein entscheidender Bestandteil einerseits der Reparatur von DNA-Doppelstrangbrüchen (DSBs) und anderseits der somatischen oder V(D)J Rekombination. Darüber hinaus werden DNA-PK und ihre Komponenten mit einer Vielzahl weiterer physiologischer Prozesse in Verbindung gebracht, darunter die Modulation der Chromatinstruktur sowie die telomere Wartung (Smith & Jackson (1999) Genes and Dev 13: 916; Goytisolo et al. (2001) Mol. Cell. Biol. 21: 3642; Williams et al. (2009) Cancer Res. 69: 2100).

Das menschliche Erbgut in Form der DNA ist fortwährend dem Angriff reaktiver Sauerstoff-Spezies (ROS) ausgesetzt, die vornehmlich als Nebenprodukte des oxidativen Metabolismus anfallen. ROS sind imstande, DNA-Schädigungen in Form von Einzelstrangbrüchen hervorzurufen. Doppelstrangbrüche können entstehen, wenn vorangegangene Einzelstrangbrüche in enger Nachbarschaft erfolgen. Darüber hinaus können Einzel- und Doppelstrangbrüche verursacht werden, wenn die DNA-Replikationsgabel auf beschädigte Basenmuster trifft. Des Weiteren sind körperfremde Einflüsse, wie ionisierende Strahlung (z.B. gamma- oder Schwerionen-Strahlung), und bestimmte Anti-Krebsmedikamente (z.B. Bleomycin) in der Lage, DNA-Doppelstrangbrüche hervorzurufen. DSB können ferner als Zwischenprodukte der somatischen Rekombination auftreten, einem Prozess, der bedeutend ist für die Ausbildung eines funktionalen Immunsystems aller Wirbeltiere. Werden DNA-Doppelstrangbrüche nicht oder fehlerhaft behoben, können Mutationen und/oder Chromosomenaberrationen auftreten, die in der Folge zum Zelltod führen können. Um den schwerwiegenden Gefährdungen, die von DNA-Doppelstrangbrüchen ausgehen, entgegenzuwirken, haben eukaryotische Zellen mehrere Mechanismen entwickelt, diese zu beheben. Höhere Eukaryoten bedienen sich dabei überwiegend der sogenannten nicht-homologen Endverknüpfung (*non-homologous end-joining,* NHEJ), bei der die DNA-abhängige Proteinkinase die Schlüsselrolle einnimmt. Biochemische Untersuchungen haben gezeigt, dass DNA-PK am wirksamsten durch das Auftreten von DNA-DSBs aktiviert wird. Zelllinien, deren DNA-PK Komponenten mutiert und nicht funktional sind, erweisen sich als strahlungsempfindlich (Smith und Jackson, 1999).

DNA-PK gehört wegen Ihrer katalytischen Domäne, die in der etwa 500 Aminosäuren zählenden *C*-terminalen katalytischen Untereinheit (DNA-PKcs) liegt, zur Familie der *Phosphatidylinositol-3-kinase-related kinases (PIKK),* wobei DNA-PK keine Lipid-Kinase darstellt (Hartley et al. (1995) Cell 82: 849; Smith & Jackson (1999) Genes and Dev 13: 916; Lempiäinen & Halazonetis (2009) EMBO J. 28: 3067).

Die Proteinkinase ATM (Ataxia-Telangiectasia-mutierte Kinase) gehört ebenfalls zur PIKK-Familie. Auch sie besitzt eine zentrale Bedeutung bei der Erkennung von DNA-Schäden. Patienten, die an Ataxia-Telangiectasia leiden, zeigen u.a. eine erhöhte Empfindlichkeit gegenüber ionisierender Strahlung. (Lavin & Shiloh (1997) Annu. Rev. Immunol. 15: 177; Rotman & Shiloh (1998) Hum. Mol. Genet. 7: 1555).

Es ist durch Izzard et al. (1999) Cancer Res. 59: 2581 beschrieben, dass der PI3-Kinase Inhibitor LY294002 in in-vitro Versuchen die Funktion von DNA-PK inhibiert. Der IC₅₀-Wert (Konzentration bei der 50 % der Enzymaktivität gehemmt ist) liegt bei wenig wirksamen 1,25 µM (5,0 mM ATP). Obwohl der Nachweis, dass der Inhibitor LY294002 Säugerzellen strahlungsempfindlicher werden lässt, d.h. die Zytotoxizität ionisierender Strahlung verstärkt wird, prinzipiell eine Anwendung bei der Bestrahlungstherapie von z.B. soliden Krebstumoren impliziert, konnte für LY294002 zellulär lediglich eine schwache Empfindlichkeitssteigerung gegenüber ionisierender Bestrahlung nachgewiesen werden (Rosenzweig et al. (1999) Clin. Cancer Res. 3: 1149). KuDOS Pharmaceuticals Ltd. haben die Leitstruktur LY294002 optimiert und verschiedene DNA-PK-Inhibitoren vorgestellt. Die Einführung einer Dibenzothiophenylgruppe führte zum Inhibitor NU-7441, einer ATPkompetitiven Verbindung mit einem IC₅₀-Wert von 20.0 nM (Hardcastle et al. (2005) J. Med. Chem. 48: 7829). KU-0060648 vereint inhibitorische Eigenschaften gegenüber DNA-PK mit einem verbesserten Löslichkeitsprofil im wässrigen Medium, jedoch werden die Kinasen der PI3K-lsoenzym-Familie durch KU-0060648 ebenfalls potent gehemmt. Das seit Langem bestehende Bedürfnis nach einem potenten und selektiven DNA-PK-Inhibitor ist folglich bisher nicht befriedigt worden.

Der Erfindung liegt die Aufgabe zugrunde, die im Stand der Technik aufgezeigten Nachteile zu überwinden und wirksame Inhibitoren der DNA-PK zu entwickeln, die selektiv gegenüber den verwandten Kinasen der PIKK Familie sowie von niedermolekularer Größe sind und insbesondere eine effektive Anwendung in der Krebstherapie als Radio- und Chemosensibilisierer ermöglichen - mit dem Ziel, die therapeutische Wirksamkeit bei gleichzeitiger Verringerung der Nebenwirkungen zu verbessern.

Die Aufgabe der Erfindung wird gemäß den unabhängigen Ansprüchen gelöst. Die Unteransprüche beinhalten bevorzugte Ausführungsformen. Erfindungsgemäß werden Verbindungen der Formel (I) bereitgestellt 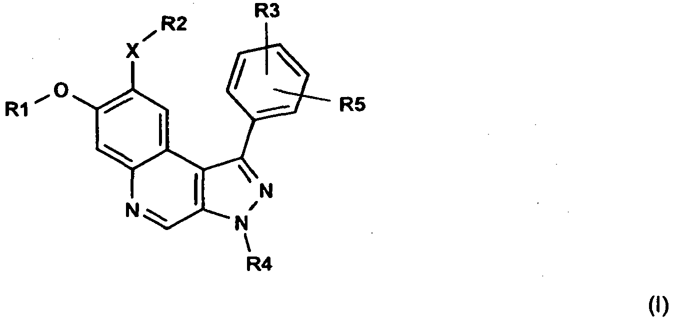 worin
- R1: Y, -Alk-OY, -Alk-NYY oder -Alk-Ar,
- R2: Y, -Alk-OY, -Alk-NYY, -C(Y)(R6)(R7), -C(Hal)(R6)(R7), -SO₂A, -SO₂-A oder -POOH-Ar,
- R3: H, Hal, CN, -Alk-CN, -Alk-NYY, Het¹ oder Het²,
- R4: Hal, Y, Cyc, CN, -Alk-CN, -Alk-COOY, -Alk-CO-NYY oder Het¹,
- R5: Hal, Y, OY, NYY, -NY-COY, COOY, -CO-NYY, -CO-NY-Alk-OY, -Alk-CO-NYY, -Alk-OY, -Alk-NYY, Ar, Het¹ oder Het²,
- R3, R5: zusammen auch -Alk-CO-NY-,
- R6: Hal, Y, -COOY, -CO-NYY, -CO-NY-OY, -CO-NY-C(=NH)-NYY, -CO-NY-Alk-OY, -CO-NY-Alk-NYY, -CO-NY-Alk-SO₂-NYY, -CO-NY-Alk-Ar, -CO-NY-Alk-Het² oder -CO-NY-O-Alk-CN,
- R7: Ar, Het¹ oder-Het¹-Het¹,
- X: CH₂, O, S oder Het¹,
- Y: H oder A,
- A: unverzweigtes oder verzweigtes Alkyl mit 1-10 C-Atomen, wobei unabhängig voneinander 1-7 H-Atome durch Hal und/oder unabhängig voneinander ein oder zwei benachbarte CH₂-Gruppen durch eine -CH=CH- und/oder -C≡C-Gruppe ersetzt sein können,
- Alk: Alkylen mit 1-6 C-Atomen, wobei unabhängig voneinander 1-4 H-Atome durch Hal und/oder OY ersetzt sein können,
- Cyc: zyklisches Alkyl mit 3-7 C-Atomen, wobei unabhängig voneinander 1-4 H-Atome durch Hal und/oder OY ersetzt sein können,
- Ar: unsubstituiertes oder einfach durch Hal, A, CN, OY, NYY, -NY-COY, COOY, Het¹, Het², -Alk-OY, -Alk-NYY, -Alk-Het¹ oder Alk-Het² substituiertes Phenyl,
- Het¹: ein- oder zweikerniges Heteroaryl mit 2-9 C-Atomen und 1-4 N-, O- und/oder S-Atomen, das unsubstituiert oder einfach durch Hal, A, CN, OY, NYY, -NY-COY, COOY, -Alk-OY oder -Alk-NYY substituiert sein kann,
- Het²: einen einkernigen gesättigten Heterozyklus mit 2-7 C-Atomen und 1-4 N-, O- und/oder S-Atomen, der unsubstituiert oder einfach durch A substituiert sein kann, und
- Hal: F, Cl, Br oder I bedeuten,
und/oder ihre physiologisch unbedenklichen Salze, Tautomere und/oder Stereoisomere, einschließlich deren Mischungen in allen Verhältnissen.

Überraschenderweise hat sich gezeigt, dass die erfindungsgemäßen Verbindungen mit inhibierenden Eigenschaften auf Serin-Threonin-Proteinkinasen versehen sind. Die Verbindungen der Formel (I) sind durch ihre Kernstruktur des Pyrazolochinolins, dem mindestens eine Alkoxy-Substitution, vorzugsweise zwei Alkoxy-Substitutionen, sowie ein optional substituiertes Phenyl anhaften, derart ausgestaltet, dass eine potente und selektive Hemmung von DNA-PK erfolgt. Die erfindungsgemäßen Verbindungen eröffnen damit völlig neue Möglichkeiten hinsichtlich der anticancerogenen Wirkung von Antikrebsmitteln. Bemerkenswerterweise spielen die Verbindungen der Formel (I) eine therapeutische Rolle als Radio- und Chemosensibilisierer in der Behandlung von Krebs.

Es ist bisher lediglich aus WO 1992/07844 bekannt, dass 2,4-Diaminochinazolin-Derivate Verstärker von chemotherapeutischen Mitteln in der Krebsbehandlung sind. Die Derivate adressieren die Mehrfachresistenz von Tumorzellen infolge von Überexpression des mdr1-Gens, dessen Genprodukt einer Efflux-P-Glycoproteinpumpe die intrazelluläre Wirkstoffkonzentration gering hält. Generisch sind auch Inhibitoren der Phosphatidylinositol-3-Kinase in WO 2009/155527 beschrieben, die weder die konkretisierte Struktur gemäß der erfindungsgemäßen Formel (I) noch die Alkoxy-Substitution aufweisen. Keines der beiden Dokumente des Stands der Technik offenbart physiko-chemische und pharmakologische Daten. Ebenso wenig ist ein vermarktetes Medikament bekannt. Im Gegensatz dazu enthüllt die vorliegende Erfindung, dass gerade Verbindungen der Formel (I) zur spezifischen Hemmung von Serin-Threonin-Proteinkinasen wie DNA-PK befähigt sind. Die erfindungsgemäßen Verbindungen und ihre Salze besitzen folglich wertvolle pharmakologische Eigenschaften bei gleichzeitig guter Verträglichkeit.

Im Rahmen der Erfindung sind die Verbindungen der Formel (I) so definiert, dass man darunter auch pharmazeutisch verwendbare Derivate, Salze, Hydrate, Solvate, Vorstufen der Verbindungen, Tautomere und optisch aktive Formen (wie z.B. Stereoisomere, Diastereomere, Enantiomere, Racemate) versteht. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Unter pharmazeutisch verwendbaren Derivaten versteht man z.B. die Salze der erfindungsgemäßen Verbindungen als auch sogenannte Vorstufen der Verbindungen. Unter Vorstufen versteht man z.B. mit Alkyl- oder Acylgruppen, Zuckern oder Oligopeptiden abgewandelte Verbindungen der Formel (I), die im Organismus rasch zu den wirksamen erfindungsgemäßen Verbindungen gespalten werden. Hierzu gehören auch bioabbaubare Polymerderivate der erfindungsgemäßen Verbindungen, wie dies z.B. in Int. J. Pharm. 115, 61-67 (1995) beschrieben ist. Jegliche Verbindung, die in-vivo in ein bioaktives Mittel, d.h. Verbindungen der Formel (I) umgewandelt werden kann, ist eine Vorstufe im Sinne dieser Erfindung. Jegliche biologisch aktive Verbindung, die aus der in-vivo Verstoffwechslung einer erfindungsgemäßen Verbindung resultiert, ist ein Metabolit im Sinne der vorliegenden Erfindung. Die Verbindungen der Formel (I) können ein oder mehrere chirale Zentren besitzen und daher in verschiedenen stereoisomeren Formen vorkommen. Die Formel (I) schließt alle diese Formen ein.

Gegenstand der Erfindung ist auch die Verwendung von Mischungen der Verbindungen der Formel (I), z.B. Gemische zweier Diastereomerer, z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Vor- und nachstehend haben die Radikale R1, R2, R3, R4, R5, R6, R7, X, Y, A, Alk, Cyc, Ar, Het¹, Het² und Hal die bei der Formel (I) angegebenen Bedeutungen, sofern nicht ausdrücklich etwas anderes angegeben ist. Bei mehrfachem Auftreten einzelner Reste innerhalb einer Verbindung oder eines Radikals nehmen die Reste unabhängig voneinander die angegebenen Bedeutungen an, sofern nicht ausdrücklich etwas anderes angegeben ist. Beispielsweise sind die Reste YY im Radikal R1, in dem sie mehrfach vorkommen, identisch oder verschieden, aber vorzugsweise in jedem Fall unabhängig voneinander unter den vorstehend und/oder nachstehend angegebenen Bedeutungen ausgewählt (z.B. Methyl und/oder Ethyl), sofern nicht ausdrücklich etwas anderes angegeben ist. Den vorliegend verwendeten Bezeichnungen zur Definition der Verbindungen liegen im Allgemeinen die Regeln der IUPAC-Organisation für chemische Verbindungen und insbesondere organische Verbindungen zugrunde. Die Bezeichnungen zur Erläuterung der o.g. Verbindungen der Erfindung haben immer die nachstehenden Bedeutungen, sofern die Beschreibung oder die Ansprüche nicht etwas anderes anzeigen.

Der Begriff "unsubstituiert" bedeutet, dass ein Radikal, eine Gruppe oder eine Rest keine Substituenten trägt. Der Begriff "substituiert" bedeutet, dass ein Radikal, eine Gruppe oder ein Rest einen oder mehrere Substituenten trägt.

"Alkyl" oder "A" bezeichnet im Sinne der Erfindung ein nicht-zyklisches, gesättigtes oder ungesättigtes Kohlenwasserstoffradikal, das unverzweigt (linear) oder verzweigt ist und vorzugsweise 1, 2, 3, 4, 5, 6, 7, 8, 9 oder 10 C-Atome besitzt, d.h. C₁₋₁₀-Alkanyl. Beispiele für Alkylradikale sind Methyl, Ethyl, Propyl, Isopropyl, 1,1-, 1,2- oder 2,2-Dimethylpropyl, 1-Ethylpropyl, 1-Ethyl-1-methylpropyl, 1-Ethyl-2-methylpropyl, 1,1,2-oder 1,2,2-Trimethylpropyl, Butyl, Isobutyl, sek.-Butyl, tert.-Butyl, 1-, 2- oder 3-Methylbutyl, 1,1-, 1,2-, 1,3-, 2,2-, 2,3- oder 3,3-Dimethylbutyl, 1- oder 2-Ethylbutyl, Pentyl, Isopentyl, Neopentyl, tert.-Pentyl, 1-, 2-, 3- oder 4-Methylpentyl, Hexyl.

In einer bevorzugten Ausführungsform der Erfindung ist "A" unverzweigtes oder verzweigtes Alkyl mit 1-10 C-Atomen, wobei unabhängig voneinander 1-7 H-Atome durch Hal und/oder unabhängig voneinander ein oder zwei benachbarte CH₂-Gruppen durch eine -CH=CH- und/oder -CΞC- Gruppe ersetzt sein können. Besonders bevorzugt als "A" ist unverzweigtes oder verzweigtes Alkyl mit 1-6 C-Atomen, wobei unabhängig voneinander 1-5 H-Atome durch Hal ersetzt sein können. Ganz besonders bevorzugt ist C₁₋₄-Alkyl, wobei unabhängig voneinander 1-3 H-Atome durch Hal ersetzt sein können. Ein solches C₁₋₄-Alkyl ist beispielsweise Methyl, Ethyl, Propyl, Isopropyl, Butyl, Isobutyl, sek.-Butyl, tert.-Butyl, Fluormethyl, Difluormethyl, Trifluormethyl, Pentafluorethyl, 1,1,1-Trifluorethyl oder Brommethyl, höchst bevorzugt Methyl, Ethyl oder Difluormethyl. Es versteht sich, dass die jeweiligen Bedeutungen von "A" unabhängig voneinander in den Radikalen einer erfindungsgemäßen Formel sind.

"Cycloalkyl" oder "Cyc" bezeichnet im Sinne der Erfindung gesättigte und teilweise ungesättigte nicht-aromatische zyklische Kohlenwasserstoffgruppen mit 1 bis 3 Ringen, die 3 bis 20, vorzugsweise 3 bis 12, besonders bevorzugt 3 bis 9 C-Atome umfassen. Die Bindung an das Grundgerüst der Formel (I) kann über jedes Ringmitglied der Cycloalkylgruppe erfolgen. Beispiele für geeignetes Cycloalkyl sind Cyclopropyl, Cyclobutyl, Cyclopentyl, Cyclohexyl, Cycloheptyl, Cyclooctyl, Cyclodecyl, Cyclopentenyl, Cyclohexenyl und Cyclooctadienyl.

In einer bevorzugten Ausführungsform der Erfindung ist "Cyc" zyklisches Alkyl mit 3-7 C-Atomen, wobei unabhängig voneinander 1-4 H-Atome durch Hal und/oder OY ersetzt sein können. Besonders bevorzugt ist zyklisches Alkyl mit 3-5 C-Atomen.

Grundgerüst der Formel (I) ist hierbei jede generische oder nicht-generische Struktur, an die irgendein Radikal im Sinne der Erfindung, wie z.B. Cyc, Ar, Het¹ oder Het², gebunden werden kann, um zu einer erfindungsgemäßen Verbindung der Formel (I) zu gelangen.

Der Begriff "Alk" bezeichnet im Sinne der Erfindung unverzweigtes oder verzweigtes Alkylen, Alkenyl oder Alkynyl mit 1, 2, 3, 4, 5 oder 6 C-Atomen, d.h. C₁₋₆-Alkylene, C₂₋₆-Alkenyle und C₂₋₆-Alkynyle. Alkenyle haben mindestens eine C-C Doppelbindung and Alkynyle mindestens eine C-C Dreifachbindung. Alkynyle können außerdem mindestens eine C-C Doppelbindung aufweisen. Beispiele für geeignete Alkylene sind Methylen, Ethylen, Propylen, Butylen, Pentylen, Hexylen, Isopropylen, Isobutylen, sek.-Butylen, 1-, 2- oder 3-Methylbutylen, 1,1-, 1,2- oder 2,2-Dimethylpropylen, 1-Ethylpropylen, 1-, 2-, 3- oder 4-Methylpentylen, 1,1-, 1,2-, 1,3-, 2,2-, 2,3- oder 3,3-Dimethylbutylen, 1- oder 2-Ethylbutylen, 1-Ethyl-1-methylpropylen, 1-Ethyl-2-methylpropylen, 1,1,2- oder 1,2,2-Trimethylpropylen. Beispiele für geeignete Alkenyle sind Allyl, Vinyl, Propenyl (-CH₂CH=CH₂; -CH=CH-CH₃; -C(=CH₂)-CH₃), 1-, 2- oder 3-Butenyl, Isobutenyl, 2-Methyl-1- oder 2-Butenyl, 3-Methyl-1-butenyl, 1,3-Butadienyl, 2-Methyl-1,3-butadienyl, 2,3-Dimethyl-1,3-butadienyl, 1-, 2-, 3- oder 4-Pentenyl and Hexenyl. Beispiele für geeignete Alkynyle sind Ethynyl, Propynyl (-CH₂-CΞCH; -CΞC-CH₃), 1-, 2- oder 3-Butynyl, Pentynyl, Hexynyl oder Pent-3-en-1-in-yl, insbesondere Propynyl.

In einer bevorzugten Ausführungsform der Erfindung ist "Alk" Alkylen mit 1-6 C-Atomen, d.h. Methylen, Ethylen, Propylen, Butylen, Pentylen oder Hexylen, wobei unabhängig voneinander 1-4 H-Atome durch Hal und/oder OY ersetzt sein können. Es ist besonders bevorzugt, dass "Alk" Alkylen mit 1-3 C-Atomen, wobei 1-2 H-Atome durch Hal und/oder OH ersetzt sein können, bedeutet. Besondere Beispiele hierfür sind Methylen, Ethylen und Propylen. Es versteht sich, dass die jeweiligen Bedeutungen von "Alk" unabhängig voneinander in den Radikalen einer erfindungsgemäßen Formel sind.

Der Begriff "Aryl", "Carboaryl" oder "Ar" bezeichnet im Sinne der Erfindung ein mono- oder polyzyklisches aromatisches Kohlenwasserstoffsystem mit 3 bis 14, vorzugsweise 4 bis 10, besonders bevorzugt 5 bis 8 C-Atomen, die gegebenenfalls substituiert sein können. Der Begriff "Aryl" schließt solche Systeme ein, in denen der aromatische Zyklus Teil eines bi- oder polyzyklischen gesättigten, teilweise ungesättigten und/oder aromatischen Systems ist, z.B. wenn der aromatische Zyklus an "Aryl", "Cycloalkyl", "Heteroaryl" oder "Heterocyclyl" über ein beliebiges Ringmitglied des Arylradikals fusioniert ist. Die Bindung an das Grundgerüst der Formel (I) kann über jedes Ringmitglied der Arylgruppe erfolgen. Beispiele für geeignetes "Aryl" sind Phenyl, Biphenyl, Naphthyl, 1-Naphthyl, 2-Naphthyl, Anthracenyl, In-danyl, In-denyl, 1,2,3,4-Tetrahydronaphthyl, insbesondere Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p- Hydroxyphenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Methylaminosulfonylphenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m- oder p-Acetylphenyl, o-, m- oder p-Formylphenyl, o-, m- oder p-Cyanphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4-bromphenyl oder 2,5-Dimethyl-4-chlorphenyl.

In einer bevorzugten Ausführungsform der Erfindung ist "Ar" unsubstituiertes oder einfach durch Hal, A, CN, OY, NYY, -NY-COY, COOY, Het¹, Het², -Alk-OY, -Alk-NYY, -Alk-Het¹ oder Alk-Het² substituiertes Phenyl. Es ist besonders bevorzugt, dass "Ar" unsubstituiertes oder einfach durch Hal substituiertes Phenyl bedeutet.

Der Begriff "Heteroaryl" bezeichnet im Sinne der Erfindung ein 2 bis 15, vorzugsweise 2 bis 9, besonders bevorzugt 5-, 6- oder 7-gliedriges mono- oder polyzyklisches aromatisches Kohlenwasserstoffradikal, das mindestens 1, wenn passend auch 2, 3, 4 oder 5 Heteroatome umfasst, insbesondere Stickstoff, Sauerstoff und/oder Schwefel, wobei die Heteroatome identisch oder verschieden sind. Die Anzahl der Stickstoffatome ist vorzugsweise 0, 1, 2, 3 oder 4, und die der Sauerstoff- und Schwefelatome ist unabhängig voneinander 0 oder 1. Der Begriff "Heteroaryl" schließt solche Systeme ein, in denen der aromatische Zyklus Teil eines bi- oder polyzyklischen gesättigten, teilweise ungesättigten und/oder aromatischen Systems ist, z.B. wenn der aromatische Zyklus an "Aryl", "Cycloalkyl", "Heteroaryl" oder "Heterocyclyl" über ein beliebiges Ringmitglied des Heteroarylradikals fusioniert ist. Die Bindung an das Grundgerüst der Formel (I) kann über jedes Ringmitglied der Heteroarylgruppe erfolgen, sofern es chemisch sinnvoll erscheint, wobei eine Bindung über die C-Atome bevorzugt ist.

"Heteroaryl" bedeutet, ungeachtet weiterer Substitutionen, z.B. 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-pyrimidinyl, 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]-oxazinyl, 1,3-Benzodioxol-5-yl, 1,4-Benzodioxan-6-yl, 2,1,3-Benzothiadiazol-4- oder -5-yl, 2,1,3-Benzoxadiazol-5-yl, Imidazolyl, Triazinyl, Phthalazinyl, Indolizinyl, Pteridinyl, Carbazolyl, Phenazinyl, Phenoxazinyl, Phenothiazinyl oder Acridinyl.

Die heterozyklischen Reste können auch teilweise oder vollständig hydriert sein. Unsubstituiertes Heteroaryl kann also z.B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1 -, -2-, -3-, -4- oder -5-pyrrolyl, 2,5-Dihydro-1-, -2-, -3-, -4-oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1 ,2,3,4-Tetrahydro-1-, -2-, -3-, -4-, -5- oder -6-pyridyl, 1-, 2-, 3-oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8-3,4-Dihydro-2H-benzo[1,4]oxazinyl, 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylen-dioxy)phenyl, oder auch 3,4-Dihydro-2H-1,5-benzodioxepin-6- oder -7-yl, 2,3-Dihydro-benzofuranyl oder 2,3-Dihydro-2-oxo-furanyl.

Es ist bevorzugt, dass "Heteroaryl" im Rahmen von "Het¹" einen ein- oder zweikernigen aromatischen Heterozyklus mit 2-9 C-Atomen und 1-4 N-, O- und/oder S-Atomen, der unsubstituiert oder einfach durch Hal, A, CN, OY, NYY, -NY-COY, COOY, -Alk-OY oder -Alk-NYY substituiert sein kann, bedeutet. Es ist besonders bevorzugt, dass "Het¹" ein- oder zweikerniges Heteroaryl mit 2-9 C-Atomen und 1-3 N- und/oder S-Atomen, das unsubstituiert oder einfach durch Hal, A, CN oder NYY substituiert sein kann, bedeutet. Es ist ganz besonders bevorzugt, dass "Het¹" Pyrazol, Pyrrol oder Thiazol bedeutet. Es versteht sich, dass die jeweiligen Bedeutungen von "Het¹" unabhängig voneinander in den Radikalen einer erfindungsgemäßen Formel sind.

Der Begriff "Heterozyklus" bezeichnet im Sinne der Erfindung ein mono- oder polyzyklisches System mit 3 bis 20 Ringatomen, vorzugsweise 3 bis 14 Ringatomen, besonders bevorzugt 3 bis 10 Ringatomen, umfassend C-Atome und 1, 2, 3, 4 oder 5 Heteroatome, insbesondere Stickstoff, Sauerstoff und/oder Schwefel, wobei die Heteroatome identisch oder verschieden sind. Das zyklische System kann gesättigt oder ein- oder mehrfach ungesättigt sein. Der Begriff "Heteroaryl" schließt solche Systeme ein, in denen der aromatische Zyklus Teil eines bi- oder polyzyklischen gesättigten, teilweise ungesättigten und/oder aromatischen Systems ist, z.B. wenn der aromatische Zyklus an "Aryl", "Cycloalkyl", "Heteroaryl" oder "Heterocyclyl" über ein beliebiges Ringmitglied des Heterozyklus fusioniert ist. Die Bindung an das Grundgerüst der Formel (I) kann über jedes Ringmitglied des Heterozyklus erfolgen. Beispiele für geeignete Heterozyklen sind Pyrrolidinyl, Thiapyrrolidinyl, Piperidinyl, Piperazinyl, Oxapiperazinyl, Oxapiperidinyl, Oxadiazolyl, Tetrahydrofuryl, Imidazolidinyl, Thiazolidinyl, Tetrahydropyranyl, Morpholinyl, Tetrahydrothiophenyl, Dihydropyranyl.

In einer Ausgestaltung der Erfindung ist "Het²" ein einkerniger gesättigter Heterozyklus mit 2-7 C-Atomen und 1-4 N-, O- und/oder S-Atomen, der unsubstituiert oder einfach durch A substituiert sein kann. Es ist bevorzugt, dass "Het²" einen einkernigen gesättigten Heterozyklus mit 2-5 C-Atomen und 1-2 N- und/oder O-Atomen, der unsubstituiert oder einfach durch A substituiert sein kann, bedeutet. Es versteht sich, dass die jeweiligen Bedeutungen von "Het²" unabhängig voneinander in den Radikalen einer erfindungsgemäßen Formel sind.

Der Begriff "Halogen", "Halogenatom", "Halogensubstituent" oder "Hal" bezeichnet im Sinne der Erfindung ein oder mehrere Atome von Fluor (F), Brom (Br), Chlor (Cl) oder Iod (I). Die Bezeichnungen "Dihalogen", "Trihalogen" und "Perhalogen" beziehen sich auf zwei, drei oder vier Substituenten, wobei jeder Substituent unabhängig voneinander aus der Gruppe von F, Cl, Br oder I ausgewählt werden kann. "Halogen" meint vorzugsweise F, Cl oder Br. F und Cl sind besonders bevorzugt, insbesondere wenn die Halogene an einer Alkyl-(Haloalkyl) oder Alkoxygruppe substituiert sind (z.B. CF₃ and CF₃O).

Das Radikal R1 bedeutet vorzugsweise H oder A, besonders bevorzugt A.

Das Radikal R2 bedeutet vorzugsweise H, A oder -CH(R6)(R7).

Das Radikal R3 bedeutet vorzugsweise CN oder Het¹, besonders bevorzugt CN oder Pyrazol, ganz besonders bevorzugt CN.

Das Radikal R4 bedeutet vorzugsweise H, A oder CN, besonders bevorzugt A.

Das Radikal R5 bedeutet vorzugsweise H, A, Hal, COOY, Alk-OA, Ar oder Het², besonders bevorzugt H, Hal, Alk-OA oder Het².

Wenn die Radikale R3 und R5 einen gemeinsamen Rest bilden, so befinden sie sich vorzugsweise an benachbarten C-Atomen.

Das Radikal R6 bedeutet vorzugsweise -CO-NYY, -CO-NY-OY, -CO-NY-C(=NH)-NYY oder -CO-NY-Alk-OY.

Das Radikal R7 bedeutet vorzugsweise Ar oder Het¹, besonders bevorzugt Ar.

Das Radikal X bedeutet vorzugsweise O oder Het¹, besonders bevorzugt O, Pyrazol oder Pyrrol, ganz besonders bevorzugt O.

Dementsprechend sind Gegenstand der Erfindung diejenigen Verbindungen der Formel (I), in denen mindestens eines der genannten Radikale eine der vorstehend angegebenen Bedeutungen hat. Nicht näher bezeichneten Radikale im Kontext einer Ausführungsform der Formel (I), Teilformel davon oder irgendeines Restes daran sollen die bei der Formel (I) angegebene Bedeutung haben, wie sie hierin offenbart ist, um die Aufgabe der Erfindung zu lösen. Das heißt, dass die genannten Radikale sämtliche ihnen zugeordneten Bedeutungen annehmen können, wie sie vor- oder nachstehend beschrieben sind, einschließlich jeglicher bevorzugter Ausführungsformen, ohne auf diese beschränkt zu sein und unabhängig von ihrem Auftreten in einem anderen bestimmten Kontext. Es versteht sich insbesondere, dass jede Ausführungsform eines bestimmten Radikals mit jeder Ausführungsform eines oder mehrerer anderer Radikale kombiniert werden kann.

In einer anderen bevorzugten Ausgestaltung der vorliegenden Erfindung werden Pyrazolochinolin-Derivate der Teilformel (IE) bereitgestellt 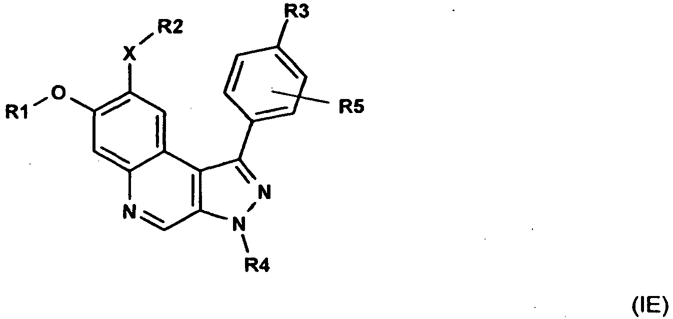 worin R1 bis R5 und X die oben angebene Bedeutung aufweisen.

In einer besonders bevorzugten Ausgestaltung der vorliegenden Erfindung werden Pyrazolochinolin-Derivate der Teilformel (IA) bereitgestellt 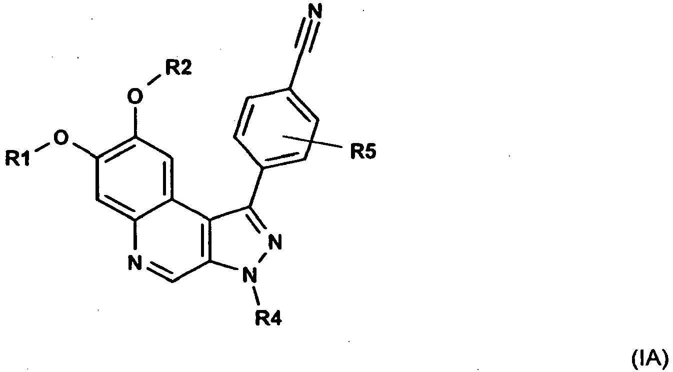 worin
- R1, R4: Y,
- R2: Y oder -CH(R6)(R7),
- R5: Hal, Y, COOY, Alk-OA oder Het²,
- R6: -CO-NYY, -CO-NY-OY, -CO-NY-C(=NH)-NYY oder -CO-NY-Alk-OY,
- R7: Ar oder Het¹,
- Y: H oder A,
- A: unverzweigtes oder verzweigtes Alkyl mit 1-4 C-Atomen, wobei unabhängig voneinander 1-3 H-Atome durch Hal ersetzt sein können,
- Alk: Alkylen mit 1-3 C-Atomen, wobei 1-2 H-Atome durch Hal und/oder OH ersetzt sein können,
- Ar: unsubstituiertes oder einfach durch Hal substituiertes Phenyl,
- Het¹: ein- oder zweikerniges Heteroaryl mit 2-9 C-Atomen und 1-3 N- und/oder S-Atomen, das unsubstituiert oder einfach durch Hal, A, CN oder NYY substituiert sein kann,
- Het²: einen einkernigen gesättigten Heterozyklus mit 3-5 C-Atomen und 1-2 N- und/oder O-Atomen, der unsubstituiert oder einfach durch A substituiert sein kann, und
- Hal: F, Cl, Br oder I bedeuten,
und/oder ihre physiologisch unbedenklichen Salze, Tautomere und/oder Stereoisomere, einschließlich deren Mischungen in allen Verhältnissen.

Ganz besonders bevorzugt sind solche Verbindungen der Formeln (I), (IA) und (IE), die in Tabelle 1 zusammengestellt sind.

**Tab. 1: Besonders bevorzugte Verbindungen der Formeln (I), (IA) und/oder (IE)**

| | |
|---|---|
| 1 | 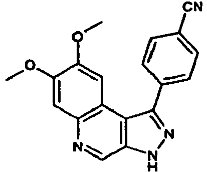 |
| 2 | 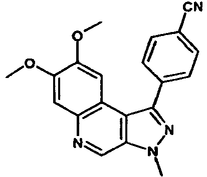 |
| 3 | 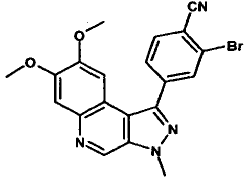 |
| 4 | 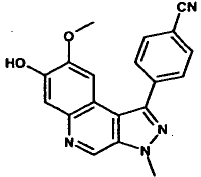 |
| 5 | 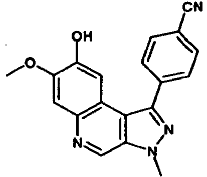 |
| 6 | 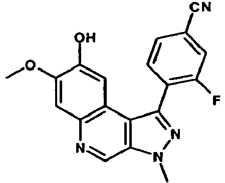 |
| 7 | 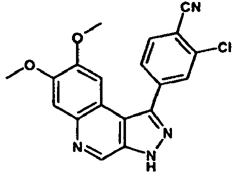 |
| 8 | 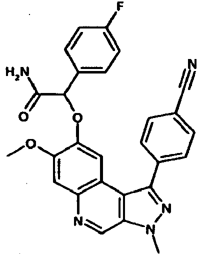 |
| 9 | 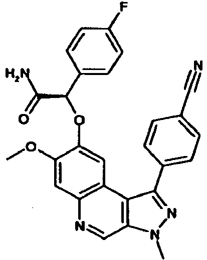 |
| 10 | 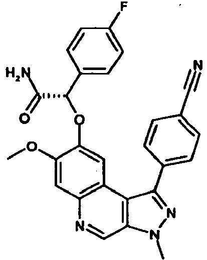 |
| 11 | 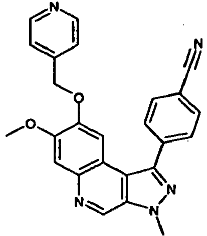 |
| 12 | 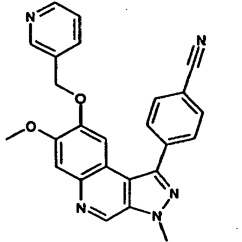 |
| 13 | 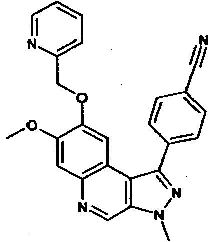 |
| 14 | 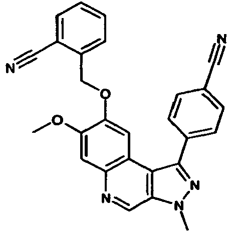 |
| 15 | 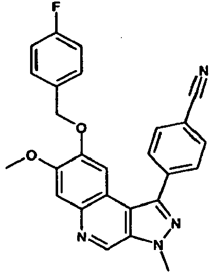 |
| 16 | 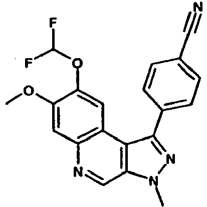 |
| 17 | 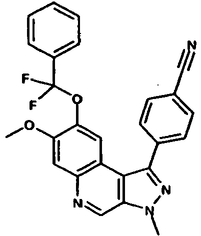 |
| 18 | 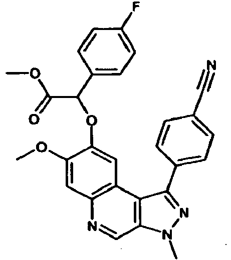 |
| 19 | 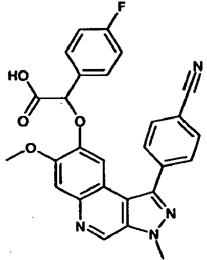 |
| 20 | 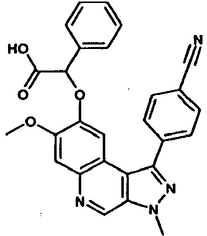 |
| 21 | 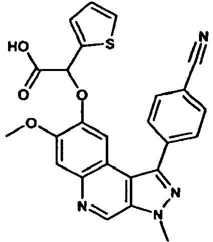 |
| 22 | 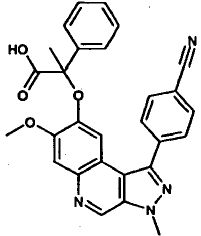 |
| 23 | 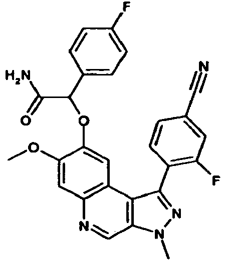 |
| 24 | 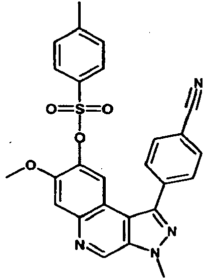 |
| 25 | 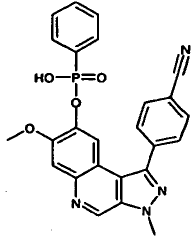 |
| 26 | 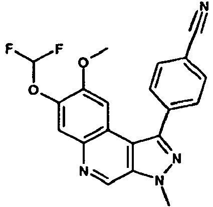 |
| 27 | 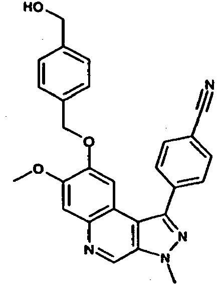 |
| 28 | 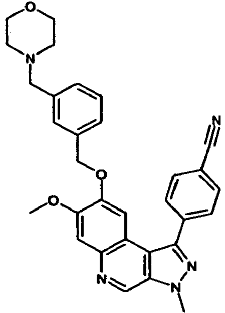 |
| 29 | 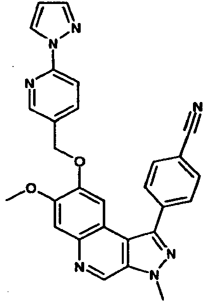 |
| 30 | 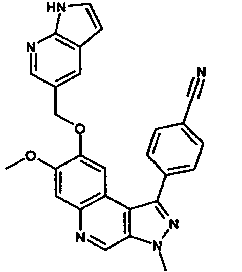 |
| 31 | 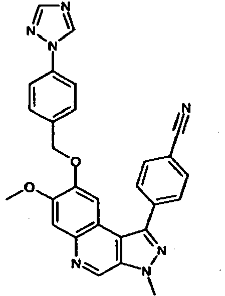 |
| 32 | 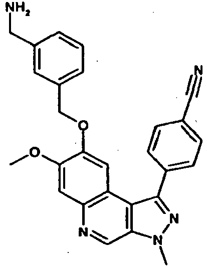 |
| 33 | 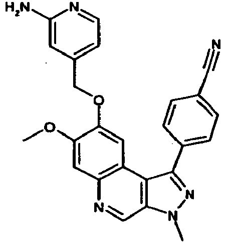 |
| 34 | 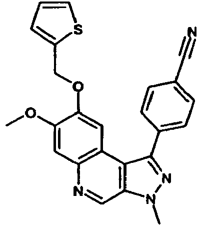 |
| 35 | 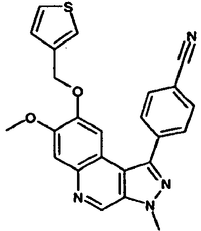 |
| 36 | 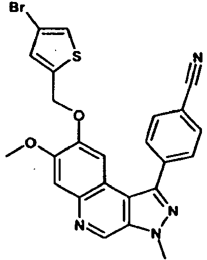 |
| 37 | 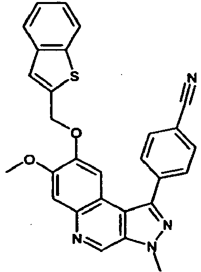 |
| 38 | 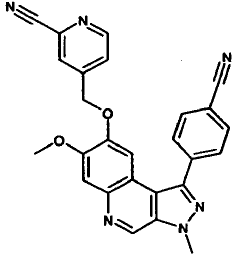 |
| 39 | 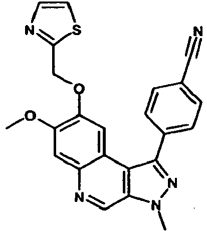 |
| 40 | 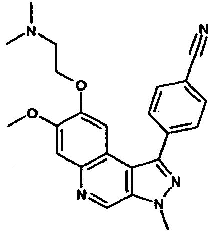 |
| 41 | 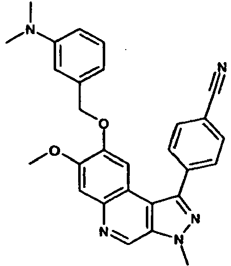 |
| 42 | 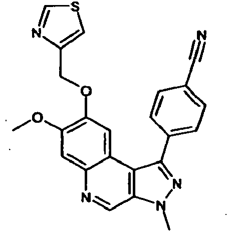 |
| 43 | 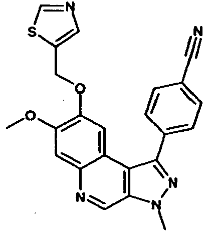 |
| 44 | 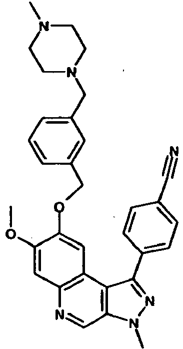 |
| 45 | 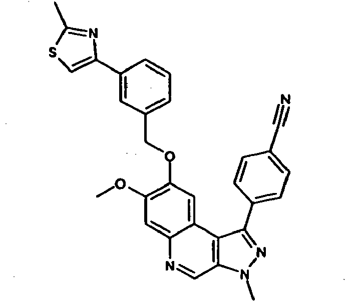 |
| 46 | 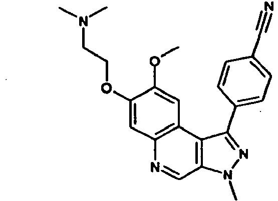 |
| 47 | 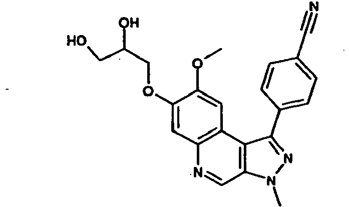 |
| 48 | 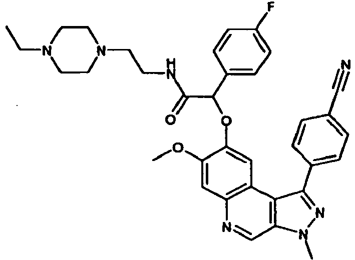 |
| 49 | 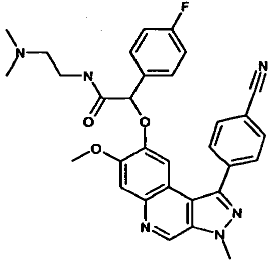 |
| 50 | 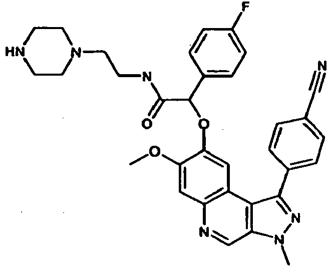 |
| 51 | 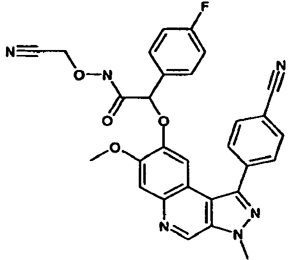 |
| 52 | 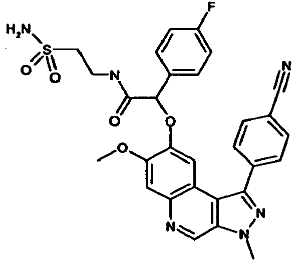 |
| 53 | 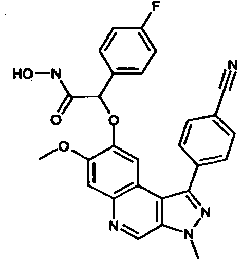 |
| 54 | 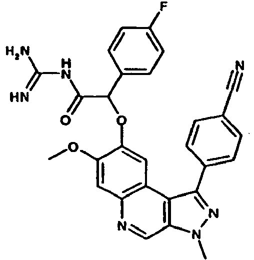 |
| 55 | 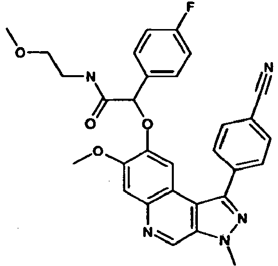 |
| 56 | 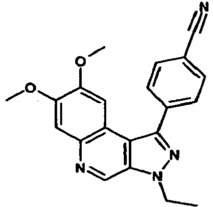 |
| 57 | 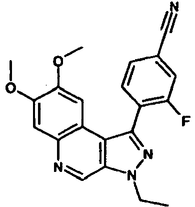 |
| 58 | 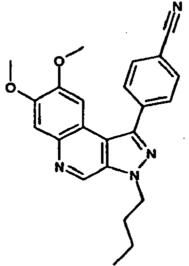 |
| 59 | 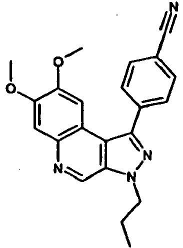 |
| 60 | 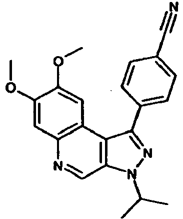 |
| 61 | 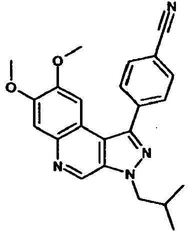 |
| 62 | 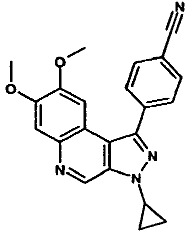 |
| 63 | 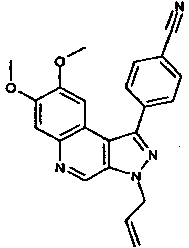 |
| 64 | 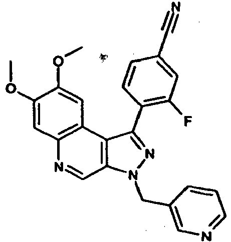 |
| 65 | 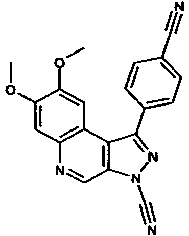 |
| 66 | 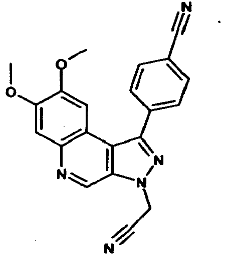 |
| 67 | 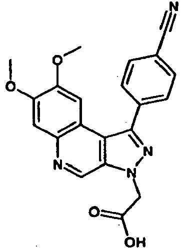 |
| 68 | 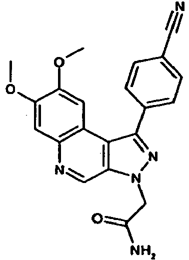 |
| 69 | 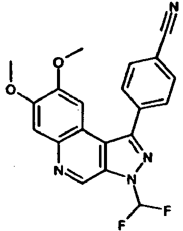 |
| 70 | 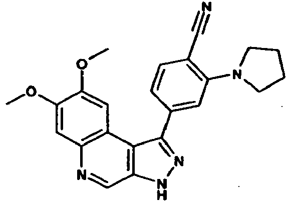 |
| 71 | 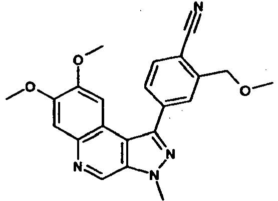 |
| 72 | 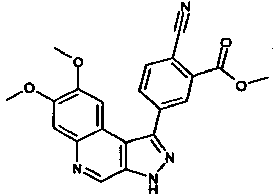 |
| 73 | 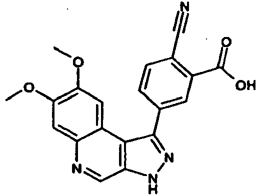 |
| 74 | 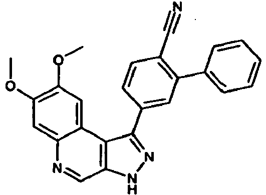 |
| 75 | 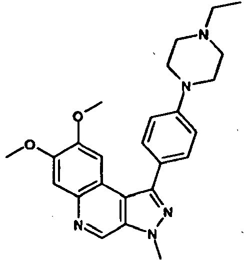 |
| 76 | 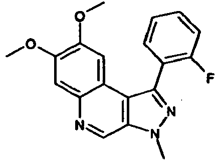 |
| 77 | 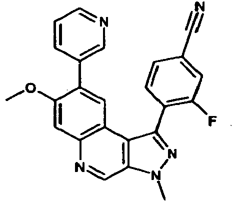 |
| 78 | 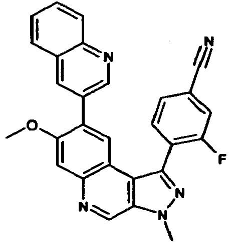 |
| 79 | 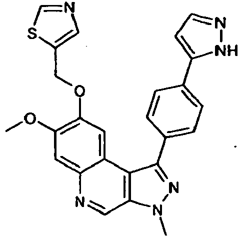 |
| 80 | 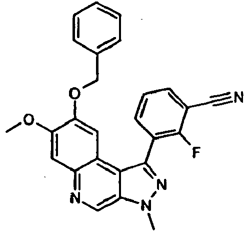 |
| 81 | 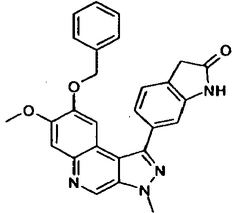 |
| 82 | 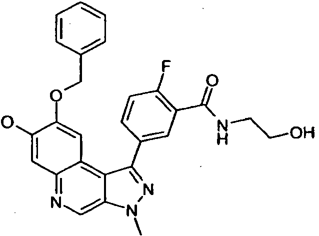 |
| 83 | 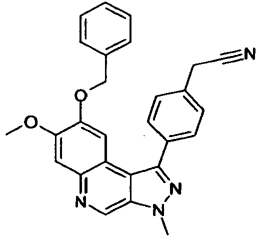 |
| 84 | 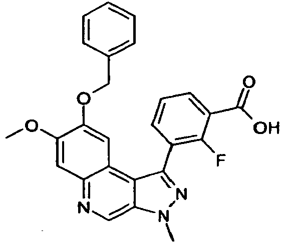 |
| 85 | 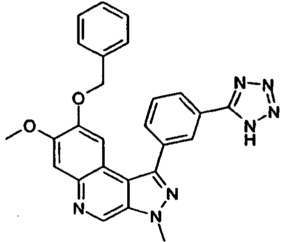 |
| 86 | 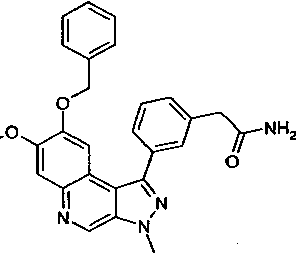 |
| 87 | 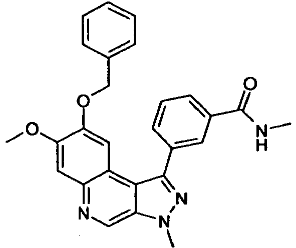 |
| 88 | 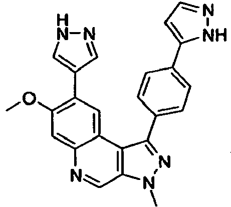 |
| 89 | 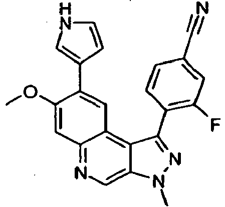 |
| 90 | 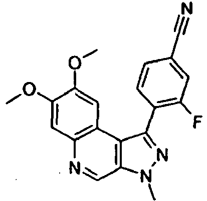 |
| 91 | 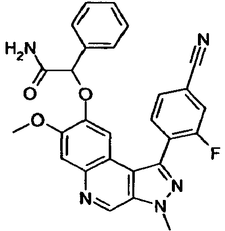 |

Die Verbindungen der Formel (I) sowie deren Teilformeln und auch die Ausgangsstoffe zu ihrer Herstellung werden nach an sich bekannten Methoden hergestellt, wie sie in der Literatur (z.B. in Standardwerken wie Houben-Weyl, Methoden der organischen Chemie, Georg-Thieme-Verlag, Stuttgart) beschrieben und/oder Fachmann bekannt sind, sowie unter Reaktionsbedingungen, die für die genannten Umsetzungen bekannt und geeignet sind. Dabei kann man auch von an sich bekannten, hier nicht näher erwähnten Varianten Gebrauch machen.

Die Reaktionszeit liegt je nach angewendeten Bedingungen zwischen einigen Minuten und 14 Tagen, die Reaktionstemperatur zwischen -15°C und 150°C, normalerweise zwischen 10°C und 100°C, besonders bevorzugt zwischen 20°C und 70°C.

Die Umsetzung erfolgt in einem inerten Lösungsmittel und in der Regel in Gegenwart eines säurebindenden Mittels, vorzugsweise einer organischen Base wie DIPEA, Triethylamin, Dimethylanilin, Pyridin, Chinolin, Piperidin oder Diethanolamin. Auch der Zusatz eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein. Als Basen eignen sich Metalloxide, wie z.B. Aluminiumoxid, Alkalimetallhydroxide (darunter Kaliumhydroxid, Natriumhydroxid und Lithiumhydroxid), Erdalkalimetallhydroxide (z.B. Bariumhydroxid und Calciumhydroxid) und Alkalimetallalkoholate (z.B. Kaliumethanolat und Natriumpropanolat). Als inerte Lösungsmittel eignen sich u.a. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Besonders bevorzugt sind Glykolether, wie Ethylenglycolmonomethylether, THF, Dichlormethan und/oder DMF.

Das Verfahren und die anschließende Aufarbeitung des Reaktionsgemisches können grundsätzlich als Batch-Reaktion oder in kontinuierlicher Reaktionsweise durchgeführt werden. Die kontinuierliche Reaktionsweise umfasst z.B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Co-Kristallisation oder durch Nanofiltration an Membranen.

Die Verbindungen der Formel (IE) können vorzugsweise erhalten werden, indem man eine Verbindung der Formel (IIA) oder aber eine Verbindung der Formel (III) umsetzt. Gegenstand der vorliegenden Erfindung ist damit auch ein Verfahren zum Herstellen von Verbindungen der Formel (IE), Teilformeln davon und/oder ihrer physiologisch unbedenklichen Salze, Tautomere und/oder Stereoisomere, einschließlich deren Mischungen in allen Verhältnissen, mit den folgenden Schritten:
(a) Umsetzen einer Verbindung der Teilformel (IIA) 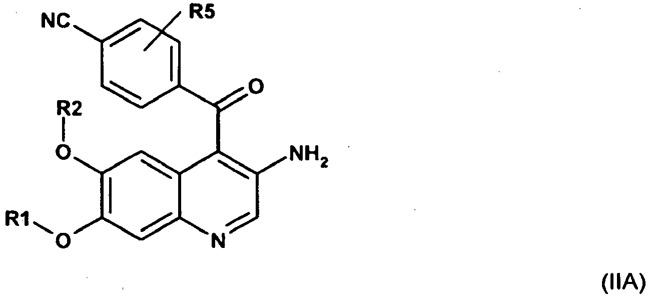 worin
   - R1, R2: unabhängig voneinander A oder -Alk-Ar,
   - R5: Hal, Y, COOY, Alk-OA oder Het²,
   - Y: H oder A
   - A: unverzweigtes oder verzweigtes Alkyl mit 1-4 C-Atomen, wobei unabhängig voneinander 1-3 H-Atome durch Hal ersetzt sein können,
   - Alk: Alkylen mit 1-3 C-Atomen, wobei 1-2 H-Atome durch Hal ersetzt sein können,
   - Ar: unsubstituiertes oder einfach durch Hal substituiertes Phenyl,
   - Het²: einen einkernigen gesättigten Heterozyklus mit 3-5 C-Atomen und 1-2 N- und/oder O-Atomen, der unsubstituiert oder einfach durch A substituiert sein kann, und
   - Hal: F, Cl, Br oder I bedeuten,
   im sauren Milieu mit einem Reduktionsmittel und mit einer Verbindung E-NO₂, worin E ein Element der 1. Hauptgruppe bedeutet,
   unter Erhalt von Verbindungen der Teilformel (IB) 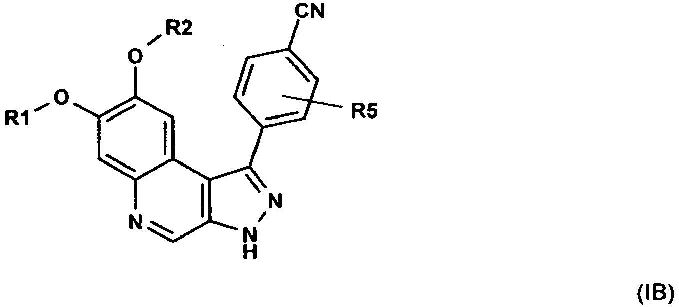 worin R1, R2 und R5 die oben in Teilformel (IIA) angegebene Bedeutung aufweisen,
   und gegebenenfalls
(b') Umsetzen der Verbindungen der Teilformel (IB) mit einer Verbindung Hal-R4, worin R4 und Hal die oben angegebene Bedeutung aufweisen,
   unter Erhalt von Verbindungen der Teilformel (IC) 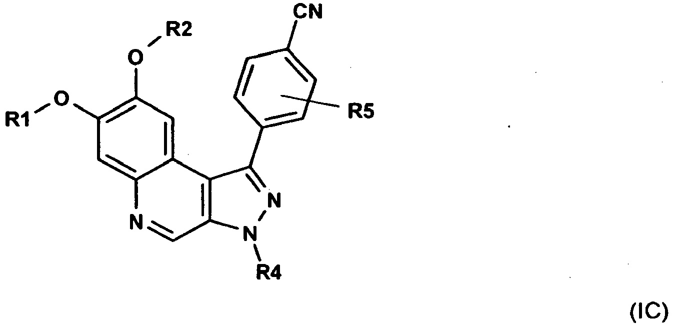 worin R1, R2 und R5 die die oben in Teilformel (IIA) angegebene Bedeutung aufweisen, und R4 die oben angegebene Bedeutung aufweist,
(b") Umwandeln von R1, -O-R2, R4, R5 und/oder der CN-Gruppe der Verbindungen der Teilformel (IC) unter Erhalt von Verbindungen der Formel (IE) 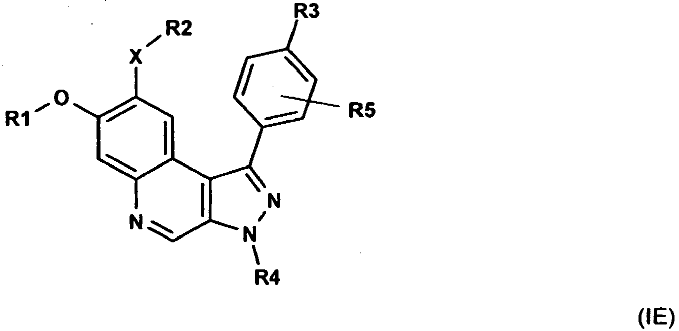 worin R1, R2, R3, R4, R5 und X die oben angegebene Bedeutung aufweisen,
   und/oder
(b"') Umwandeln einer Base oder Säure der Verbindungen der Formel (IE) oder Teilformeln (IB) oder (IC) in eines ihrer physiologisch unbedenklichen Salze.

Ein anderer Gegenstand der vorliegenden Erfindung ist ein alternatives Verfahren zum Herstellen von Verbindungen der Formel (I), Teilformeln davon und/oder ihrer physiologisch unbedenklichen Salze, Tautomere und/oder Stereoisomere, einschließlich deren Mischungen in allen Verhältnissen, mit den folgenden Schritten:
(a) Umsetzen einer Verbindung der Formel (III) 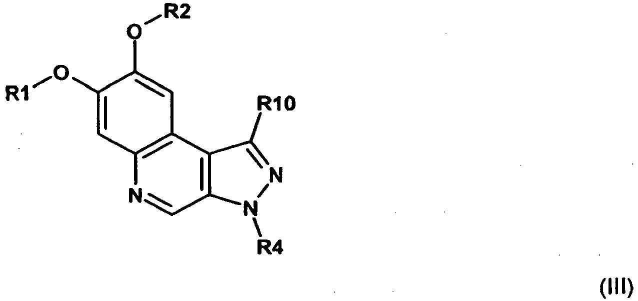 worin
   - R1, R2: unabhängig voneinander A oder -Alk-Ar,
   - R4: Y,
   - R10: Hal,
   - Y: H oder A,
   - A: unverzweigtes oder verzweigtes Alkyl mit 1-4 C-Atomen, wobei unabhängig voneinander 1-3 H-Atome durch Hal ersetzt sein können,
   - Alk: Alkylen mit 1-3 C-Atomen, wobei 1-2 H-Atome durch Hal ersetzt sein können,
   - Ar: unsubstituiertes oder einfach durch Hal substituiertes Phenyl, und
   - Hal: F, Cl, Br oder I bedeuten,
   mit einer Verbindung der Formel (IV) 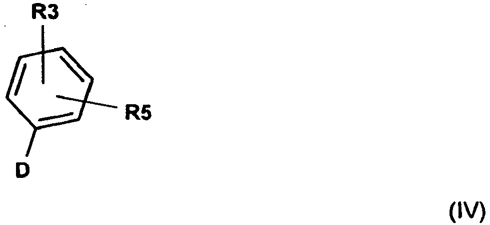 worin
   - D: Borsäure, Borester, organische Zinnverbindung oder Bortrifluormethansulfonat bedeutet, und
   - R3 und R5: die oben angegebene Bedeutung aufweisen,
   unter Erhalt von Verbindungen der Teilformel (ID) 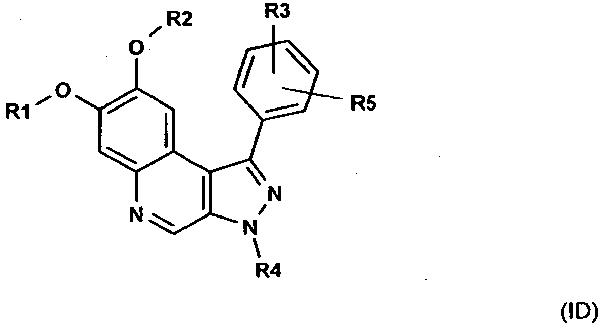 worin R1, R2 und R4 die oben in Formel (III) angegebene Bedeutung aufweisen, und R3 und R5 die oben angegebene Bedeutung aufweisen,
   und gegebenenfalls
(b') Umwandeln von R1, -O-R2, R3, R4 und/oder R5 der Verbindungen der Teilformel (ID) unter Erhalt von Verbindungen der Formel (I) 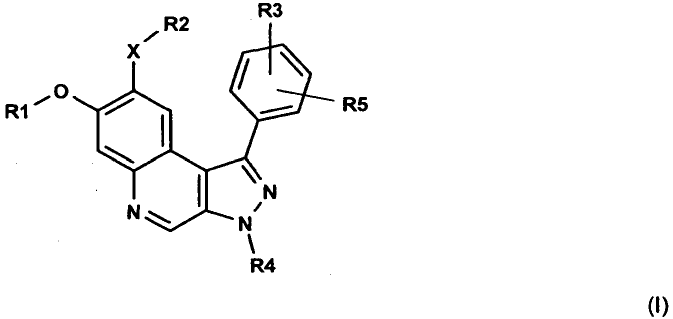 worin R1, R2, R3, R4, R5 und X die oben angegebene Bedeutung aufweisen, und/oder
(b") Umwandeln einer Base oder Säure der Verbindungen der Formel (I) oder Teilformel (ID) in eines ihrer physiologisch unbedenklichen Salze.

Die Erfindung betrifft auch Zwischenverbindungen der Formel (II) 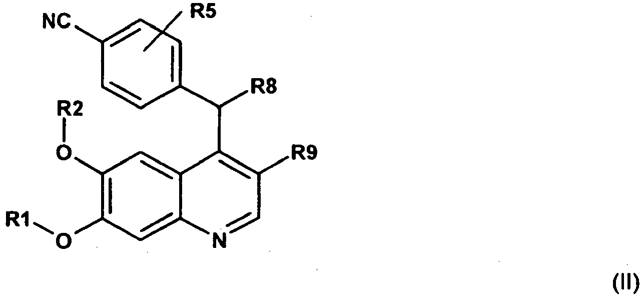 worin
- R8: CN oder =O, und
- R9: NO₂ oder NYY bedeuten, und
- R1, R2, R5 und Y: die oben angegebene Bedeutung aufweisen,
und/oder ihre Salze, Tautomere und/oder Stereoisomere, einschließlich deren Mischungen in allen Verhältnissen.

In einer bevorzugten Ausführungsform der vorliegenden Erfindung werden Zwischenverbindungen mit der Teilformel (IIA) bereitgestellt 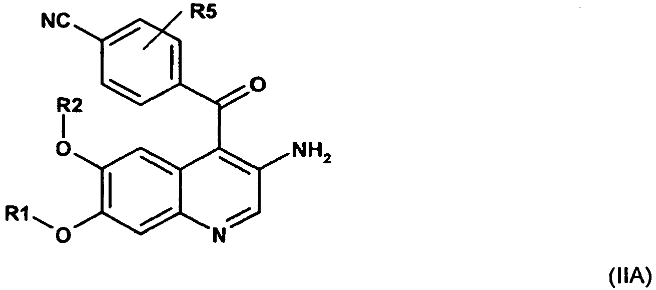 worin
- R1, R2: unabhängig voneinander A oder -Alk-Ar,
- R5: Hal, Y, COOY, Alk-OA oder Het²,
- Y: H oder A
- A: unverzweigtes oder verzweigtes Alkyl mit 1-4 C-Atomen, wobei unabhängig voneinander 1-3 H-Atome durch Hal ersetzt sein können,
- Alk: Alkylen mit 1-3 C-Atomen, wobei 1-2 H-Atome durch Hal ersetzt sein können,
- Ar: unsubstituiertes oder einfach durch Hal substituiertes Phenyl,
- Het²: einen einkernigen gesättigten Heterozyklus mit 3-5 C-Atomen und 1-2 N- und/oder O-Atomen, der unsubstituiert oder einfach durch A substituiert sein kann, und
- Hal: F, Cl, Br oder I bedeuten,
und/oder ihre Salze, Tautomere und/oder Stereoisomere, einschließlich deren Mischungen in allen Verhältnissen.

Gegenstand der Erfindung ist auch ein Verfahren zum Herstellen von Zwischenverbindungen der Formel (II) und/oder ihrer Salze, Tautomere und/oder Stereoisomere, einschließlich deren Mischungen in allen Verhältnissen, mit den folgenden Schritten:
(a) Umsetzen einer Verbindung der Formel (V) 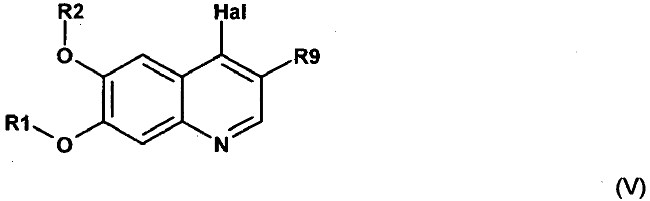 worin R1, R2, R9 und Hal die in oben angegebene Bedeutung aufweisen,

   mit einer Verbindung der Formel (VI) 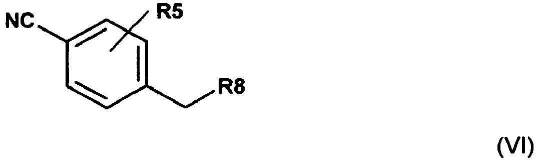 worin R5 und R8 die oben angegebene Bedeutung aufweisen,
   unter Erhalt von Verbindungen der Formel (II) 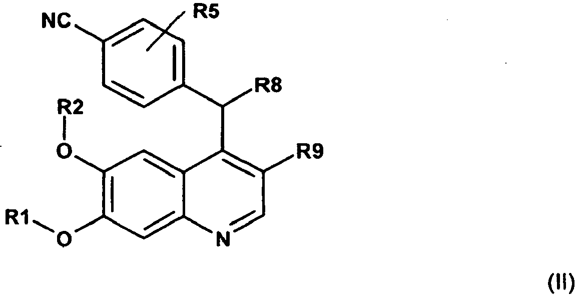 worin R1, R2, R5, R8 und R9 die in oben angegebene Bedeutung aufweisen.
   und gegebenenfalls
(b) Umwandeln einer Base oder Säure der Verbindungen der Formel (II) in eines ihrer Salze.

Die Erfindung betrifft weiterhin Zwischenverbindungen der Formel (III) 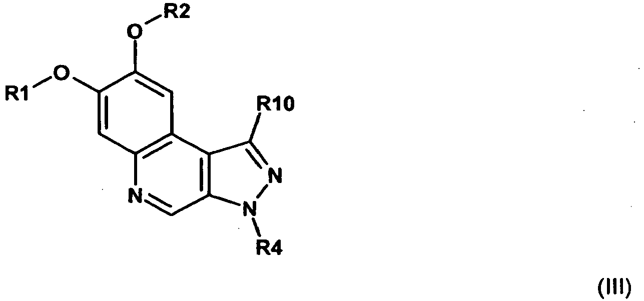 worin
- R10: H oder Hal bedeutet, und
- R1, R2, R4 und Hal: die oben angegebene Bedeutung aufweisen,
und/oder ihre Salze, Tautomere und/oder Stereoisomere, einschließlich deren Mischungen in allen Verhältnissen.

In einer bevorzugten Ausführungsform der vorliegenden Erfindung werden Zwischenverbindungen der Formel (III) bereitgestellt, worin
- R1, R2: unabhängig voneinander A oder -Alk-Ar,
- R4: Y,
- R10: Hal,
- Y: H oder A,
- A: unverzweigtes oder verzweigtes Alkyl mit 1-4 C-Atomen, wobei unabhängig voneinander 1-3 H-Atome durch Hal ersetzt sein können,
- Alk: Alkylen mit 1-3 C-Atomen, wobei 1-2 H-Atome durch Hal ersetzt sein können,
- Ar: unsubstituiertes oder einfach durch Hal substituiertes Phenyl, und
- Hal: F, Cl, Br oder I bedeuten,
und/oder ihre Salze, Tautomere und/oder Stereoisomere, einschließlich deren Mischungen in allen Verhältnissen.

Noch ein weiterer Gegenstand der Erfindung ist ein Verfahren zum Herstellen von Zwischenverbindungen der Formel (III), Teilformeln davon und/oder ihrer Salze, Tautomere und/oder Stereoisomere, einschließlich deren Mischungen in allen Verhältnissen, mit den folgenden Schritten:
(a) Umsetzen einer Verbindung der Formel (VII) 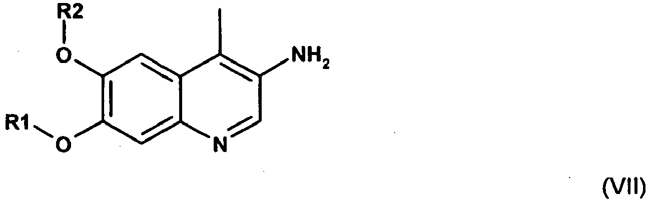 worin R1 und R2 die oben angegebene Bedeutung aufweisen,
   im sauren Milieu mit einer Verbindung E-NO₂, worin E ein Element der 1. Hauptgruppe bedeutet,
   unter Erhalt von Verbindungen der Teilformel (IIIA) 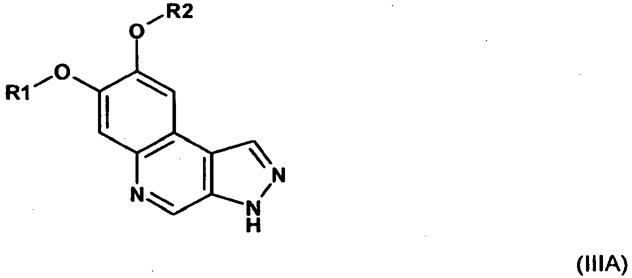 worin R1 und R2 die oben angegebene Bedeutung aufweisen,
   und gegebenenfalls
(b') Halogenieren der Verbindungen der Teilformel (IIIA) unter Erhalt von Verbindungen der Teilformel (IIIB) 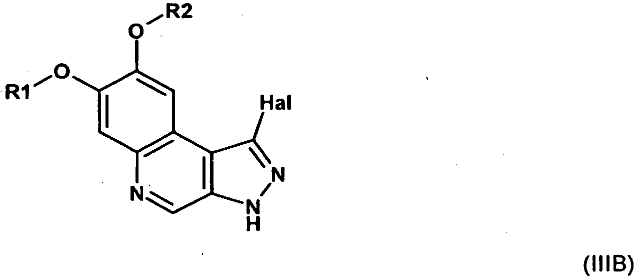 worin R1, R2 und Hal die oben angegebene Bedeutung aufweisen,
(b") Umsetzen der Verbindungen der Formel (IIIA) oder (IIIB) mit einer Verbindung Hal-R4, worin R4 und Hal die oben angegebene Bedeutung aufweisen,
   unter Erhalt von Verbindungen der Formel (III) 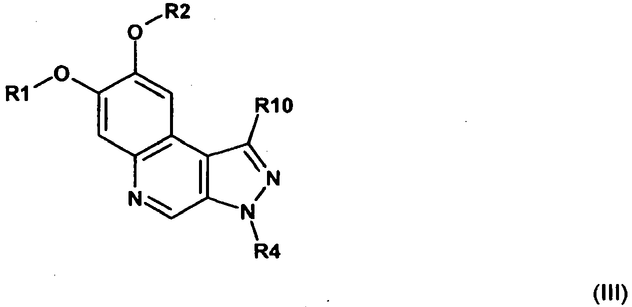 worin R1, R2, R4 und R10 die oben angegebene Bedeutung aufweisen,
   und/oder
(b"') Umwandeln einer Base oder Säure der Verbindungen der Formel (III) in eines ihrer Salze.

Die Ausgangsverbindungen sind in der Regel bekannt. Sind sie neu, so können sie nach an sich bekannten Methoden hergestellt werden. Die Verbindungen der Formeln (IV), (V), (VI) und (VII) können nach bekannten Methoden bereitgestellt werden. Fall gewünscht, können die Ausgangsstoffe in-situ gebildet werden, so dass man sie aus dem Reaktionsgemisch nicht isoliert, sondern sofort weiter zu den erfindungsgemäßen Verbindungen umsetzt. Es ist ebenso möglich, die Reaktion schrittweise durchzuführen.

Die genannten erfindungsgemäßen Verbindungen lassen sich in ihrer endgültigen Nichtsalzform verwenden. Andererseits umfasst die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formeln (I), (II) und (III) sowie ihrer Teilformeln werden größtenteils konventionell hergestellt. Sofern die Verbindungen eine Carbonsäuregruppe enthalten, lässt sich eines ihrer geeigneten Salze dadurch bilden, dass man die Verbindung mit einer geeigneten Base zum entsprechenden Basenadditionssalz umsetzt. Solche Basen sind zum Beispiel Alkalimetallhydroxide (z.B. Kaliumhydroxid, Natriumhydroxid und Lithiumhydroxid), Erdalkalimetallhydroxide (z.B. Bariumhydroxid und Calciumhydroxid), Alkalimetallalkoholate (z.B. Kaliumethanolat und Natriumpropanolat) sowie verschiedene organische Basen wie Piperidin, Diethanolamin und N-Methylglutamin. Eine Base der Formeln (I), (II) und (III) sowie deren Teilformeln kann mit einer Säure in das zugehörige Säureadditionssalz übergeführt werden, beispielsweise durch Umsetzung äquivalenter Mengen der Base und der Säure in einem inerten Lösungsmittel, wie z.B. Ethanol, und Eindampfen im Anschluss. Für diese Umsetzung kommen insbesondere Säuren in Frage, die physiologisch unbedenkliche Salze liefern, wie z.B. Halogenwasserstoffe (z.B. Chlorwasserstoff, Bromwasserstoff oder Jodwasserstoff), andere Mineralsäuren und ihre entsprechenden Salzen (z.B. Sulfat, Nitrat oder Phosphat und dergleichen), Alkyl- und Monoarylsulfonaten (z.B. Ethansulfonat, Toluolsulfonat und Benzolsulfonat) sowie andere organische Säuren und ihre entsprechenden Salze (z.B. Acetat, Trifluoracetat, Tartrat, Maleat, Succinat, Citrat, Benzoat, Salicylat, Ascorbat und dergleichen. Salze mit physiologisch bedenklichen Säuren, z.B. Pikrate, können zur Isolierung und/oder Aufreinigung der Verbindungen der Formel (I) verwendet werden.

Im Hinblick auf das oben Gesagte sieht man, dass unter dem Ausdruck "pharmazeutisch unbedenkliches Salz" im vorliegenden Zusammenhang ein Wirkstoff zu verstehen ist, der eine Verbindung der Formel (I) in der Form eines ihrer Salze enthält, insbesondere dann, wenn diese Salzform dem Wirkstoff im Vergleich zu der freien Form des Wirkstoffs verbesserte pharmakokinetische Eigenschaften verleiht. Die pharmazeutisch unbedenkliche Salzform des Wirkstoffs kann diesem Wirkstoff auch erst eine gewünschte pharmakokinetische Eigenschaft verleihen und kann sogar die Pharmakodynamik dieses Wirkstoffs in Bezug auf seine therapeutische Wirksamkeit im Körper positiv beeinflussen.

Erfindungsgemäße Verbindungen können aufgrund ihrer Molekülstruktur chiral sein und dementsprechend in verschiedenen enantiomeren Formen auftreten. Sie können daher in racemischer oder in optisch aktiver Form vorliegen. Da sich die pharmazeutische Wirksamkeit der Racemate bzw. der Stereoisomeren der Verbindungen der Formel (I) unterscheiden kann, mag es wünschenswert sein, die Enantiomere zu verwenden. In diesen Fällen kann das Endprodukt oder bereits das Zwischenprodukt in enantiomere Verbindungen, durch dem Fachmann bekannte chemische oder physikalische Maßnahmen, aufgetrennt oder bereits als solche bei der Synthese eingesetzt werden.

Es wurde überraschend gefunden, dass die erfindungsgemäßen Verbindungen eine spezifische Hemmung von en Serin-Threonin-Proteinkinasen bewirken. Ein weiterer Gegenstand der Erfindung betrifft deshalb die Verwendung von Verbindungen der Formel (I) bzw. Teilformeln davon und/oder ihrer physiologisch unbedenklichen Salze, Tautomere und/oder Stereoisomere, einschließlich deren Mischungen in allen Verhältnissen, zur Hemmung von Serin-Threonin-Proteinkinasen, vorzugsweise PIKK, besonders bevorzugt DNA-PK. Der Begriff "Hemmung" bezieht sich auf jede Verringerung der Aktivität, die auf der Wirkung der spezifischen erfindungsgemäßen Verbindungen basiert, indem letztere in der Lage sind, mit dem Zielmolekül derart zu interagieren, dass eine Erkennung, Bindung und Blockierung ermöglicht wird. Die Verbindungen zeichnen sich durch hohe Affinität zu wenigstens einer Serin-Threonin-Proteinkinasen aus, wodurch eine verlässliche Bindung und vorzugsweise vollständige Blockade der Kinaseaktivität sichergestellt wird. Die Verbindungen sind besonders bevorzugt mono-spezifisch, um eine ausschließliche und unmittelbare Erkennung der ausgewählten Kinase zu garantieren. Der Begriff der "Erkennung" bezieht sich hierbei auf jede Art der Wechselwirkung zwischen der Verbindung und den genannten Zielmolekülen, insbesondere kovalente oder nicht-kovalente Bindungen, wie z.B. eine kovalente Bindung, hydrophobe/ hydrophile Wechselwirkungen, van der Waals'sche Kräfte, Ionenbeziehung, Wasserstoffbrücken, Ligand-Rezeptor-Wechselwirkungen, Basenpaarungen von Nukleotiden oder Wechselwirkungen zwischen Epitop und Antikörper-Bindungsstelle.

Die erfindungsgemäßen Verbindungen zeigen eine vorteilhafte biologische Aktivität, die in den hierin beschriebenen Tests nachweisbar ist, wie z.B. auf Enzymen basierenden Assays. Die Messung der Kinaseaktivität ist eine dem Fachmann wohlbekannte Technik. Generische Testsysteme zur Bestimmung der Kinaseaktivität mit Substraten, z.B. Histon (Alessi et al. (1996) FEBS Lett. 399(3): 333) oder dem basischen Myelinprotein sind in der Literatur beschrieben (Campos-González & Glenney (1992) JBC 267: 14535). Zur Identifikation von Kinase-Inhibitoren stehen verschiedene Assay-Systeme zur Verfügung. Beim Scintillation Proximity Assay (Sorg et al. (2002) J Biomolecular Screening 7: 11) und dem FlashPlate Assay werden die radioaktive Phosphorylierung eines Proteins oder Peptids als Substrat mit ATP gemessen. Bei Vorliegen einer inhibitorischen Verbindung ist kein oder ein vermindertes radioaktives Signal nachweisbar. Ferner sind die Homogeneous Timeresolved Fluorescence Resonance Energy Transfer- (HTR-FRET-) und Fluoreszenzpolarisations- (FP-) Technologien als Assay-Verfahren nützlich (Sills et al. (2002) J Biomolecular Screening 191). Andere nicht-radioaktive ELISA-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszens nachweisbar.

Die vorgenannte Verwendung der Verbindungen kann in in-vitro oder in-vivo Modellen geschehen. Die Suszeptibilität einer bestimmten Zelle gegenüber der Behandlung mit den erfindungsgemäßen Verbindungen kann durch Testen in-vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in-vitro können kultivierte Zellen aus einer Biopsieprobe verwendet werden. Die Menge der nach der Behandlung zurückbleibenden Zellen wird dann bestimmt. Die Verwendung in-vitro erfolgt insbesondere an Proben von Säugerspezies, die an Krebs, Tumoren, Metastasen, Störungen der Angiogenese, retroviralen Erkrankungen, Immunerkrankungen und/oder pathogenen Alterungsprozessen leiden. Der Wirt oder Patient kann jeglicher Säugerspezies angehören, z. B. einer Primatenspezies, insbesondere Menschen, aber auch Nagetieren (einschließlich Mäusen, Ratten und Hamstern), Kaninchen, Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Das Testen mehrerer spezifischer Verbindungen ermöglicht die Auswahl des Wirkstoffs, der am besten für die Behandlung des Patienten geeignet erscheint. Die in-vivo Dosis der gewählten Verbindung wird vorteilhaft auf die Suszeptibilität der Kinase und/oder Schwere der Erkrankung des Patienten mit Blick auf die in-vitro Daten abgestimmt, wodurch die therapeutische Wirksamkeit merklich erhöht ist. Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die nachfolgende Lehre der Erfindung und deren Ausführungsformen betreffend die Verwendung von Verbindungen der Formel (I) für die Herstellung eines Arzneimittels zur Prophylaxe, Therapie und/oder Verlaufskontrolle ist gültig und ohne Einschränkungen auf die Verwendung der Verbindungen zur Hemmung der Kinaseaktivität anwendbar, sofern es sinnvoll erscheint.

Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden. In derartigen Tests zeigen und bewirken die erfindungsgemäßen Verbindungen einen inhibierenden Effekt, der gewöhnlich durch IC₅₀-Werte in einem geeigneten Bereich, bevorzugt im mikromolaren Bereich und bevorzugter im nanomolaren Bereich dokumentiert wird. Die Kinase wird insbesondere zu 50 % gehemmt, wenn die Konzentration der Verbindungen kleiner als 1 µM ist, vorzugsweise kleiner als 0,5 µM, besonders bevorzugt kleiner als 0,1 µM. Diese Konzentration wird als IC₅₀-Wert bezeichnet.

Gegenstand der Erfindung ist auch ein Arzneimittel umfassend mindestens eine Verbindung der Formel (I) bzw. Teilformeln davon und/oder ihre physiologisch unbedenklichen Salze, Tautomere und/oder Stereoisomere, einschließlich deren Mischungen in allen Verhältnissen. Noch ein Gegenstand der Erfindung ist eine pharmazeutische Zusammensetzung umfassend als Wirkstoff eine wirksame Menge von mindestens einer Verbindung der Formel (I) bzw. Teilformeln davon und/oder ihrer physiologisch unbedenklichen Salze, Tautomere und/oder Stereoisomere, einschließlich deren Mischungen in allen Verhältnissen, zusammen mit pharmazeutisch verträglichen Hilfsstoffen.

Ein "Arzneimittel", "Medikament" sowie eine "pharmazeutischen Zusammensetzung" oder "pharmazeutische Formulierung" ist hierbei jedes Mittel, welches in der Prophylaxe, Therapie, Verlaufskontrolle oder Nachbehandlung von Patienten eingesetzt werden kann, die zumindest zeitweise eine pathogene Modifikation des Gesamtzustandes bzw. des Zustandes einzelner Teile des Patientenorganismus zeigen, vorzugsweise infolge von Krebs, Tumoren, Metastasen, Störungen der Angiogenese, retroviralen Erkrankungen, Immunerkrankungen und/oder beschleunigten Alterungsprozessen, besonders bevorzugt infolge von Krebs, Tumoren, Metastasen und/oder Störungen der Angiogenese.

Um die protektive oder therapeutische Wirkung der erfindungsgemäßen Verbindungen zu erhöhen, können pharmazeutisch verträgliche Adjuvantien zugesetzt werden. Im Sinne der Erfindung ist jede Substanz, die mit den Verbindungen gemäß der Erfindung eine Wirkung ermöglicht, verstärkt oder modifiziert, ein "Adjuvans". Bekannte Adjuvantien sind z.B. Aluminiumverbindungen, wie z.B. Aluminiumhydroxid oder Aluminiumphosphat, Saponine, wie z.B. QS 21, Muramyldipeptid oder Muramyltripeptid, Proteine, wie z.B. Gammainterferon oder TNF, MF 59, Phosphatdibylcholin, Squalen oder Polyole. Ebenfalls kann die Co-Applikation von Ei-Albumin in komplettem Freund'schen Adjuvans eine gesteigerte zellvermittelte Immunität hervorrufen und somit die Wirkung gebildeter neutralisierender Antikörper unterstützen. Des Weiteren kann DNA, die eine immunstimulatorische Eigenschaft hat, oder die ein Protein mit Adjuvanseffekt kodiert, wie z.B. ein Cytokin, parallel oder in einem Konstrukt appliziert werden.

Das Einbringen des pharmazeutischen Mittels in eine Zelle respektive einen Organismus kann erfindungsgemäß auf jede Art und Weise geschehen, die es ermöglicht, dass die Kinasen mit den in der Zusammensetzung enthaltenen Verbindungen in Kontakt gebracht werden, infolgedessen eine Antwort induziert wird. Das pharmazeutische Mittel der vorliegenden Erfindung kann oral, transdermal, transmucosal, transurethal, vaginal, rektal, pulmonal, enteral und/oder parenteral angewendet werden. Die gewählte Art der Verabreichung richtet sich nach der Indikation, der zu verabreichenden Dosis, Individuumsspezifischen Parametern etc. Insbesondere ermöglichen die verschiedenen Arten der Verabreichung eine ortsspezifische Therapie, die Nebenwirkungen minimiert und die Wirkstoffdosis verringert. Ganz besonders bevorzugte Injektionen sind die intradermale, subkutane, intramuskuläre oder intravenöse Injektion. Die Applikation kann z.B. mit Hilfe so genannter Impfpistolen oder mittels Spritzen geschehen. Es ist auch möglich, die Substanz als Aerosol bereitzustellen, welches von dem Organismus, bevorzugt einem humanen Patienten, inhaliert wird.

Die Darreichungsformen des pharmazeutischen Mittels werden mit den üblichen festen oder flüssigen Trägerstoffen und/oder Verdünnungsmitteln und den üblicherweise eingesetzten Hilfsstoffen entsprechend der gewünschten Applikationsart in einer geeigneten Dosierung und in an sich bekannter Weise hergestellt. Grundsätzlich können also pharmazeutisch annehmbare und dem Fachkundigen bekannte Exzipienten einen Teil des erfindungsgemäßen pharmazeutischen Mittels bilden, wobei die Menge des Exzipientenmaterials, die mit dem Wirkstoff kombiniert wird, um eine Einzeldosierung herzustellen, in Abhängigkeit vom zu behandelnden Individuum und der Art der Verabreichung variiert. Diese pharmazeutisch verträglichen Zusätze umfassen Salze, Puffer, Füllstoffe, Stabilisatoren, Komplexbildner, Antioxidantien, Lösungsmittel, Bindemittel, Gleitmittel, Tablettenüberzüge, Geschmacksstoffe, Farbstoffe, Konservierungsmittel, Stellmittel und dergleichen. Beispiele für solche Exzipienten sind Wasser, pflanzliche Öle, Benzylalkohole, Alkylenglycol, Polyethylenglycol, Glycerintriacetat, Gelatine, Kohlenhydrate, wie z.B. Laktose oder Stärke, Magnesiumstearat, Talk und Vaseline.

Die pharmazeutische Formulierung kann als Tablette, Filmtablette, Dragee, Lutschtablette, Kapsel, Pille, Pulver, Granulat, Sirup, Saft, Tropfen, Lösung, Dispersion, Suspension, Suppositor, Emulsion, Implantat, Creme, Gel, Salbe, Paste, Lotion, Serum, Öl, Spray, Aerosol, Kleber, Pflaster oder Verband vorliegen. Als orale Darreichungsform werden vorzugsweise Tabletten, Filmtabletten, Dragees, Lutschtabletten, Kapseln, Pillen, Pulver, Granulate, Sirupe, Säfte, Tropfen, Lösungen, Dispersionen oder Suspensionen - auch als Depotform - zubereitet. Des Weiteren sind parenterale Arzneiformen, wie z.B. Suppositorien, Suspensionen, Emulsionen, Implantate oder Lösungen, in Betracht zu ziehen, vorzugsweise ölige oder wässrige Lösungen. Zur topischen Applikation wird der Arzneimittelwirkstoff mit mindestens einem pharmazeutisch annehmbaren Trägerstoff, wie z.B. mikrokristalliner Cellulose, und ggf. weiteren Hilfsstoffen, wie z.B. Feuchtigkeitsspendern, zu auf die Haut auftragbaren festen Formulierungen, wie z.B. Cremes, Gelen, Salben, Pasten, Pulver oder Emulsionen bzw. zu auf die Haut auftragbaren flüssigen Formulierungen, wie z.B. Lösungen, Suspensionen, Lotionen, Seren, Ölen, Sprays oder Aerosole in üblicher Weise formuliert. Vorzugsweise liegt das pharmazeutische Mittel als Injektionslösung vor. Für die Herstellung der Injektionslösung können wässrige Medien, wie z.B. destilliertes Wasser oder physiologische Salzlösungen verwendet werden, wobei letztere saure und basische Additionssalze einschließen. Das pharmazeutische Mittel kann auch als feste Zusammensetzung, beispielsweise im lyophilisierten Zustand vorliegen, und dann durch Zugabe eines auflösenden Mittels, wie z.B. destilliertes Wasser, vor der Verwendung bereitet werden. Die Grundlagen der Herstellung von Lyophilisaten sind dem Fachmann vertraut.

Die Konzentration der aktiven Verbindung in der Formulierung kann 0,1 bis 100 Gewichtsprozente betragen. Entscheidend ist, dass die pharmazeutische Zusammensetzung als Wirkstoff eine effektive Menge der Verbindung zusammen mit den pharmazeutisch verträglichen Hilfsstoffen umfasst. Die Begriffe "effektive Menge" oder "wirksame Dosis" werden hierin austauschbar verwendet und bezeichnen eine Menge des pharmazeutischen Wirkstoffs, die eine prophylaktisch oder therapeutisch relevante Wirkung auf eine Krankheit oder pathologische Veränderung in Zelle, Gewebe, Organ oder Säuger hat. Eine "prophylaktische Wirkung" verhindert das Ausbrechen einer Krankheit oder sogar die Infektion mit einem Pathogen nach dem Eindringen einzelner Vertreter derart, dass deren nachfolgende Ausbreitung stark verringert wird oder sie sogar vollständig inaktiviert werden. Eine "prophylaktische Wirkung" umfasst auch die Erhöhung der normalen physiologischen Funktion. Eine Prophylaxe ist insbesondere ratsam, wenn ein Individuum Veranlagungen für den Ausbruch der vorgenannten Krankheiten aufweist, wie z.B. eine familiäre Vorbelastung, einen Gendefekt oder eine kürzlich überstandene Krankheit. Eine "therapeutisch relevante Wirkung" befreit teilweise oder vollständig von einem, mehreren oder allen Krankheitssymptomen oder führt zur teilweisen oder vollständigen Rückkehr eines, mehrerer oder aller physiologischer oder biochemischer Parameter, die mit der Krankheit oder pathologischen Veränderung in Zusammenhang stehen oder ursächlich dafür sind, in den Normalzustand. Auch wird die Verlaufskontrolle als Art der therapeutischen Behandlung verstanden, wenn die Verbindungen in bestimmten Intervallen verabreicht werden, z.B. um die Symptome einer Krankheit vollständig zu beseitigen. Die jeweilige Dosis bzw. der Dosisbereich für die Gabe der erfindungsgemäßen Verbindungen ist groß genug, um den gewünschten prophylaktischen oder therapeutischen Effekt der Induktion einer biologischen oder medizinischen Antwort zu erreichen. Im Allgemeinen wird die Dosis mit dem Alter, der Konstitution und dem Geschlecht des Patienten variieren sowie die Schwere der Erkrankung berücksichtigen. Es versteht sich, dass die spezifische Dosis, Häufigkeit und Dauer der Verabreichung darüber hinaus von einer Vielzahl an Faktoren abhängen, wie z.B. der Zielsteuerungs- und Bindungsfähigkeit der Verbindungen, Ernährungsgewohnheiten des zu behandelnden Individuums, Art der Verabreichung, Ausscheidungsrate und Kombination mit anderen Medikamenten. Die individuelle Dosis kann sowohl in Bezug auf die primäre Erkrankung als auch in Bezug auf das Eintreten eventueller Komplikationen eingestellt werden. Die exakte Dosis ist durch einen Fachmann mit bekannten Mitteln und Methoden feststellbar. Diese Lehre der Erfindung ist gültig und ohne Einschränkungen auf die pharmazeutische Zusammensetzung umfassend die Verbindungen der Formel (I) anwendbar, sofern es sinnvoll erscheint.

In einer Ausführungsform der Erfindung werden die Verbindungen in einer Dosis von 0,01 mg bis 1 g pro Dosierungseinheit verabreicht, vorzugsweise zwischen 1 bis 700 mg, besonders bevorzugt 5 bis 100 mg. Die tägliche Dosierung liegt insbesondere zwischen 0,02 und 100 mg/kg Körpergewicht.

Zur Unterstützung der medizinischen Wirkung kann die pharmazeutische Zusammensetzung in einer Ausgestaltung der Erfindung auch einen oder mehrere weitere Wirkstoffe umfassen, wobei eine gleichzeitige oder aufeinander folgende Verabreichung denkbar ist. Die therapeutische Wirkung der erfindungsgemäßen pharmazeutischen Zusammensetzung kann beispielsweise darin bestehen, dass durch die Hemmung der DNA-PK als erwünschter Nebeneffekt bestimmte Antikrebsmittel besser wirken oder durch die Verminderung der Dosis die Anzahl der Nebenwirkungen dieser Medikamente reduziert wird.

In einer bevorzugten Ausführungsform der Erfindung wird die erfindungsgemäße pharmazeutische Zusammensetzung mit einem Antikrebsmittel kombiniert. Wie hier verwendet, betrifft der Begriff "Antikrebsmittel" jedes Mittel, das einem Patienten mit Krebs, Tumoren, Metastasen und/oder Störungen der Angiogenese zum Zweck der Behandlung des Krebses verabreicht wird. Das Antikrebsmittel ist besonders bevorzugt ausgewählt aus der Gruppe umfassend Cytokine, Chemokine, pro-apoptotische Mittel, Interferone, radioaktive Verbindungen, Östrogenrezeptor-Modulatoren, Androgenrezeptor-Modulatoren, Retinoidrezeptor-Modulatoren, Zytotoxika, Zytostatika, Prenyl-Proteintransferase-Hemmer und Angiogenese-Hemmer oder Kombinationen davon. Es ist bevorzugt, dass das Antikrebsmittel den Nukleinsäure- und/oder Proteinstoffwechsel, die Zellteilung, DNA-Replikation, Purin-, Pyrimidin- und/oder Aminosäure-Biosynthese, Genexpression, mRNA-Prozessierung, Proteinsynthese, Apoptose oder Kombinationen davon verändert, insbesondere abschwächt.

Die Erfindung kann auch als Kit praktiziert werden, der die erfindungsgemäßen Verbindungen enthält. Das Kit besteht aus getrennten Packungen von (a) einer wirksamen Menge einer Verbindung der Formel (I) und/oder ihrer physiologisch unbedenklichen Salze, Tautomere und/oder Stereoisomere, einschließlich deren Mischungen in allen Verhältnissen, und (b) einer wirksamen Menge eines weiteren Wirkstoffs. Das Kit enthält geeignete Behälter, wie z.B. Schachteln oder Kartons, individuelle Flaschen, Beutel oder Ampullen. Das Kit kann z.B. separate Ampullen enthalten, in denen jeweils eine wirksame Menge an einer Verbindung der Formel (I) und/oder ihrer pharmazeutisch verwendbaren Salze, Tautomere und/oder Stereoisomere, einschließlich deren Mischungen in allen Verhältnissen, und einer wirksamen Menge eines weiteren Arzneimittelwirkstoffs gelöst oder in lyophilisierter Form vorliegt. Das Kit der Erfindung kann auch einen Artikel beinhalten, der schriftliche Anweisungen enthält oder den Anwender auf schriftliche Anweisungen hinweist, die den Umgang mit den Verbindungen der Erfindung erläutern.

Erfindungsgemäß werden die Verbindungen der Formel (I) bzw. deren Teilformeln und/oder ihrer physiologisch unbedenklichen Salze, Tautomere und/oder Stereoisomere, einschließlich deren Mischungen in allen Verhältnissen, zur Prophylaxe, Therapie und/oder Verlaufskontrolle von Erkrankungen verwendet, die durch die Aktivität von Serin-Threonin-Proteinkinasen hervorgerufen, vermittelt und/oder verbreitet werden. Gegenstand der vorliegenden Erfindung ist deshalb auch die Verwendung von Verbindungen der Formel (I) bzw. deren Teilformeln und/oder ihrer physiologisch unbedenklichen Salze, Tautomere und/oder Stereoisomere, einschließlich deren Mischungen in allen Verhältnissen, zur Herstellung eines Arzneimittels zur Prophylaxe, Therapie und/oder Verlaufskontrolle von Erkrankungen, die durch die Aktivität von Serin-Threonin-Proteinkinasen hervorgerufen, vermittelt und/oder verbreitet werden. Erfindungsgemäß sind Verbindungen der Formel (I) bzw. deren Teilformeln und/oder ihre physiologisch unbedenklichen Salze, Tautomere und/oder Stereoisomere, einschließlich deren Mischungen in allen Verhältnissen, zur Verwendung in der Prophylaxe, Therapie und/oder Verlaufskontrolle von Erkrankungen geeignet, die durch Aktivität von Serin-Threonin-Proteinkinasen hervorgerufen, vermittelt und/oder verbreitet werden. Zur Identifizierung eines entsprechenden Signalübertragungswegs und zum Nachweis von Wechselwirkungen zwischen verschiedenen Signalübertragungswegen sind geeignete Modelle oder Modellsysteme entwickelt worden, z.B. Zellkulturmodelle (Khwaja et al. (1997) EMBO 16: 2783) und Modelle transgener Tiere (White et al. (2001) Oncogene 20: 7064). Zur Bestimmung bestimmter Stufen in der Signalübertragungskaskade können wechselwirkende Verbindungen genutzt werden, um das Signal zu modulieren (Stephens et al. (2000) Biochemical J 351: 95). Darüber hinaus können die erfindungsgemäßen Verbindungen auch als Reagenzien zur Testung kinaseabhängiger Signalübertragungswege in Tieren und/oder Zellkulturmodellen oder in den in dieser Anmeldung genannten klinischen Erkrankungen verwendet werden. Wie hierin besprochen, sind diese Signalwege für verschiedene Erkrankungen relevant. Dementsprechend sind die erfindungsgemäßen Verbindungen nützlich bei der Prophylaxe, Therapie und/oder Verlaufskontrolle von Erkrankungen, die von Signalwegen mit Beteiligung von Serin-Threonin-Proteinkinasen abhängig sind.

Erfindungsgemäß sind die Verbindungen der Formel (I) bzw. deren Teilformeln und/oder ihre physiologisch unbedenklichen Salze, Tautomere und/oder Stereoisomere, einschließlich deren Mischungen in allen Verhältnissen, zur Verwendung in der Prophylaxe, Therapie und/oder Verlaufskontrolle von Krebs, Tumoren, Metastasen, Störungen der Angiogenese, retroviralen Erkrankungen und/oder Immunerkrankungen geeignet, insbesondere Krebs, Tumoren, Metastasen und/oder Störungen der Angiogenese. Erfindungsgemäß sind die Verbindungen der Formel (I) bzw. deren Teilformeln und/oder ihre physiologisch unbedenklichen Salze, Tautomere und/oder Stereoisomere, einschließlich deren Mischungen in allen Verhältnissen, auch zur Verwendung bei der Verlangsamung von Alterungsprozessen geeignet, wobei die Verlangsamung anhand des Vergleichs der Lebenspanne des behandelten Wirts oder dessen Zellen, Zellkulturen, Geweben oder Organen mit entsprechenden Positiv- oder Negativkontrollen und/oder Statistiken erfolgt. Es versteht sich, dass auch der Wirt der pharmazeutischen Verbindungen in den Schutzumfang der vorliegenden Erfindung eingeschlossen ist.

Der Tumor ist insbesondere ausgewählt aus der Gruppe von Erkrankungen von Plattenepithel, Blase, Magen, Nieren, Kopf, Hals, Ösophagus, Gebärmutterhals, Schilddrüse, Darm, Leber, Gehirn, Prostata, Urogenitaltrakts, lymphatisches System, Kehlkopf, Lunge, Haut, Blut und Immunsystem, und/oder der Krebs ist ausgewählt aus der Gruppe von Monozytenleukämie, Lungenadenokarzinom, kleinzelliges Lungenkarzinom, Bauchspeicheldrüsenkrebs, Glioblastom, Darmkarzinom, Brustkarzinom, akute myelotische Leukämie, chronische myelotische Leukämie, akute lymphatische Leukämie, chronische lymphatische Leukämie, Hodgkin-Lymphom und non-Hodgkin-Lymphom.

Eine weitere Ausgestaltung der vorliegenden Erfindung betrifft die erfindungsgemäßen Verbindungen in Kombination mit Radiotherapie und/oder mit mindestens einem weiteren Wirkstoff, vorzugsweise in Kombination mit Radiotherapie und/oder einem Antikrebsmittel. Technische Bestrahlungsmethoden, die klinisch Verwendung finden, umfassen vorzugsweise Photonenbestrahlung (klassische, elektromagnetische Röntgen-/Gamma-Strahlung), Protonenbestrahlung, Schwerionenbestrahlung (ionisierter Kohlenstoff) sowie Neutronenbestrahlung, ohne hierauf beschränkt zu sein. Dem Fachmann sind diese Radiotherapien und weitere geeignete Bestrahlungstherapien im Sinne der Erfindung bekannt, wie z.B. aus Herrmann et al. (2006) Klinische Strahlenbiologie, Elsevier München, 4. Auflage, 67-68; Bhide & Nutting (2010) BMC Medicine 8: 25; Choi & Hung (2010) Current Urology Reports 11 (3): 172. Als häufigste Anwendung ist die Photonenbestrahlung technisch durch die Verfahren IMRT (Intensitäts-modulierte Radiotherapie) sowie durch bildgebende Verfahren (dreidimensional konforme Radiotherapie) bei der Bestrahlungsplanung und -durchführung zur möglichst exakten Fokusierung verfeinert worden. Die erfindungsgemäßen Verbindungen erzielen bei existierenden Krebs-Chemotherapien und Bestrahlungen synergistische Effekte und/oder stellen die Wirksamkeit existierender Krebs-Chemotherapien und Bestrahlungen wieder her. Die synergistische Wirkung der Hemmung von VEGF in Kombination mit Radiotherapie ist im Stand der Technik beschrieben (WO 00/61186). Als weitere Arzneimittelwirkstoffe sind besonders solche Chemotherapeutika bevorzugt, die die Angiogenese hemmen und dadurch das Wachstum und die Verbreitung von Tumorzellen inhibieren. Beispiele hierfür sind VEGF-Rezeptorinhibitoren, beinhaltend Ribozyme und Antisense, die auf VEGF-Rezeptoren gerichtet sind, sowie Angiostatin und Endostatin. Weitere Beispiele antineoplastischer Agenzien, die in Kombination mit den erfindungsgemäßen Verbindungen verwendet werden können, beinhalten im Allgemeinen alkylierende Agenzien, Antimetaboliten, Epidophyllotoxin, ein antineoplastisches Enzym, einen Topoisomerase-Inhibitor, Procarbazin, Mitoxantron oder Platin-Koordinations-Komplexe. In einer anderen Ausführungsform ist das Antikrebsmittel besonders bevorzugt ausgewählt aus der Gruppe von Östrogenrezeptor-Modulator, Androgenrezeptor-Modulator, Retinoidrezeptor-Modulator, Zytotoxikum, Zytostatikum, Prenyl-Proteintransferase-Hemmer und Angiogenese-Hemmer. Im Übrigen ist die vorherige Lehre der Erfindung und deren Ausführungsformen betreffend pharmazeutische Zusammensetzung gültig und ohne Einschränkungen auf die zweite medizinische Indikation anwendbar, sofern es sinnvoll erscheint. Eine ganz besonders bevorzugte Ausführungsform umfasst die erfindungsgemäßen Verbindungen in Kombination mit Radiotherapie und/oder einem Zytostatikum.

Noch eine weitere Ausgestaltung der Erfindung bezieht sich auf die Verwendung von mindestens einer Verbindung der Formel (I) und/oder ihrer physiologisch unbedenklichen Salze, Tautomere und/oder Stereoisomere, einschließlich deren Mischungen in allen Verhältnissen, zur Sensibilisierung von Krebszellen gegenüber Antikrebsmittel und/oder ionisierender Strahlung unter der Maßgabe, dass die Sensibilisierung in-vivo nicht am menschlichen oder tierischen Körper erfolgt. Die Sensibilisierung erfolgt vorzugsweise ex-vivo oder in-vitro, indem die Verbindungen Zellen, Zellkulturen, Geweben oder Organen verabreicht werden, die Serin-Threonin-Proteinkinasen umfassen. Die ex-vivo-Verwendung wird insbesondere bei tierischen Zellen angewandt, die aus einem tierischen Organismus stammen, der von einer Krankheit betroffen ist, die ausgewählt ist aus der Gruppe von Krebs, Tumoren, Metastasen und/oder Störungen der Angiogenese. Die ex-vivo-behandelten Zellen können entweder für Folgeuntersuchungen weiter in Kultur gehalten oder in ein Tier überführt werden, wobei es sich um das Wirtstier oder ein anderes Tier handeln kann. Die erfindungsgemäße ex-vivo-Sensibilisierung ist insbesondere von Vorteil, um den spezifische Wirkung der Verbindungen zu testen, so dass unter Auswertung dieser ex-vivo-Daten die in-vivo-Dosis entsprechend voreingestellt werden kann. Im Ergebnis dessen wird der therapeutische Effekt signifikant erhöht.

Die Erfindung lehrt ferner ein Verfahren zur Prophylaxe, Therapie und/oder Verlaufskontrolle von Krebs, Tumoren, Metastasen, Störungen der Angiogenese, retroviralen Erkrankungen, Immunerkrankungen und/oder Alterungsprozessen, wobei eine wirksame Menge mindestens einer erfindungsgemäßen Verbindung und/oder ihrer physiologisch unbedenklichen Salze, Tautomere und/oder Stereoisomere, einschließlich deren Mischungen in allen Verhältnissen, einem zu behandelnden Probanden verabreicht wird. Bevorzugte Probanden im Sinne der Erfindung sind Mensch oder Tier, besonders bevorzugt der Mensch. Dem Fachmann ist hierbei bekannt, dass er die erfindungsgemäßen Verbindungen, die selbstverständlich auch als das erfindungsgemäße pharmazeutische Mittel verwendet werden können, in verschiedenen Dosierungen einem Organismus, insbesondere einem humanen Patienten, applizieren kann. Die wirksame Menge und die Art der Applikation können vom Fachmann durch Routineversuche bestimmt werden. Die vorherige Lehre der Erfindung und deren Ausführungsformen sind gültig und ohne Einschränkungen auf das Behandlungsverfahren anwendbar, sofern es sinnvoll erscheint.

Sämtliche genannte sowie weitere Bestandteile bzw. Komponenten sind dem Fachmann geläufig und können in Routineversuchen eine spezielle Ausgestaltung für die erfindungsgemäße Lehre erfahren. Sämtliche in der Beschreibung zitierten Dokumente sollen hiermit in ihrer Gesamtheit in die Offenbarung der vorliegenden Erfindung als Referenz einbezogen werden.

Im Rahmen der hier vorgestellten Erfindung wurden erstmals neuartige Pyrazolochinolin-Verbindungen der Formel (I) bereitgestellt. Die erfindungsgemäßen Verbindungen steuern affin und/oder selektiv Serin-Threonin-Proteinkinasen, insbesondere DNA-PK, an. Die Verbindungen aus Formel (I) und deren Derivate zeichnen sich durch eine hohe Spezifität und Stabilität, niedrige Herstellungskosten und leichte Handhabung aus. Auf diesen Eigenschaften basieren eine reproduzierbaren Wirkungsweise, einschließlich des Fehlens von Kreuzreaktivitäten, und die verlässliche und sichere Wechselwirkung mit den entsprechenden Zielstrukturen. Die Erfindung beinhaltet auch die Verwendung der vorliegenden Pyrazolochinolin-Derivate zur Hemmung, Regulation und/oder Modulation der Signalkaskade von Serin-Threonin-Proteinkinasen, insbesondere DNA-PK, und bietet damit neuartige Werkzeuge für Forschung und/oder Diagnostik.

Arzneimittel und pharmazeutische Zusammensetzungen, die die genannten Verbindungen enthalten, und der Einsatz dieser Verbindungen zur Behandlung Kinase-vermittelter Störungen sind außerdem ein vielversprechenden Ansatz für ein breites Spektrum von Therapien, wodurch sich bei Mensch und Tier eine direkte und unmittelbare Linderung von Symptomen erreichen lässt. Dies ist besonders für die wirksame Bekämpfung schwerer Krankheiten wie Krebs von Vorteil, entweder als Monotherapie oder in Kombination mit anderen anti-neoplastischen Therapien. Die Schlüsselbeteiligung von DNA-PK an DNA-Reparaturprozessen und der Nachweis, dass der DNA-PK-Inhibitoren Säugerzellen strahlungsempfindlicher werden lässt, ermöglicht eine therapeutische Anwendung der DNA-PK oder DNA-PK/ATM oder ATM spezifischen Inhibitoren im Rahmen der Behandlung von z.B. soliden Krebstumoren durch Bestrahlungs- und/oder einer auf DNA-DSBs abzielenden Chemotherapie. Die Verbindungen der Formel (I), ihre Salze, Isomere, Tautomere, Enantiomere, Diastereomere, Racemate, Derivate, Prodrugs und/oder Metaboliten sind nicht nur bei den genannten klinischen Krankheitsbildern wirkungsvoll, sondern ebenso in der Diagnose und Therapie sämtlicher Erkrankungen in Zusammenhang mit der DNA-PK-Signalkaskade, vor allem im Hinblick auf die Hemmung von Zellproliferation und -migration. Darüber hinaus sind die erfindungsgemäßen Inhibitoren in der Behandlung von retroviralen Erkrankungen durch ein Zurückdrängen der retroviralen Integration nutzbar (R. Daniel (1999) Science 284: 644). Schließlich können die erfindungsgemäßen Hemmstoffe als Immunmodulatoren sowie Modulatoren der telomeren Wartung eingesetzt werden. Die niedermolekularen Inhibitoren werden einzeln und/oder in Kombination mit anderen Behandlungsmaßnahmen verwendet, wie z.B. chirurgischen Eingriffen, Immun-, Strahlen- und/oder Chemotherapie. Letztere beziehen sich auf eine zielgerichtete Therapie mit einem beliebigen NME (d.h. NCE und/oder NBE) als Mono- und/oder On-Target-/Off-Target-Kombinationstherapie.

Aufgrund ihrer überraschend starken und/oder selektiven Hemmung von solchen Enzymen, die über die Reparatur von dsDNA zelluläre Prozesse regeln, können die Verbindungen der Erfindung bei vorteilhaft niedriger Dosierung verabreicht werden, während sie im Vergleich mit den weniger potenten bzw. weniger selektiven Inhibitoren des Stands der Technik eine ähnliche oder sogar überlegene biologische Wirksamkeit erzielen. Die reduzierte Dosis geht auch mit verringerten oder keinen medizinischen Nebenwirkungen einher. Darüber hinaus wird die hoch selektive Hemmung durch die erfindungsgemäßen Verbindungen auch durch eine Verringerung unerwünschter Nebenwirkungen reflektiert, die unabhängig von der Dosierung ist.

Es versteht sich, dass diese Erfindung nicht auf die spezifischen Verbindungen, pharmazeutischen Zusammensetzungen, Verwendungen und Verfahren beschränkt ist, wie sie hierin beschrieben sind, da solche Dinge variieren können. Es versteht sich des Weiteren, dass die vorliegend verwendete Terminologie ausschließlich dem Zweck der Beschreibung besonderer Ausführungsformen dient und nicht den Schutzumfang der Erfindung einschränken soll. Wie vorliegend in der Spezifikation einschließlich der anhängigen Ansprüche verwendet, schließen Wortformen im Singular, wie z. B. "ein", nein", einer", "der" oder "das" die Entsprechung im Plural ein, sofern der Kontext nicht eindeutig etwas anderes vorgibt. Beispielsweise enthält der Bezug auf "eine Verbindung" ein einzelne Verbindung oder mehrere Verbindungen, die wiederum identisch oder verschieden sein können, oder der Bezug auf "ein Verfahren" schließt äquivalente Schritte und Verfahren ein, die dem Fachmann bekannt sind.

Im Folgenden wird die Erfindung anhand nicht limitierender Beispiele für konkrete Ausführungsformen näher erläutert. Die Beispiele sind insbesondere dahingehend zu interpretieren, dass sie nicht auf die konkret veranschaulichten Merkmalskombinationen beschränkt sind, sondern die beispielhaften Merkmale wiederum frei kombiniert werden können, solange die Aufgabe der Erfindung gelöst wird.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

NMR (1 H) wurde mit den folgenden Parametern durchgeführt.
Geräte: Bruker Avance DRX 500, Bruker Avance 400, Bruker DPX 300
Referenz: TMS
TD (Time Domaine = Anzahl der Datenpunkte oder digitale Auflösung): 65536
Lösungsmittel: DMSO d6
NS (Number of Scans = Häufigkeit der Abtastung): 32
SF (Spectrometer Frequence = Sendefrequenz): 500 MHz
TE (Temperatur): 303 K

HPLC-MS wurde mit den folgenden Parametern durchgeführt.
Gerät: Agilent Technologies 1200 Series
Methoden: ESI1ROD.M und POLAR.M (3,8 min., Lösungsmittel-Gradient)
Säule: ChromolithSpeedROD RP18e50-4.6
Lösungsmittel: Acetonitril + 0,05 % HCOOH / VE-Wasser + 0,04 % HCOOH
Detektionswellenlänge: 220 nm
MS-Typ: API-ES

### BEISPIEL 1: Synthese von 4-(8-Hydroxy-7-methoxy-3-methyl-3H-pyrazolo[3,4-c]chinolin-1-yl)-benzonitril

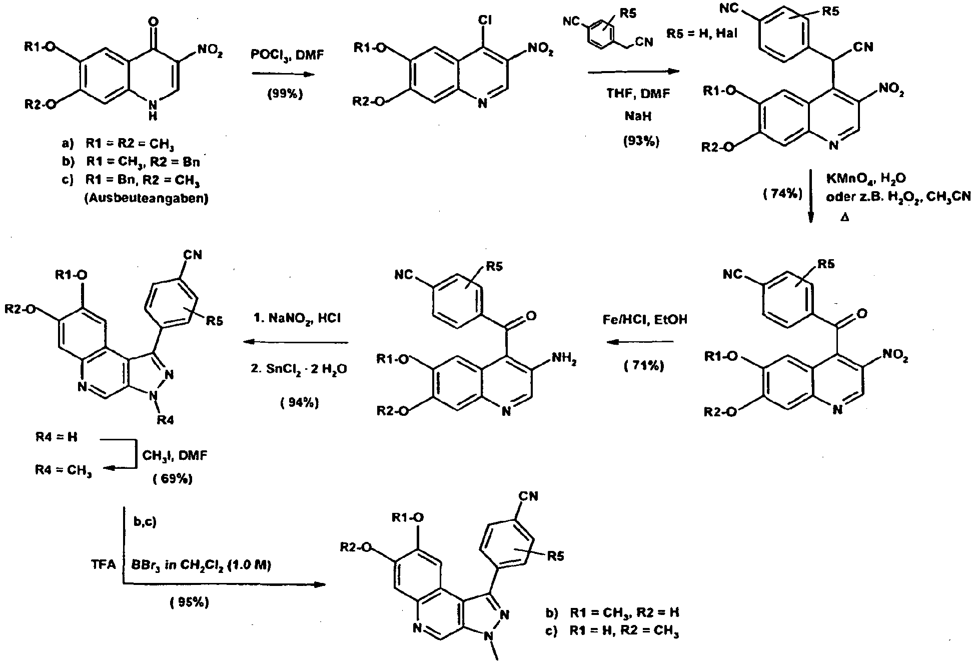

6-Benzyloxy-7-methoxy-3-nitro-1H-chinolin-4-on (9,10 g, 27,89 mmol, vgl. Acta Pharmacologica Sinica (2008) 29(12): 1529) wird in trockenem *N,N*-Dimethylformamid (70 ml) suspendiert. Anschließend wird Phosphorylchlorid (2,82 ml, 30,68 mmol) zugefügt und 30 min auf 100°C erhitzt. Nach dem Abkühlen wird das Reaktionsgemisch unter Rühren auf 500 ml Eiswasser gegeben und weitere 30 min gerührt. Der entstandene Niederschlag wird abgesaugt, mit Wasser gewaschen und im Vakuum getrocknet, wobei 6-Benzyloxy-4-chlor-7-methoxy-3-nitro-chinolin (9,57 g, 27,76 mmol) als hellbeiger Feststoff mit Schmelzpunkt 169.6°C isoliert wird. MS: 345.1 (M+H⁺), DC (HPTLC): R_{f} = 0.44 (Cyclohexan/Essigsäureethylester 2:1 Volumenanteile).

Unter einer Stickstoff-Atmosphäre wird 4-Cyanophenylacetonitril (3,86 g, 27,12 mmol) in trockenem Tetrahydrofuran (185 ml) gelöst. Anschließend wird unter Eisbadkühlung portionsweise Natriumhydrid (60 %-ige Dispersion in Paraffinöl, 2,17 g, 54,25 mmol) zugesetzt und weitere 30 min gerührt. Danach wird eine Suspension von 6-Benzyloxy-4-chlor-7-methoxy-3-nitro-chinolin (9,35 g, 27,12 mmol) in *N,N*-Dimethylformamid (50 ml) bei Raumtemperatur zugegeben und anschließend weitere 2 h gerührt. Nach Beendigung der Reaktion wird auf 2,5 I Wasser gegeben und unter kräftigem Rühren mit 1,0 M Salzsäure neutralisiert. Nach 30 min rühren wird die Suspension auf ca. pH 2 angesäuert und nach weiteren 30 min wird der erhaltene Niederschlag abgesaugt, mit Wasser nachgewaschen und im Hochvakuum über Nacht getrocknet. Anschließend wird das Rohprodukt über Flashkieselgel (Lösungsmittelgradient Cyclohexan / 0-50 vol.% Essigsäureethylester) aufgereinigt, wobei 4-[(6-Benzyloxy-7-methoxy-3-nitro-chinolin-4-yl)-cyano-methyl]-benzonitril (11,31 g, 25,11 mmol) als Feststoff mit einem Schmelzpunkt von 147,9°C erhalten wird. MS: 451.1 (M+H⁺), DC (HPTLC) (HPTLC): R_{f} = 0.68 (Cyclohexan/Essigsäureethylester 1:1 Volumenanteile).

4-[(6-Benzyloxy-7-methoxy-3-nitro-chinolin-4-yl)-cyano-methyl]-benzonitril (5,0 g, 11,1 mmol) wird in Wasser (250 ml) suspendiert und zum Sieden erhitzt. Anschließend wird Kaliumpermanganat (5,26 g, 33,3 mmol) portionsweise so zugegeben, dass die nachfolgende Zugabe erst dann erfolgt, wenn sich die vorherige entfärbt hat (Zeitbedarf: ca. 2.5 h). Danach wird weitere 2 h unter Rückfluss erhitzt. Nach Abkühlung auf ca. 60°C wird abgesaugt. Das wässrige Filtrat wird verworfen, der Nutschkuchen viermal in *N,N-*Dimethylformamid (je 250 ml) suspendiert und abgesaugt (DC (HPTLC) Produktkoritrolle). Die vereinigten DMF Lösungen werden mit 1,0 M Salzsäure auf ca. pH 4 angesäuert (Farbumschlag) und im Vakuum zur Trockne eingeengt, wobei ein Öl erhalten wird. Der Rückstand wird in Tetrahydrofuran (5,0 ml) aufgenommen und anschließend mit Essigsäureethylester (100 ml) versetzt, 30 min gerührt, und anschließend im Eisbad eingekühlt. Der erhaltene Niederschlag wird abgenutscht und im Hochvakuum getrocknet, wobei 4-(6-Benzyloxy-7-methoxy-3-nitro-chinolin-4-carbonyl)-benzonitril (3,61 g, 8,22 mmol) als Feststoff mit einem Schmelzpunkt von 262°C erhalten wird. MS: 440.1 (M+H⁺), DC (HPTLC) (HPTLC): R_{f} = 0.44 (Cyclohexan/Essigsäureethylester 1:1 Volumenanteile).

4-(6-Benzyloxy-7-methoxy-3-nitro-chinolin-4-carbonyl)-benzonitril (8,82 g, 20,07 mmol), Eisenpulver (11,21 g, 200,7 mmol) und 2,0 M Salzsäure (22,76 ml) werden in Methanol (455 ml) suspendiert (Rührmotor) und 18 h auf 66°C erhitzt. Nach Beendigung der Reaktion (DC (HPTLC) Kontrolle) wird über Kieselgur abgesaugt und mit Tetrahydrofuran (500 ml) nachgewaschen. Das Filtrat wird im Vakuum auf die Hälfte seines Volumens eingeengt. Anschließend wird halbgesättigte NaCl-Lösung (300 ml) zugegeben und zweimal mit Essigsäureethylester (je 300 ml) extrahiert. Die vereinigten organischen Phasen werden über Natriumsulfat getrocknet, abgesaugt und im Vakuum zur Trockne eingeengt. Der erhaltene Rückstand wird in Essigsäureethylester gelöst und über wenig Flashkieselgel filtriert. Das Filtrat wird im Vakuum eingeengt, der Rückstand in Essigsäureethylester (70 ml) und Ethanol (20 ml) suspendiert, anschließend abgesaugt und im Hochvakuum getrocknet, wobei 4-(3-Amino-6-benzyloxy-7-methoxy-chinolin-4-carbonyl)-benzonitril (5,83 g, 14,23 mmol) als Feststoff mit einem Schmelzpunkt von 192,6°C erhalten wird. MS: 410.1 (M+H⁺), DC (HPTLC): R_{f} = 0.36 (Essigsäureethylester).

Eine Lösung aus Natriumnitrit (483 mg, 6,99 mmol) in Wasser (2,5 ml) wird bei (-)10°C innerhalb von 1,5 h einer Suspension von 4-(3-Amino-6-benzyloxy-7-methoxy-chinolin-4-carbonyl)-benzonitril (2,60 g, 6,35 mmol) in konzentrierter Salzsäure (50 ml) zugetropft. Anschließend wird weitere 30 min gerührt. Danach wird eine Lösung aus Zinn-(II)-chlorid Dihydrat (5,02 g, 22,23 mmol) in konzentrierter Salzsäure (3,8 ml) bei (-)5°C zugegeben. Die erhaltene Suspension wird 1 h bei Raumtemperatur gerührt, anschließend mit Wasser (500 ml) verdünnt, 30 min gerührt und abgesaugt. Der Filterkuchen wird mit Wasser nachgewaschen und im Hochvakuum getrocknet, wobei 4-(8-Benzyloxy-7-methoxy-3H-pyrazolo[3,4-c]chinolin-1-yl)-benzonitril (2,42 g, 5,95 mmol) als Feststoff mit einem Schmelzpunkt von 222,8°C (Zersetzung) erhalten wird. MS: 407.1 (M+H⁺), DC (HPTLC): R_{f} = 0.27 (Essigsäureethylester).

4-(8-Benzyloxy-7-methoxy-3*H*-pyrazolo[3,4-c]chinolin-1-yl)-benzonitril (2,50 g, 6,15 mmol) wird in *N,N*-Dimethylformamid (72 ml) gelöst. Anschließend werden K₂CO₃ (1,70 g, 12,3 mmol) und lodmethan (421 µl, 6,76 mmol) bei Raumtemperatur zugegeben und die Reaktionsmischung 18 h gerührt (DC (HPTLC) Kontrolle). Danach wird auf Wasser (500 ml) gegeben und 30 min gerührt. Der erhaltene Niederschlag wird abgesaugt und über Flashkieselgel (120 g, Lösungsmittelgradient Cyclohexan / 0-100 vol.% Essigsäureethylester / 0-40 vol.% Ethanol) chromatographiert. Der Rückstand der Produktfraktionen wird in 2-Propanol suspendiert, abgesaugt und im Hochvakuum getrocknet, wobei 4-(8-Benzyloxy-7-methoxy-3-methyl-3H-pyrazolo[3,4-c]chinolin-1-yl)-benzonitril (1,79 g, 4,27 mmol) als Feststoff mit einem Schmelzpunkt von 230,4°C erhalten wird. MS: 421.1 (M+H⁺), DC (HPTLC): R_{f} = 0.44 (Essigsäureethylester/Ethanol 8:1, Volumenanteile). (Anm.: 4-(8-Be,nzyloxy-7-methoxy-3-methyl-3*H*-pyrazolo[3,4-c]chinolin-1-yl)-benzonitril / 4-(8-Benzyloxy-7-methoxy-2-methyl-2*H*-pyrazolo[3,4-c]chinolin-1-yl)-benzonitril ca. 3:1, Rohproduktanteile)

Einer Lösung von 4-(8-Benzyloxy-7-methoxy-3-methyl-3*H*-pyrazolo[3,4-c]chinolin-1-yl)-benzonitril (3,82 g, 9,09 mmol) in Trifluoressigsäure (38 ml) unter einer trockenen Stickstoff-Atmosphäre wird unter Eisbadkühlung langsam eine 1,0 M Bortribromidlösung in Dichlormethan (38,2 ml, 38,2 mmol) zugetropft. Anschließend werden nach beendeter Zugabe weitere 30 min gerührt. Nach vollständiger Reaktion (HPLC-MS Kontrolle) wird die Reaktionsmischung vorsichtig auf Wasser (800 ml) gegeben und zweimal mit Essigsäureethylester (je 200 ml) extrahiert. Die vereinigten organischen Phasen werden mit Wasser (150 ml) und halbgesättigter NaHCO₃-Lösung (200 ml) gewaschen, anschließend mit Na₂SO₄ getrocknet und abgesaugt. Das Filtrat wird im Vakuum eingeengt, in wenig Ethanol suspendiert, abgesaugt und im Hochvakuum getrocknet, wobei die Titelverbindung 4-(8-Hydroxy-7-methoxy-3-methyl-3*H*-pyrazolo[3,4-c]chinolin-1-yl)-benzonitril (2,86 g, 8,66 mmol) als Feststoff mit einem Schmelzpunkt von 288,9°C erhalten wird. MS: 331.1 (M+H⁺), DC (HPTLC): R_{f} = 0.34 (Essigsäureethylester/Ethanol 8:1, Volumenanteile).

Verbindungen, die entsprechend Beispiel 1 hergestellt werden, finden sich in der nachfolgenden Tabelle 2.

**Tab. 2 Verbindungen der Formeln (I), (IA) und (IE)**

| **Nr.** | **Strukturformel** | **Name** | **Analytik** | **IC₅₀ DNA-PK [µM]** |
|---|---|---|---|---|
| 1 | 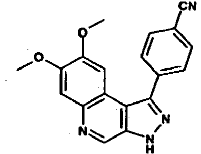 | 4-(7,8-Dimethoxy-3*H-*pyrazolo[3,4-c]chinolin-1-yl)-benzonitril | MS: 330,9 (M+H⁺), DC (HPTLC): R_{f} = 0,37 (Essigsäureethylester) | < 0,1 |
| 2 | 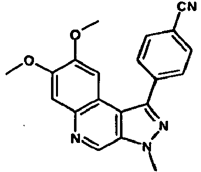 | 4-(7,8-Dimethoxy-3-methyl-3*H*-pyrazolo[3,4-c]chinolin-1-yl)-benzonitril | MS: 345,2 (M+H⁺), DC (HPTLC): R_{f} = 0,21 (Essigsäureethylester) | < 0,1 |
| 3 | 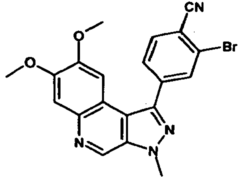 | 2-Brom-4-(7,8-dimethoxy-3*H*-pyrazolo[3,4-c]chinolin-1-yl)-benzonitril | MS: 423.01425,0 (M+H⁺), DC (HPTLC): R_{f} = 0,44 (Essigsäure-ethylester/Ethanol 8:1, Volumenanteile) | < 0,1 |
| 4 | 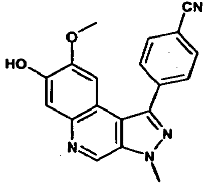 | 4-(7-Hydroxy-8-methoxy-3-methyl-3*H*-pyrazolo[3.4-c]chinolin-1-yl)-benzonitril | MS: 331,1 (M+H⁺), DC (HPTLC): R_{f} = 0,44 (Essigsäureethylester/Ethanol 8:1, Volumenanteile) | < 0,1 |
| 5 | 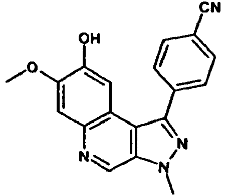 | 4-(8-Hydroxy-7-methoxy-3-methyl-3*H*-pyrazolo[3,4-c]chinofin-1-yl)-benzonitril | ¹H NMR (400 MHz, DMSO) δ = 9.43 (s, 1H), 8.09-8.07 (d, J = 8 Hz, 2H), 7.93 - 7.91 (d, J = 8 Hz, 2H), 7.62 (s, 1H), 7.40 (s, 1H), 4.33 (s, 3H), 3.95 (s, 3H). | < 0,1 |
| 6 | 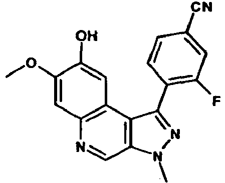 | 3-Fluor-4-(8-hydroxy-7-methoxy-3-methyl-3H-pyrazolo[3.4-c]chinolin-1-yl)-benzonitril | MS: 349,1 (M+H⁺), DC (HPTLC): R_{f} = 0,33 (Essigsäure-ethylester/Ethanol 8:1, Volumenanteile) | < 0,1 |
| 7 | 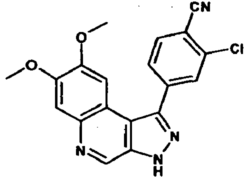 | 2-Chlor-4-(7,8-dimethoxy-3H-pyrazolo[3,4-c]chinolin-1-yl)-benzonitril | MS: 365,0 (M+H⁺, Monochlorisotopenverteilung), DC (HPTLC): R_{f} = 0,49 (Essigsäureethylester) | < 0,1 |

### BEISPIEL 2: Synthese von 1-Brom-7,8-dimethoxy-3-methyl-3H-pyrazolo[3,4-c]chinolin

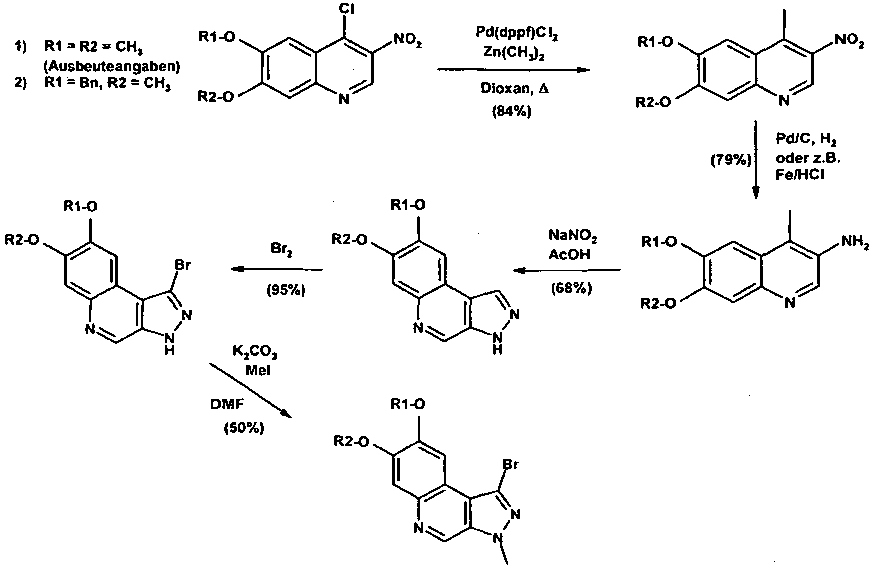

Einer Lösung von 4-Chlor-6,7-dimethoxy-3-nitro-chinolin (7,5 g, 27,92 mmol) in wasser- und Sauerstoff-freiem Dioxan (200 ml, Vorbehandlung: 30 min Stickstoffgas-Durchleitung) werden Zn(CH₃)₂(1,2 M in Toluol, 15,35 ml, 18,43 mmol) und Pd(dppf)Cl₂ (2,28g, 2,92 mmol) zugefügt. Die Reaktionsmischung wird für 8 h auf 80°C erhitzt, wobei eine Lösung erhalten wird. Nach dem Abkühlen auf Raumtemperatur wird langsam Wasser (65 ml) zugegeben und mit Essigsäureethylester (60 ml) extrahiert. Nach Abtrennung wird die organische Phase mit wenig Salzsäure (1,0 M, 5 ml) gewaschen. Die wässrige Phase wird dreimal mit Essigsäureethylester extrahiert (je 60 ml). Die vereinten organischen Phasen werden über Na₂SO₄ getrocknet, abgesaugt und im Vakuum zur Trockne eingeengt. Der Rückstand wird über Flashkieselgel chromatographisch aufgereinigt (Lösungsmittel Cyclohexan / Essigester 2:1, Volumenanteile), wobei 6,7-Dimethoxy-4-methyl-3-nitro-chinolin (5,82 g, 23,44 mmol) als Feststoff erhalten wird. MS: 249.0 (M+H⁺), DC (HPTLC): R_{f} = 0.45 (Cyclohexan/Essigsäureethylester 1:1, Volumenanteile).

6,7-Dimethoxy-4-methyl-3-nitro-chinolin (5,80 g, 23,36 mmol) wird in Tetrahydrofuran (60 ml) gelöst. Anschließend wird Palladium auf Kohle (5%, 2,90 g, wasserfeucht) zugefügt und die Suspension 16 h in einer Wasserstoff-Atmosphäre bei Raumtemperatur turbulent gerührt. Nach Beendigung der Reaktion wird über Kieselgur abgesaugt und mit Tetrahydrofuran nachgewaschen. Das Filtrat wird im Vakuum zur Trockne eingeengt, wobei 3-Amino-6,7-dimethoxy-4-methyl-chinolin (4,05 g, 18,56 mmol) als Feststoff erhalten wird. MS: 219,0 (M+H⁺), DC (HPTLC): R_{f} = 0.35 (Essigsäureethylester/Ethanol 8:1, Volumenanteile).

Einer Lösung von 3-Amino-6,7-dimethoxy-4-methyl-chinolin (2,70 g, 12,39 mmol) in Eisessig (90 ml) wird Natriumnitrit (1,28 g, 18,60 mmol), gelöst in Wasser (2,5 ml) zugefügt. Die Reaktionsmischung wird 3 h bei Raumtemperatur gerührt. Nach Beendigung der Reaktion wird im Vakuum eingeengt. Der Rückstand wird in Wasser aufgenommen, filtriert und mit Wasser nachgewaschen. Das Filtrat wird im Vakuum zur Trockne eingeengt. Der erhaltene Rückstand wird über wenig Flashkieselgel in einer Fritte mehrfach mit Cyclohexan/Essigsäureethylester 4:1 (Volumenanteile) zur Abtrennung von Verunreinigungen abgesaugt. Das Filtrat wird verworfen. Anschließend wird der Nutschkuchen mit Essigsäureethylester/0-10 vol.% Ethanol gewaschen und das Filtrat im Vakuum zur Trockne eingeengt, wobei 7,8-Dimethoxy-3*H*-pyrazolo[3,4-c]chinolin (2,29 g, Reinheit 85%, 8,48 mmol) als Feststoff erhalten wird. MS: 230,0 (M+H⁺), DC (HPTLC): R_{f} = 0.45 (Essigsäureethylester/Ethanol 8:1, Volumenanteile).

7,8-Dimethoxy-3*H*-pyrazolo[3,4-c]chinolin (500 mg, 2,18 mmol) wird in Wasser (40 ml) gelöst. Anschließend wird Brom (220 µl, 4.36 mmol) unter Lichtausschluss bei Raumtemperatur zugetropft. Danach wird die Reaktionslösung 1 h gerührt. Nach Beendigung der Reaktion wird im Vakuum zur Trockne eingeengt, wobei 1-Brom-7,8-dimethoxy-3*H*-pyrazolo[3,4-c]chinolin (752 mg, Reinheit 85%, 2,07 mmol) als Feststoff erhalten wird. MS: 308,0/310,0 (M+H⁺, Monobromisotopenverteilung), DC (HPTLC): R_{f} = 0.50 (Essigsäureethylester/Ethanol 8:1, Volumenanteile).

Einer Lösung von 1-Brom-7,8-dimethoxy-3*H*-pyrazolo[3,4-c]chinolin (300 mg, 972 µmol) in wasserfreiem *N,N*-Dimethylformamid (36 ml) wird Kaliumcarbonat (270 mg, 1,95 mmol) und Methyliodid (78 µl, 1,27 mmol) bei Raumtemperatur zugefügt. Die Reaktionsmischung wird anschließend 18 h bei Raumtemperatur gerührt. Nach Beendigung der Reaktion wird auf Wasser (150 ml) gegossen und mit Essigsäureethylester (150 ml) verdünnt. Die Phasen werden getrennt und die wässrige Phase mehrfach mit Essigsäureethylester extrahiert. Die vereinigten organischen Phasen werden über Na₂SO₄ getrocknet, abgesaugt und das Filtrat im Vakuum zur Trockne eingeengt, wobei 282 mg (876 µmol) einer Mischung aus der Titelverbindung (R_{f} = 0,45) und 1-Brom-7,8-dimethoxy-2-methyl-2*H*-pyrazolo[3,4-c]chinolin (R_{f} = 0,55) im Mengenverhältnis 3:1 erhalten werden. Zur Aufreinigung wird das Regioisomerengemisch in heißem Essigsäureethylester so gelöst, dass ein minimales Lösungsmittelvolumen für die vollständige Lösung ausreichend ist. Anschließend wird langsam auf 0°C gekühlt, wobei ein Niederschlag reinen 1-Brom-7,8-dimethoxy-3-methyl-3*H-*pyrazolo[3,4-c]chinolins (125 mg) erhalten wird. Nach dem Absaugen wird das im Vakuum eingeengte Filtrat (Regioisomerengemisch ca. 1:1) erneut in heißem Essigsäureethylester gelöst und der Lösung wenig Cyclohexan zugefügt. Nach 24 h wird der erhaltene Niederschlag abgesaugt, wobei weiteres 1-Brom-7,8-dimethoxy-3-methyl-3*H*-pyrazolo[3,4-c]chinolin (31 mg) erhalten wird. Die vereinten Kristallisate werden im Hochvakuum getrocknet, wobei die Titelverbindung 1-Brom-7,8-dimethoxy-3-methyl-3*H*-pyrazolo[3,4-c]chinolin (156 mg, 484 µmol) mit einem Schmelzpunkt von 235,5°C erhalten wird.
MS: 322,0/324,0 (M+H⁺, Monobromisotopenverteilung), DC (HPTLC): R_{f} = 0.45 (Essigsäureethylester/Ethanol 8:1, Volumenanteile).

Verbindungen, die entsprechend der Synthesevorschriften aus Beispiel 1 und 2 hergestellt werden, finden sich in der nachfolgenden Tabelle 3.

**Tab. 3 Verbindungen der Formel (III)**

| **Nr**. | **Strukturformel** | **Name** | **Analytik** |
|---|---|---|---|
| 3-1 | 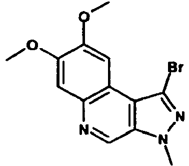 | 1-Brom-7,8-dimethoxy-3-methyl-3*H-*pyrazolo[3,4-c]chinolin | MS: 322,0/324,0 (M+H⁺) |
| | | | DC (HPTLC): R_{f} = 0,45 (Essigsäure-ethylester/Ethanol 8:1, Volumenanteile) |
| 3-2 | 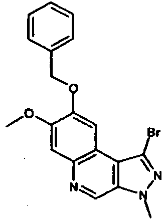 | 8-Benzyloxy-1-brom-7-methoxy-3-methyl-3*H*-pyrazolo[3,4-clchinolin | MS: 398,0/400,0 (M+H⁺) |
| | | | DC (HPTLC): R_{f} = 0,62 (Essigsäure-ethylester/Ethanoi 5:1, Volumenanteile) |

### BEISPIEL 3: Synthese von 2-[1-(4-Cyano-phenyl)-7-methoxy-3-methyl-3H-pyrazolo[3,4-c]chinolin-8-yloxy]-2-(4-fluor-phenyl)-acetamid

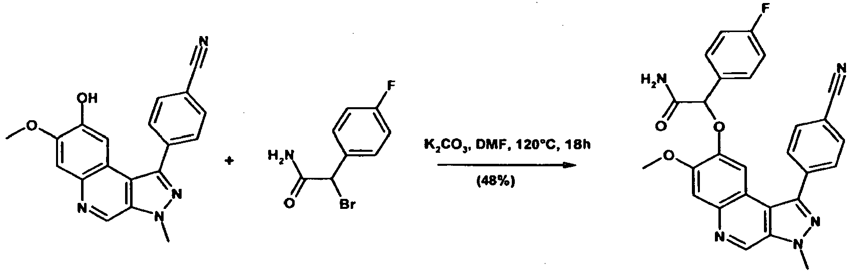

4-(8-Hydroxy-7-methoxy-3-methyl-3*H*-pyrazolo[3,4-c]chinolin-1-yl)-benzonitril (97,7 mg, 296 µmol) wird in *N,N-*Dimethylformamid (3,7 ml) gelöst. Anschließend werden Kaliumcarbonat (102 mg, 738 µmol) und 2-Brom-2-(4-fluor-phenyl)-acetamid (170 mg, 733 µmol) zugefügt. Die Reaktionsmischung wird 18 h bei 120°C gerührt. Nach Beendigung der Reaktion wird auf Wasser (50 ml) gegossen und dreimal mit Essigsäureethylester (je 50 ml) extrahiert. Die vereinigten organischen Phasen werden über Na₂SO₄ getrocknet, abgesaugt und im Vakuum eingeengt. Danach wird der Rückstand in wenig 2-Propanol suspendiert, abgesaugt und im Hochvakuum getrocknet, wobei 2-[1-(4-Cyano-phenyl)-7-methoxy-3-methyl-3*H*-pyrazolo[3,4-c]chinolin-8-yloxy]-2-(4-fluor-phenyl)-acetamid (68 mg, 141 µmol) als Feststoff mit einem Schmelzpunkt von 249,2°C erhalten wird. MS: 482.1 (M+H⁺), DC (HPTLC): R_{f} = 0.31 (Essigsäureethylester/Ethanol 8:1, Volumenanteile). Verbindungen, die methodisch nach der Synthesevorschrift aus Beispiel 3 hergestellt werden, finden sich in der nachfolgenden Tabelle 4.

**Tab. 4 Verbindungen der Formeln (I), (IA) und (IE)**

| **Nr.** | **Strukturformel** | **Name** | **Analytik** | **IC₅₀ DNA-PK [µM]** |
|---|---|---|---|---|
| 8 | 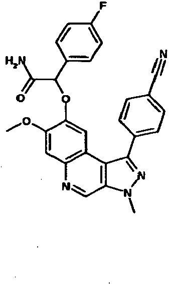 | 2-[1-(4-Cyano-phenyl)-7-methoxy-3-methyl-3*H-*pyrazolo[3,4-c]chinolin-8-yloxy]-2-(4-fluor-phenyl)-acetamid | ¹H NMR (400 MHz, DMSO) δ = 9.30 (s, 1H), 8.08 (d, *J*=8.4, 2H), 7.88 (d, *J*=8.4, 2H), 7.68 (s, 1H), 7.59 (s, 1H), 7.49 (s, 1H), 7.47-7.39 (m, 2H), 7.36 (s, 1H), 7.18 (d, *J*=8.9, 1H), 7.16 (d, *J*=8.9, 1H), 5.44 (s, 1H), 4.30 (s, 3H), 3.97 (s, 3H). | < 0,1 |
| | | | MS:482,1 (M+H)⁺ | |
| | | | DC (HPTLC): R_{f} = 0,31 (Essigsäure-ethylester/Ethanol 1:1, Volumenanteile) | |
| 9 | 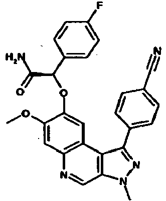 | (*R*)-2-[1-(4-Cyano-phenyl)-7-methoxy-3-methyl-3*H-*pyrazolo[3,4-c]chinolin-8-yloxy]-2-(4-fluor-phenyl)-acetamid | MS: 482.1 (M+H⁺) | < 0,1 |
| | | | [α]²⁰_{D} = -184 (c 4 mg/2 ml THF) | |
| 10 | 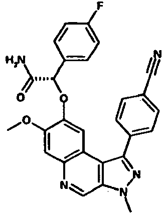 | (*S*)-2-[1-(4-Cyano-phenyl)-7-methoxy-3-methyl-3*H-*pyrazolo[3,4-c]chinolin-8-yloxy]-2-(4-fluor-phenyl)-acetamid | MS: 482.1 (M+H⁺) | < 0,1 |
| | | | [α]²⁰_{D} = +130 (c 4 mg/2 ml THF) | |
| 11 | 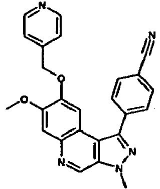 | 4-[7-Methoxy-3-methyl-8-(pyridin-4-ylmethoxy)-3H-pyrazolo[3,4-c]chinolin-1-yl]-benzonitril | MS: 421,8 (M+H)⁺ | < 0,1 |
| | | | DC (HPTLC): R_{f} = 0,41 (Ethanol) | |
| 12 | 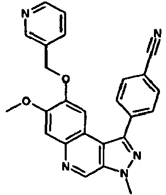 | 4-[7-Methoxy-3-methyl-8-(pyridin-3-ylmethoxy)-3H-pyrazolo[3,4-c]chinolin-1-yl]-benzonitril | MS: 421,8 (M+H)⁺ | < 0,1 |
| | | | DC (HPTLC): R_{f} = 0,49 (Essigsäure-ethylester/Ethanol 1:1, Volumenanteile) | |
| 13 | 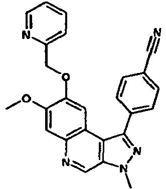 | 4-[7-Methoxy-3-methyl-8-(pyridin-2-ylmethoxy)-3H-pyrazolo[3,4-c]chinolin-1-yl]-benzonitril | MS: 422,1 (M+H)⁺ | > 0,5 |
| | | | DC (HPTLC): R_{f} = 0,35 (Essigsäure-ethylesterlEthanol 8:1, Volumenanteile) | |
| 14 | 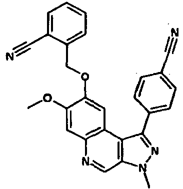 | 4-[8-(2-Cyano-benzyloxy)-7-methoxy-3-methyl-8-3H-pyrazolo[3,4-c]chinolin-1-yl]-benzonitril | MS: 446,1 (M+H)⁺ | 0,1 - 0,5 |
| | | | DC (HPTLC): R_{f} = 0,45 (Essigsäureethylester/Ethanol 8:1, Volumenanteile) | |
| 15 | 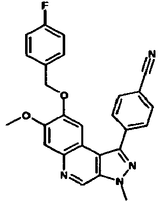 | 4-[8-(4-Fluor-benzyloxy)-7-methoxy-3-methyl-3H-pyrazolo[3,4-c]chinolin-1-yl]-benzonitril | MS: 439,1 (M+H)⁺ | < 0,1 |
| | | | DC (HPTLC): R_{f} = 0,44 (Essigsäureethylester/Ethanol 8:1, Volumenanteile) | |
| 16 | 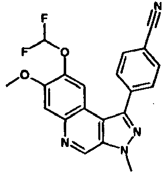 | 4-(8-Difluormethoxy-7-methoxy-3-methyl-3H-pyrazolo[3,4-c]chinolin-1-yl)-benzonitril | MS: 381,1 (M+H)⁺ | < 0,1 |
| | | | DC (HPTLC): R_{f} = 0,37 (Essigsäureethylester/Ethanol 8:1, Volumenanteile) | |
| 17 | 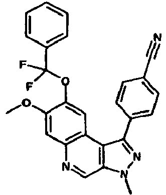 | 4-[8-(Difluorphenyl-methoxy)-7-methoxy-3-methyl-3H-pyrazolo[3,4-c]chinolin-1-yl]-benzonitril | MS: 457,1 (M+H)⁺ | 0,1 - 0,5 |
| | | | DC (HPTLC): R_{f} = 0,47 (Essigsäure-ethylester/Ethanol 8:1, Volumenanteile) | |
| 18 | 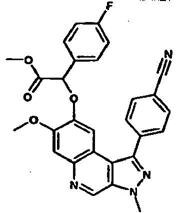 | 1-(4-Cyano-phenyl)-7-methoxy-3-methyl-3H-pyrazolo[3,4-c]chinolin-8-yloxy]-(4-fluorphenyl)-essigsäuremethylester | MS: 497,1 (M+H)⁺ | 0,1 - 0,5 |
| | | | DC (HPTLC): R_{f} = 0,47 (Essigsäure-ethylester/Ethanol 8:1, Volumenanteile) | |
| 19 | 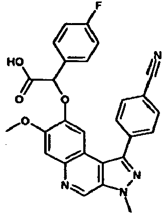 | [1-(4-Cyano-phenyl)-7-methoxy-3-methyl-3H-pyrazolo[3,4-c]chinolin-8-yloxy]-(4-fluor-phenyl)-essigsäure | MS: 483,1 (M+H)⁺ | < 0,1 |
| | | | DC (HPTLC): R_{f} = 0,11 (Ethanol) | |
| 20 | 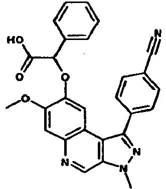 | [1-(4-Cyano-phenyl)-7-methoxy-3-methyl-3H-pyrazolo[3,4-c]chinolin-8-yloxy]-phenyl-essigsäure | MS: 451,1 (M+H)⁺ | < 0,1 |
| | | | DC (HPTLC): R_{f} = 0,20 (Essigsäureethylester/Ethanol 1:1, Volumenanteile) | |
| 21 | 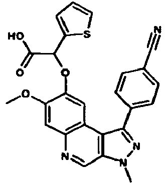 | [1-(4-Cyano-phenyl)-7-methoxy-3-methyl-3H-pyrazolo[3,4-c]chinolin-8-yloxy]-thiophen-2-yl-essigsäure | MS: 471,1 (M+H)⁺ | < 0,1 |
| | | | DC (HPTLC): R_{f} = 0,35 (Essigsäure-ethylester/Ethanol 1:1, Volumenanteile) | |
| 22 | 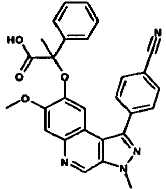 | 2-[1-(4-Cyano-phenyl)-7-methoxy-3-methyl-3H-pyrazolo[3,4-c]chinolin-8-yloxy]-2-phenyl-propansäure | MS: 479,2 (M+H)⁺ | > 0,5 |
| | | | DC (HPTLC): R_{f} = 0,44 (Ethanol) | |
| 23 | 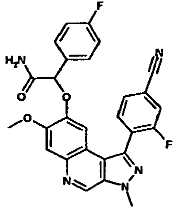 | 2-[1-(4-Cyano-2-fluor-phenyl)-7-methoxy-3-methyl-3H-pyrazolo[3,4-c]chinolin-8-yloxy]-2-(4-fluor-phenyl)-acetamid | MS: 457,1 (M+H)⁺ | < 0,1 |
| | | | DC (HPTLC): R_{f} = 0,27 (Essigsäureethylester/Ethanol 8:1, Volumenanteile) | |
| 24 | 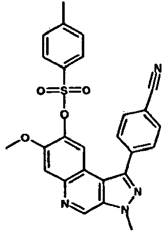 | Toluol-4-sulfonsäure-[1-(4-cyano-phenyl)-7-methoxy-3-methyl-3H-pyrazolo[3,4-c]chinolin-8-yl]-ester | MS: 485,1 (M+H)⁺ | 0,1 - 0, 5 |
| | | | DC (HPTLC): R_{f} = 0,64 (Essigsäure-ethylester/Ethanol 1:1, Volumenanteile) | |
| 25 | 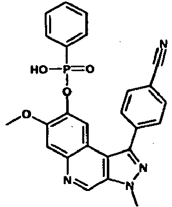 | Phenyl-phosphonsäure-[1-(4-cyano-phenyl)-7-methoxy-3-methyl-3H-pyrazolo[3,4-c]chinolin-8-yl]-ester | MS: 471,1 (M+H)⁺ | 0,1 - 0,5 |
| | | | DC (HPTLC): R_{f} = 0,68 (Ethanol) | |
| 26 | 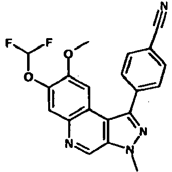 | 4-(7-Difluormethoxy-8-methoxy-3-methyl-3H-pyrazolo[3,4-c]chinolin-1-yl)-benzonitril | MS: 381,1 (M+H)⁺ | > 0,5 |
| | | | DC (HPTLC): R_{f} = 0,64 (Essigsäureethylester/Ethanol 8:1, Volumenanteile) | |
| 27 | 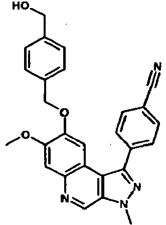 | 4-[8-(4-Hydroxymethyl-benzyloxy)-7-methoxy-3-methyl-3H-pyrazolo[3,4-c]chinolin-1-yl]-benzonitril | MS: 451,1 (M+H)⁺ | < 0,1 |
| | | | DC (HPTLC): R_{f} = 0,34 (Essigsäure-ethylester/Ethanol 8:1, Volumenanteile) | |
| 28 | 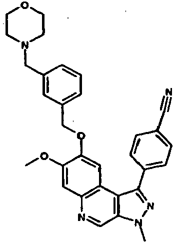 | 4-[7-Methoxy-3-methyl-8-(3-morpholin-4-ylmethyl-benzyloxy)-3H-pyrazolo[3,4-c]chinolin-1-yl]-benzonitril | MS: 520,2 (M+H)⁺ | 0,1 - 0,5 |
| | | | DC (HPTLC): R_{f} = 0,36 (Essigsäure-ethylester/Ethanol 8:1, Volumenanteile) | |
| 29 | 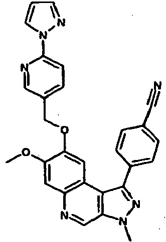 | 4-[7-Methoxy-3-methyl-8-(6-pyrazol-1-yl-pyridin-3-ylmethoxy)-3H-pyrazolo[3,4-c]chinolin-1-yl]-benzonitril | MS: 488,2 (M+H)⁺ | > 0,5 |
| | | | DC (HPTLC): R_{f} = 0,36 (Essigsäure-ethylester/Ethanol 8:1, Volumenanteile) | |
| 30 | 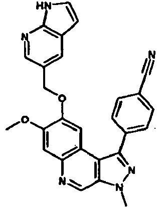 | 4-[7-Methoxy-3-methyl-8-(1 H-pyrrolo[2,3-b]pyridin-5-ylmethoxy)-3H-pyrazolo[3,4-c]chinolin-1-yl]-benzonitril | MS: 461,1 (M+H)⁺ | 0,1 - 0,5 |
| | | | DC (HPTLC): R_{f} = 0,27 (Essigsäureethylester/Ethanol 8:1, Volumenanteile) | |
| 31 | 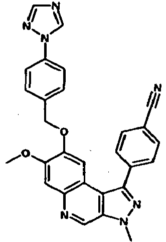 | 4-[7-Methoxy-3-methyl-8-(4-[1,2,4]triazol-1-yl-benzyloxy)-3H-pyrazolo[3,4-c]chinolin-1-yl]-benzonitril | MS: 488,2 (M+H)⁺ | > 0,5 |
| | | | DC (HPTLC): R_{f} = 0,23 (Essigsäure-ethylester/Ethanol 8:1, Volumenanteile) | |
| 32 | 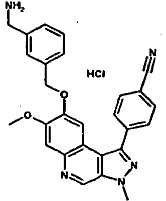 | 4-[8-(3-Aminomethyl-benzyloxy)-7-methoxy-3-methyl-3H-pyrazolo[3,4-c]chinolin-1-yl]-benzonitril-hydrochlorid | MS: 450,5 (M+H)⁺ | 0,1 - 0,5 |
| | | | DC (HPTLC): R_{f} = 0,23 (Methanol/ Hünigsbase) 99:1, Volumenanteile) | |
| 33 | 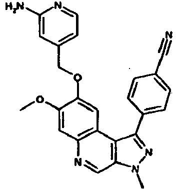 | 4-[8-(2-Amino-pyridin-4-ylmethoxy)-7-methoxy-3-methyl-3H-pyrazolo[3,4-c]chinolin-1-yl]-benzonitril | MS: 437,1 (M+H)⁺ | < 0,1 |
| | | | DC (HPTLC): R_{f} = 0,13 (Essigsäureethylester/Ethanol 8:1, Volumenanteile) | |
| 34 | 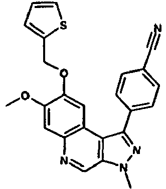 | 4-[7-Methoxy-3-methyl-8-(thiophen-2-ylmethoxy)-3H-pyrazolo[3,4-c]chinolin-1-yl]-benzonitril | MS: 427,1 (M+H)⁺ | < 0,1 |
| | | | DC (HPTLC): R_{f} = 0,43 (Essigsäureethylester/Ethanol 8:1, Volumenanteile) | |
| 35 | 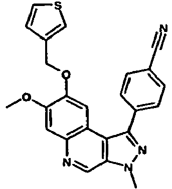 | 4-[7-Methoxy-3-methyl-8-(thiophen-3-ylmethoxy)-3H-pyrazolo[3,4-c]chinolin-1-yl]-benzonitril | MS: 427,1 (M+H)⁺ | < 0,1 |
| | | | DC (HPTLC): R_{f} = 0,41 (Essigsäureethylester/Ethanol 8:1, Volumenanteile) | |
| 36 | 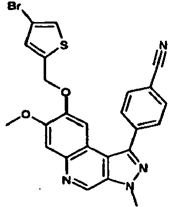 | 4-[8-(4-Brom-thiophen-2-ylmethoxy)-7-methoxy-3-methyl-3H-pyrazolo[3,4-c]chinolin-1-yl]-benzonitril | MS: 505,0/507,0 (M+H)⁺ | < 0,1 |
| | | | DC (HPTLC): R_{f} = 0,56 (Essigsäureethylester/Ethanol 8:1, Volumenanteile) | |
| 37 | 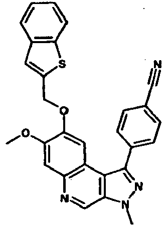 | 4-[8-(Benzo[b]thiophen-2-ylmethoxy)-7-methoxy-3-methyl-3H-pyrazolo[3,4-c]chinolin-1-yl]-benzonitril | MS: 477,1 (M+H)⁺ | > 0,5 |
| | | | DC (HPTLC): R_{f} = 0,52 (Essigsäureethylester/Ethanol 8:1, Volumenanteile) | |
| 38 | 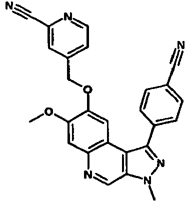 | 4-[1-(4-Cyano-phenyl)-7-methoxy-3-methyl-3H-pyrazolo[3,4-c]chinolin-8-yloxymethyl]-pyridin-2-nitril | MS: 447,1 (M+H)⁺ | 0,1 - 0,5 |
| | | | DC (HPTLC): R_{f} = 0,53 (Essigsäure-ethylester/Ethanol 8:1, Volumenanteile) | |
| 39 | 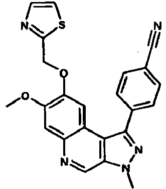 | 4-[7-Methoxy-3-methyl-8-(thiazol-2-ylmethoxy)-3H-pyrazolo[3,4-c]chinolin-1-yl]-benzonitril | MS: 428,1 (M+H)⁺ | < 0,1 |
| | | | DC (HPTLC): R_{f} = 0,42 (Essigsäureethylester/Ethanol 8:1, Volumenanteile) | |

### BEISPIEL 4: Synthese von 4-[8-(2-Dimethylamino-ethoxy)-7-methoxy-3-methyl-3H-pyrazolo[3,4-c]chinolin-1-yl]-benzonitril

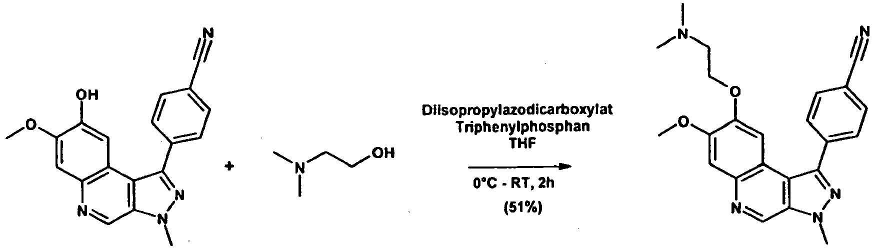

4-(8-Hydroxy-7-methoxy-3-methyl-3*H*-pyrazolo[3,4-c]chinolin-1-yl)-benzonitril (132 mg, 398 µmol) wird in Tetrahydrofuran (3,3 ml) gelöst. Anschließend werden Triphenylphosphan (251 mg, 956 µmol) und 2-(Dimethylamino)-ethanol (56 µl, 558 µmol) zugefügt. Unter Kühlung wird danach bei 5°C Diisopropylazodicarboxylat (125 µl, 637 µmol) zugetropft. Nach beendeter Zugabe wird weitere 2 h bei Raumtemperatur gerührt. Anschließend wird die Reakionslösung im Vakuum zur Trockne eingeengt und über Flashkieselgel (Lösemittelgradient Cyclohexan / 0-100 vol.% Essigsäureethylester / 0-40 vol.% Ethanol) chromatographiert, wobei nach Einengen der Produktfraktionen und Trocknung im Hochvakuum die Titelverbindung 4-[8-(2-Dimethylamino-ethoxy)-7-methoxy-3-methyl-3*H-*pyrazolo[3,4-c]chinolin-1-yl]-benzonitril (81 mg, 202 µmol) als Feststoff mit einem Schmelzpunkt von 212,2°C erhalten wird. MS: 401.9 (M+H⁺), DC (HPTLC): R_{f} = 0.09 (Essigsäureethylester/Ethanol 1:1, Volumenanteile).

Verbindungen, die methodisch entsprechend der Synthesevorschrift aus Beispiel 4 hergestellt werden, finden sich in der nachfolgenden Tabelle 5.

**Tab. 5 Verbindungen der Formeln (I), (IA) und (IE)**

| **Nr.** | **Strukturformel** | **Name** | **Analytik** | **IC₅₀ DNA-PK [µM]** |
|---|---|---|---|---|
| 40 | 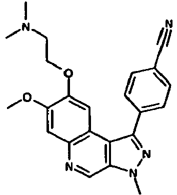 | 4-[8-(2-Dimethylamino-ethoxy)-7-methoxy-3-methyl-3*H*-pyrazolo[3,4-c]chinolin-1-yl]-benzonitril | MS: 401.9 (M+H)⁺ | > 0,1 |
| | | | DC (HPTLC): R_{f} = 0,09 (Essigsäureethylester/Ethanol 1:1, Volumenanteile) | |
| 41 | 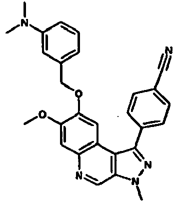 | 4-[8-(3-Dimethylamino-benzyloxy)-7-methoxy-3-methyl-3H-pyrazolo[3,4-c]chinolin-1-yl]-benzonitril | MS: 464.2 (M+H)⁺ | 0,1 - 0,5 |
| | | | DC (HPTLC): R_{f} = 0,36 (Essigsäureethylester/Ethanol 8:1, Volumenanteile) | |
| 42 | 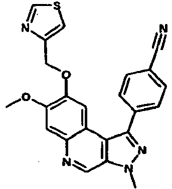 | 4-[7-Methoxy-3-methyl-8-(thiazol-4-ylmethoxy)-3H-pyrazolo[3,4-c]chinolin-1-yl]-benzonitril | MS: 428,1 (M+H)⁺ | < 0,1 |
| | | | DC (HPTLC): R_{f} = 0,37 (Essigsäureethylester/Ethanol 8:1, Volumenanteile) | |
| 43 | 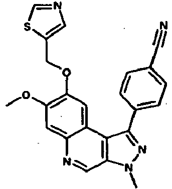 | 4-[7-Methoxy-3-methyl-8-(thiazol-5-ylmethoxy)-3H-pyrazolo[3,4-c]chinolin-1-yl]-benzonitril | MS: 428,1 (M+H)⁺ | < 0,1 |
| | | | DC (HPTLC): R_{f} = 0,37 (Essigsäure-ethylester/Ethanol 8:1, Volumenanteile) | |
| 44 | 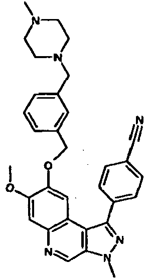 | 4-{7-Methoxy-3-methyl-8-[3-(4-methyl-piperazin-1-ylmethyl)-benzyloxy]-3H-pyrazolo[3,4-c]chinolin-1-yl}-benzonitril | MS: 523,2 (M+H)⁺ | > 0,5 |
| | | | DC (HPTLC): R_{f} = 0,10 (Dichlormethan/ Ethanol 1:1, Volumenanteile) | |
| 45 | 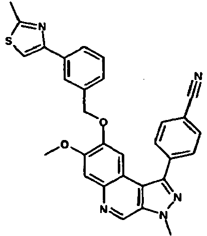 | 4-{7-Methoxy-3-methyl-8-[3-(2-methyl-thiazol-4-yl)-benzyloxy]-3H-pyrazolo[3,4-c]chinolin-1-yl}-benzonitril | MS: 518,1 (M+H)⁺ | 0,1 - 0,5 |
| | | | DC (HPTLC): R_{f} = 0,53 (Essigsäure-ethylester/Ethanol 8:1, Volumenanteile) | |
| 46 | 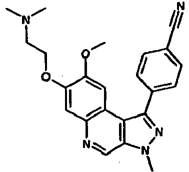 | 4-[7-(2-Dimethylamino-ethoxy)-8-methoxy-3-methyl-3H-pyrazolo[3,4-c]chinolin-1-yl]-benzonitril | MS: 402,2 (M+H)⁺ | > 0,5 |
| | | | DC (HPTLC): R_{f} = 0,05 (Methanol) | |
| 47 | 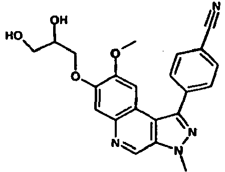 | 4-[7-(2,3-Dihydroxypropoxy)-8-methoxy-3-methyl-3H-pyrazolo[3,4-c]chinolin-1-yl]-benzonitril | MS: 405,1 (M+H)⁺ | > 0,5 |
| | | | DC (HPTLC): R_{f} = 0,46 (Dichlormethan/Ethanol 5:1, Volumenanteile) | |

Die Verbindung der u.g. Strukturformel (Tab. 5a) kann methodisch nach den Synthesevorschriften zu den Beispielen 1, 4 und 8 - beginnend mit der Verbindung der Formel 3-2 - hergestellt werden.

**Tab. 5a Verbindung der Formeln (I) und (IE)**

| **Nr.** | **Strukturformel** | **Name** | **Analytik** | **IC₅₀ DNA-PK [µM]** |
|---|---|---|---|---|
| 79 | 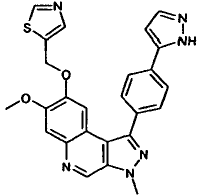 | 7-Methoxy-3-methyl-1-[4-(2H-pyrazol-3-yl)-phenyl]-8-(thiazol-5-ylmethoxy)-3H-pyrazolo[3,4-c]chinolin | MS: 469,0 (M+H)+ | < 0,1 |
| | | | DC (HPTLC): Rf = 0,24 (Essigsäure-ethylester/Ethanol 5:1, Volumenanteile) | |

### BEISPIEL 5: Synthese von 2-[1-(4-Cyanophenyl)-7-methoxy-3-methyl-3H-pyrazolo[3,4-c]chinolin-8-yloxy]-N-[2-(4-ethylpiperazin-1-yl)-ethyl]-2-(4-fluorphenyl)-acetamid

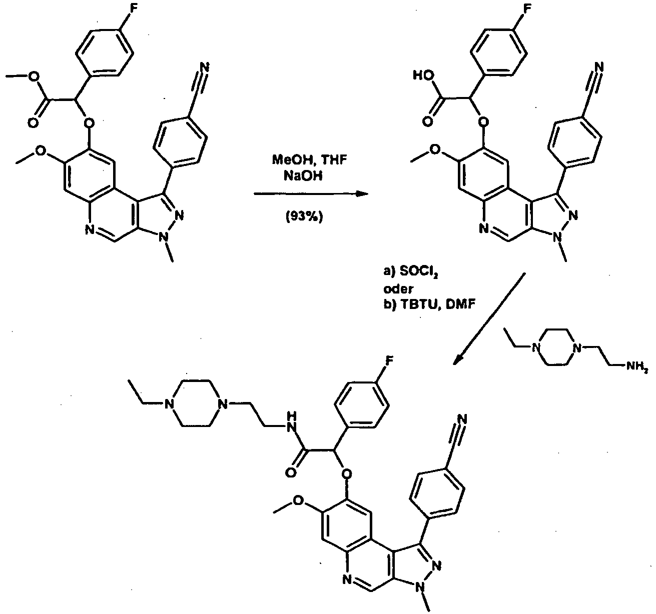

[1-(4-Cyanophenyl)-7-methoxy-3-methyl-3*H*-pyrazolo[3,4-c]chinolin-8-yloxy]-(4-fluorphenyl)-essigsäuremethylester (260 mg, 524 µmol) wird in Tetrahydrofuran und Methanol (je 66 ml) gelöst. Anschließend wird Natronlauge (1,0 M, 1,58 ml, 1,58 mmol) zugetropft. Die Reaktionsmischung wird 18 h bei Raumtemperatur gerührt. Nach Beendigung der Umsetzung wird im Vakuum zur Trockne eingeengt und der erhaltene Rückstand in Wasser (100 ml) aufgenommen. Durch Zugabe von Salzsäure (1,0 M) wird vorsichtig auf pH 5 angesäuert und weitere 30 min gerührt. Der entstandene Niederschlag wird abgesaugt und mit Wasser nachgewaschen. Der Filterkuchen wird anschließend in 2-Propanol (5 ml) suspendiert, erneut abgesaugt und der Rückstand im Hochvakuum über Nacht getrocknet, wobei [1-(4-Cyanophenyl)-7-methoxy-3-methyl-3*H*-pyrazolo[3,4-c]chinolin-8-yloxy]-(4-fluorphenyl)-essigsäure (235 mg, 488 µmol) als Feststoff mit einem Schmelzpunkt von 211,6°C erhalten wird. MS: 483.1 (M+H⁺), DC (HPTLC): R_{f} = 0.11 (Ethanol).

[1-(4-Cyanophenyl)-7-methoxy-3-methyl-3*H*-pyrazolo[3,4-c]chinolin-8-yloxy]-(4-fluorphenyl)-essigsäure (190 mg, 394 µmol) wird in Thionylchlorid (2,0 ml) gelöst und 2 h am Rückfluss erhitzt. Danach wird im Vakuum zur Trockne eingeengt und zweimal mit destilliertem, wasserfreiem Toluol koevaporiert, wobei [1-(4-Cyanophenyl)-7-methoxy-3-methyl-3*H*-pyrazolo[3,4-c]chinolin-8-yloxy]-(4-fluorphenyl)-essigsäurechlorid (195 mg, 389 µmol) erhalten wird. Anschließend werden 35 mg (69,9 µmol) des erhaltenen Säurechlorids in wasserfreiem *N,N*-Dimethylformamid (2,0 ml) in einer trockenen Stickstoff-Atmosphäre gelöst und unter Eisbadkühlung mit 2-(4-Ethyl-piperazin-1-yl)-ethylamin (22 mg, 140 µmol) versetzt. Die Reaktionslösung wird 2 h bei Raumtemperatur gerührt. Danach wird auf Wasser (30 ml) gegossen und viermal mit Essigsäureethylester (je 30 ml) extrahiert. Die vereinigten organischen Phasen werden zweimal mit Wasser (je 20 ml) gewaschen, anschließend mit Na₂SO₄ getrocknet, abgesaugt und das Filtrat im Vakuum zur Trockne eingeengt. Der Rückstand wird in Dimethylsulfoxid gelöst und chromatographiert (pHPLC, Lösungsmittelgradient Wasser / 1-30 vol.% Acetonitril, 0,1 vol.% Ameisensäure), wobei nach dem Gefriertrocknen die Titelverbindung 2-[1-(4-Cyanophenyl)-7-methoxy-3-methyl-3*H*-pyrazolo[3,4-c]chinolin-8-yloxy]-*N*-[2-(4-ethylpiperazin-1-yl)-ethyl]-2-(4-fluorphenyl)-acetamid (21 mg, 33,8 µmol) als Lyophilisat mit einem Schmelzbereich von 110-112°C erhalten wird. MS: 622,3 (M+H⁺), DC (HPTLC): R_{f} = 0,40 (Methanol/Hünigsbase 99:1, Volumenanteile).

Verbindungen, die entsprechend der Synthesevorschrift aus Beispiel 5 hergestellt werden, finden sich in der nachfolgenden Tabelle 6.

**Tab. 6 Verbindungen der Formeln (I), (IA) und (IE)**

| **Nr.** | **Strukturformel** | **Name** | **Analytik** | **IC₅₀ DNA-PK [µM]** |
|---|---|---|---|---|
| 48 | 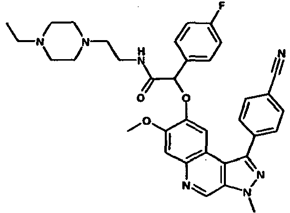 | 2-[1-(4-Cyanophenyl)-7-methoxy-3-methyl-3*H*-pyrazolo[3,4-c]chinolin-8-yloxy]-*N-*[2-(4-ethylpiperazin-1-yl)-ethyl]-2-(4-fluorphenyl)-acetamid | ¹H NMR (500 MHz, DMSO) δ = 9.33 (s, 1H), 8.24 - 8.08 (m, 1H), 8.05 (d, *J*=8.3, 2H), 7.89 (d, *J*=8.3, 2H), 7.71 (s, 1H), 7.41 (dd, *J*=8.6, 5.5, 2H), 7.34 (s, 1H), 7.19 (d, *J*=8.9, 1H), 7.17 (d, *J*=8.8, 1H), 5.53 (s, 1H), 4.31 (s, 3H), 3.98 (s, 3H), 3.40 (bm, 2H), 3.19 (bm, 2H), 3.01 (bm, 4H), 2.81 (bm, 3H), 2.33 (bm, 3H), 1.17 (t, *J*=7.3, 3H). | 0,1 - 0,5 |
| | | | MS: 622,3 (M+H)⁺ | |
| | | | DC (HPTLC): R_{f} = 0,40 (Methanol/Hünigsbase 99:1, Volumenanteile) | |
| 49 | 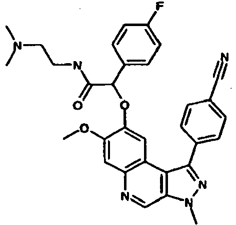 | 2-[1-(4-Cyano-phenyl)-7-methoxy-3-methyl-3H-pyrazolo[3,4-c]chinolin-8-yloxy]-*N-*(2-dimethylaminoethyl)-2-(4-fluorphenyl)-acetamid | MS: 553,2 (M+H)⁺ | 0,1 - 0,5 |
| | | | DC (HPTLC): R_{f} = 0,27 (Methanol/Hünigsbase 99:1, Volumenanteile) | |
| 50 | 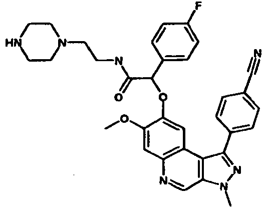 | 2-[1-(4-Cyano-phenyl)-7-methoxy-3-methyl-3H-pyrazolo[3,4-c]chinolin-8-yloxy]-2-(4-fluor-phenyl)-N-(2-piperazin-1-yl-ethyl)-acetamid | MS: 594,2 (M+H)⁺ | > 0,5 |
| | | | DC (HPTLC): R_{f} = 0,09 (Methanol/Hünigsbase 99:1, Volumenanteile) | |
| 51 | 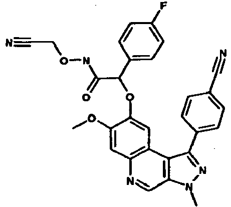 | N-Cyanomethoxy-2-[1-(4-cyano-phenyl)- ' H " 7-methoxy-3-methyl-3H-pyrazolo[3,4-c]chinolin-8-yloxy]-2-(4-fluor-phenyl)-acetamid | MS: 539,9 (M+H)⁺ | 0,1 - 0,5 |
| | | | DC (HPTLC): R_{f} = 0,49 (Essigsäureethylester/Ethanol 5:1, Volumenanteile) | |
| 52 | 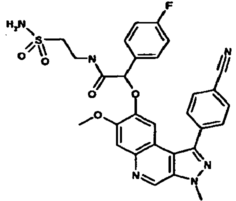 | 2-[1-(4-Cyano-phenyl)-7-methoxy-3-methyl-3H-pyrazolo[3,4-c]chinolin-8-yloxy]-2-(4-fluor-phenyl)-N-(2-sulfamoyl-ethyl)-acetamid | MS: 588,9 (M+H)⁺ | 0,1 - 0,5 |
| | | | DC (HPTLC): R_{f} = 0,45 (Essigsäureethylester/Ethanol 5:1, Volumenanteile) | |
| 53 | 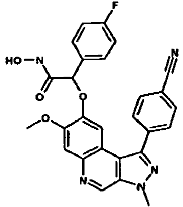 | 2-[1-(4-Cyano-phenyl)-7-methoxy-3-methyl-3H-pyrazolo[3,4-c]chinolin-8-yloxy]-2-(4-fluor-phenyl)-N-hydroxy-acetamid | MS: 497,9 (M+H)⁺ | < 0,1 |
| | | | DC (HPTLC): R_{f} = 0,24 (Essigsäureethylester/Ethanol 5:1, Volumenanteile) | |
| 54 | 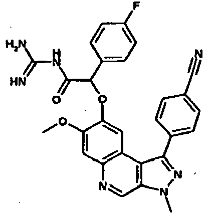 | N-[2-[1-(4-Cyano-phenyl)-7-methoxy-3-methyl-3H-pyrazolo[3,4-c]chinolin-8-yloxy]-2-(4-fluor-phenyl)-acetyl]-guanidin | MS: 524,2 (M+H)⁺ | < 0,1 |
| | | | DC (HPTLC): R_{f} = 0,29 (Ethanol) | |
| 55 | 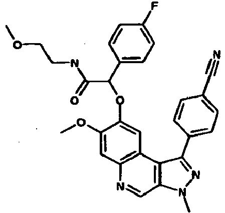 | 2-[1-(4-Cyano-phenyl)-7-methoxy-3-methyl-3H-pyrazolo[3,4-c]chinolin-8-yloxy]-2-(4-fluor-phenyl)-N-(2-methoxy-ethyl)-acetamid | MS: 540,2 (M+H)⁺ | < 0,1 |
| | | | DC (HPTLC): R_{f} = 0,28 (Essigsäureethylester/Ethanol 8:1, Volumenanteile) | |

### BEISPIEL 6: Synthese 4-(7,8-Dimethoxy-3-ethyl-3H-pyrazolo[3,4-c]chinolin-1-yl)-benzonitril

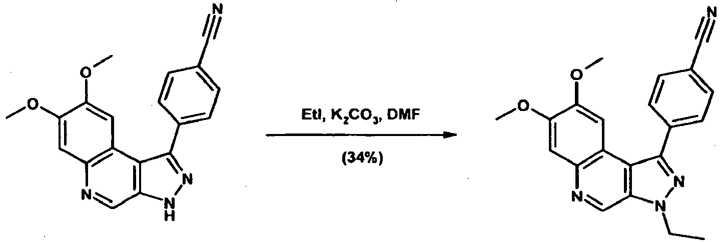

4-(7,8-Dimethoxy-3*H*-pyrazolo[3,4-c]chinolin-1-yl)-benzonitril (132 mg, 400 µmol) wird in *N,N*-Dimethylformamid (5 ml) gelöst. Anschließend wird Kaliumcarbonat (111 mg, 800 µmol) und Ethyliodid (37 µl, 440 µmol) zugefügt. Die Reaktionsmischung wird 3 h bei Raumtemperatur gerührt. Anschließend wird auf Wasser (100 ml) gegossen und zweimal mit Essigsäureethylester (je 100 ml) extrahiert. Die vereinigten organischen Phasen werden einmal mit Wasser (50 ml) gewaschen, über Na₂SO₄ getrocknet, abgesaugt und das erhaltene Filtrat im Vakuum zur Trockne eingeengt. Der Rückstand wird über Flashkieselgel chromatographiert (Lösemittelgradient n-Heptan / 0-100 vol.% Essigsäureethylester / Ethanol 0-30 vol.%). Nach dem Einengen der Produktfraktionen wird in wenig Tetrahydrofuran gelöst und mit 2-Propanol (5 ml) versetzt und eingeengt. Der erhaltene Niederschlag wird abgesaugt und im Hochvakuum getrocknet, wobei die Titelverbindung 4-(7,8-Dimethoxy-3-ethyl-3*H*-pyrazolo[3,4-c]chinolin-1-yl)-benzonitril (49 mg, 136 mmol) als Feststoff mit einem Schmelzpunkt von 227,4°C erhalten wird. MS: 359,0 (M+H⁺), DC (HPTLC): R_{f} = 0,51 (Essigsäureethylester / Ethanol 8:1, Volumenanteile). Verbindungen, die entsprechend der Synthesevorschrift aus Beispiel 6 hergestellt werden, finden sich in der nachfolgenden Tabelle 7.

**Tab. 7 Verbindungen der Formeln (I), (IA) und (IE)**

| **Nr.** | **Strukturformel** | **Name** | **Analytik** | **IC₅₀ DNA-PA [µM]** |
|---|---|---|---|---|
| 56 | 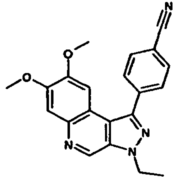 | 4-(7,8-Dimethoxy-3-ethyl-3*H-*pyrazolo[3,4-c]chinolin-1-yl)-benzonitril | MS: 359,0 (M+H)⁺ | < 0,1 |
| | | | DC (HPTLC): R_{f} = 0,51 (Essigsäure-ethylester/Ethanol 8:1, Volumenanteile) | |
| 57 | 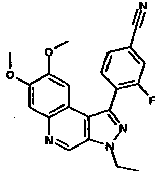 | 4-(3-Ethyl-7,8-dimethoxy-3H-pyrazolo[3,4-c]chinolin-1-yl)-3-fluor-benzonitril | MS: 376,9 (M+H)⁺ | 0,1 - 0,5 |
| | | | DC (HPTLC): R_{f} = 0,62 (Dichlormethan/Ethanol 10:1, Volumenanteile) | |
| 58 | 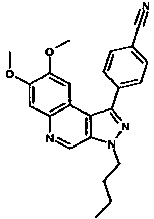 | 4-(3-Butyl-7,8-dimethoxy-3H-pyrazolo[3,4-c]chinolin-1-yl)-benzonitril | MS: 387,0 (M+H)⁺ | > 0,5 |
| | | | DC (HPTLC): R_{f} = 0,42 (Essigsäureethylester) | |
| 59 | 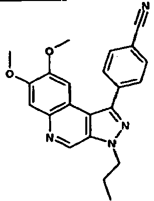 | 4-(7,8-Dimethoxy-3-propyl-3H-pyrazolo[3,4-c]chinolin-1-yl)-benzonitril | MS: 372,8 (M+H)⁺ | 0,1 - 0,5 |
| | | | DC (HPTLC): R_{f} = 0,34 (Essigsäureethylester) | |
| 60 | 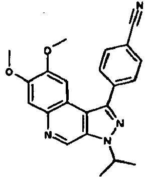 | 4-(3-Isopropyl-7,8-dimethoxy-3H-pyrazolo[3,4-c]chinolin-1-yl)-benzonitril | MS: 373,0 (M+H)⁺ | 0,1-0,5 |
| | | | DC (HPTLC): R_{f} = 0,35 (Essigsäureethylester) | |
| 61 | 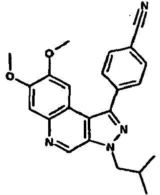 | 4-(3-Isobutyl-7,8-dimethoxy-3H-pyrazolo[3,4-c]chinolin-1-yl)-benzonitril | MS: 387,0 (M+H)⁺ | > 0,5 |
| | | | DC (HPTLC): R_{f} = 0.47 (Essigsäureethylester) | |
| 62 | 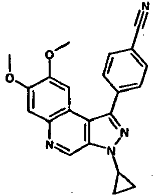 | 4-(3-Cyclopropyl-7,8-dimethoxy-3H-pyrazolo[3,4-c]chinolin-1-yl)-benzonitril | MS: 372,0 (M+H)⁺ | 0,1 - 0,5 |
| | | | DC (HPTLC): R_{f} = 0,50 (Essigsäure-ethylester/Ethanol 5:1, Volumenanteile) | |
| 63 | 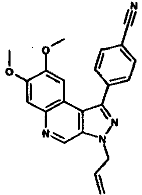 | 4-(3-Allyl-7,8-dimethoxy-3H-pyrazolo[3,4-c]chinolin-1-yl)-benzonitril | MS: 371,1, (M+H)⁺ | 0,1 - 0,5 |
| | | | DC (HPTLC): R_{f} = 0,46 (Essigsäureethylester) | |
| 64 | 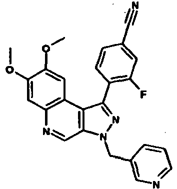 | 4-(7,8-Dimethoxy-3-pyridin-3-ylmethyl-3H-pyrazolo[3,4-c]chinolin-1-yl)-3-fluor-benzonitril | MS: 439,9 (M+H)⁺ | > 0,5 |
| | | | DC (HPTLC): R_{f} = 0,16 (Essigsäure-ethylester/Ethanol 8:1, Volumenanteile) | |
| 65 | 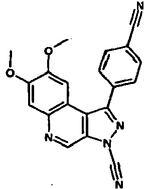 | 1-(4-Cyano-phenyl)-7,8-dimethoxy-pyrazolo[3,4-c]chinoline-3-carbonitrile | MS: 356,1 (M+H)⁺ | 0,1 - 0,5 |
| | | | DC (HPTLC): R_{f} = 0,70 (Essigsäureethylester) | |
| 66 | 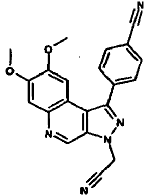 | 4-(3-Cyanomethyl-7,8-dimethoxy-3H-pyrazolo[3,4-c]chinolin-1-yl)-benzonitril | MS: 369,8 (M+H)⁺ | < 0,1 |
| | | | DC (HPTLC): R_{f} = 0,42 (Essigsäureethylester) | |
| 67 | 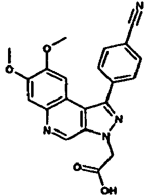 | [1-(4-Cyano-phenyl)-7,8-dimethoxy-pyrazolo[3,4-c]chinolin-3-yl]-essigsäure | MS: 389,1 (M+H)⁺ | 0,1 - 0,5 |
| | | | DC (HPTLC): R_{f} = 0,55 (Ethanol) | |
| 68 | 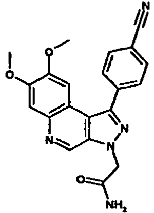 | 2-[1-(4-Cyano-phenyl)-7,8-dimethoxy-pyrazolo[3,4-c]chinolin-3-yl]-acetamid | MS: 388,1 (M+H)⁺ | 0,1 - 0,5 |
| | | | DC (HPTLC): R_{f} = 0,36 (Essigsäureethylester) | |
| 69 | 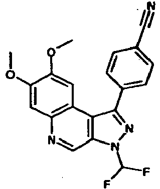 | 4-(3-Difluormethyl-7,8-dimethoxy-3H-pyrazolo[3,4-c]chinolin-1-yl)-benzonitril | MS: 381,1 (M+H)⁺ | < 0,1 |
| | | | DC (HPTLC): R_{f} = 0,49 (Essigsäure-ethylester/Ethanol 8:1, Volumenanteile) | |

### BEISPIEL 7A: Synthese von 4-(7,8-Dimethoxy-3H-pyrazolo[3,4-c]chinolin-1-yl)-2-pyrrolidin-benzonitril

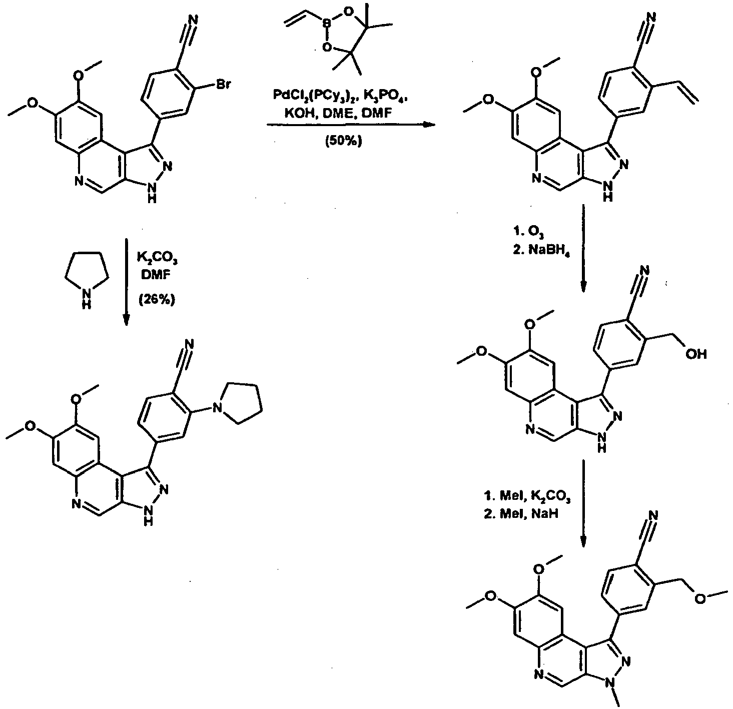

2-Brom-4-(7,8-dimethoxy-3*H*-pyrazolo[3,4-c]chinolin-1-yl)-benzonitril (90 mg, 220 µmol) wird in *N,N*-Dimethylformamid (3,0 ml) gelöst. Anschließend werden Pyrrolidin (1,0 ml, 12,1 mmol) und Kaliumcarbonat (61 mg, 440 µmol) zugefügt. Die Reaktionsmischung wird 20 min bei 180°C (Mikrowelle) erhitzt. Zur Aufarbeitung wird mit Essigsäureethylester und halbgesättigter Kochsalzlösung extrahiert. Die organische Phase wird über Na₂SO₄ getrocknet, abgesaugt und im Vakuum zur Trockne eingeengt. Der Rückstand wird chromatograpisch (pHPLC, Lösungsmittelgradient Wasser / 1-30 vol.% Acetonitril, 0,1 vol.% Ameisensäure) aufgereinigt, wobei 4-(7,8-Dimethoxy-3*H*-pyrazolo[3,4-c]chinolin-1-yl)-2-pyrrolidin-benzonitril (23 mg, 58 µmol) als Feststoff mit einem Schmelzpunkt von 291°C (Zersetzung) anfällt. MS: 400,2 (M+H⁺), DC (HPTLC): R_{f} = 0,41 (Essigsäureethylester / Ethanol 8:1, Volumenanteile).

### BEISPIEL 7B: Synthese von 4-(7,8-Dimethoxy-3-methyl-3H-pyrazolo[3,4-c]chinolin-1-yl)-2-methoxymethyl-benzonitril

Unter einer Argon-Schutzgasatmosphäre wird 2-Brom-4-(7,8-dimethoxy-3*H*-pyrazolo[3,4-c]chinolin-1-yl)-benzonitril (540 mg, 1,32 mmol) in *N,N*-Dimethylformamid (4,0 ml) gelöst. Anschließend werden Trikaliumphosphat (578 mg, 2,64 mmol), KOH (67 mg, 1,19 mmol), 1,2-Dimethoxyethan (9,0 ml) und Wasser (50 µl), Vinylboronsäurepinakolester (942 µl, 5,28 mmol) und *trans*-Bis(tricyclohexylphosphan)-Palladium-(II)-dichlorid (98 mg, 132 µmol) zugefügt. Die erhaltene Suspension wird 30 min auf 150°C erhitzt (Mikrowelle). Zur Aufarbeitung wird mit Essigsäureethylester und halbgesättigter Kochsalzlösung extrahiert. Die organische Phase wird über Na₂SO₄ getrocknet, abgesaugt und im Vakuum zur Trockne eingeengt. Der Rückstand wird über Flashkieselgel (Lösungsmittelgradient Cyclohexan / 0-80 vol.% Essigsäureethylester) aufgereinigt, wobei 4-(7,8-Dimethoxy-3*H*-pyrazolo[3,4-c]chinolin-1-yl)-2-vinyl-benzonitril (247 mg, 693 µmol) als Feststoff anfallen. MS: 357,2 (M+H⁺), DC (HPTLC): R_{f} = 0,47 (Essigsäureethylester / Ethanol 8:1, Volumenanteile).

4-(7,8-Dimethoxy-3*H*-pyrazolo[3,4-c]chinolin-1-yl)-2-vinyl-benzonitril (247 mg, 693 µmol) wird in *N,N*-Dimethylformamid (1,5 ml), Tetrahydrofuran (6,0 ml) und Ethanol (9,0 ml) gelöst und auf (-) 78°C gekühlt. Anschließend wird die Reaktionslösung mit Ozon (Sauerstofffluss Ozongenerator 50l/h) für 4 min behandelt, danach wird Stickstoff durchgeleitet (2 min). Nach LC-MS Kontrolle (Aldehyd-Intermediat) wird NaBH₄ (24 mg, 624 µmol) zugegeben und die Reaktionsmischung 20 min bei (-)78°C gerührt. Anschließend wird das Kältebad entfernt, so dass sich die Reaktionslösung auf Raumtemperatur erwärmt. Zur Aufreinigung wird mit Essigsäureethylester und halbgesättigter Kochsalzlösung extrahiert. Die organische Phase wird über Na₂SO₄ getrocknet, abgesaugt und im Vakuum bei 40°C zur Trockne eingeengt. Der Rückstand wird chromatograpisch (pHPLC, Lösungsmittelgradient Wasser / 1-30 vol.% Acetonitril, 0,1 vol.% Ameisensäure) aufgereinigt und die Produktfraktionen lyophilisiert, wobei 4-(7,8-Dimethoxy-3*H*-pyrazolo[3,4-c]chinolin-1-yl)-2-hydroxymethyl-benzonitril (46 mg, 128 µmol) als Feststoff anfallen. MS: 361,4 (M+H⁺), DC (HPTLC): R_{f} = 0,38 (Essigsäureethylester / Ethanol 8:1, Volumenanteile).

Unter Argon wird 4-(7,8-Dimethoxy-3*H*-pyrazolo[3,4-c]chinolin-1-yl)-2-hydroxymethyl-benzonitril (78 mg, 216 µmol) in *N,N*-Dimethylformamid (1,0 ml) gelöst und anschließend Methyliodid (34 µl, 541 µmol) und Kaliumcarbonat (60 mg, 433 µmol) zugefügt. Die Suspension wird 1 h bei Raumtemperatur gerührt. Zur Aufreinigung wird mit Essigsäureethylester und halbgesättigter Kochsalzlösung extrahiert. Die organische Phase wird über Na₂SO₄ getrocknet, abgesaugt und im Vakuum zur Trockne eingeengt. Der Rückstand wird chromatograpisch (pHPLC, Lösungsmittelgradient Wasser / 1-30 vol.% Acetonitril, 0,1 vol.% Ameisensäure) aufgereinigt und die Produktfraktionen lyophilisiert, wobei 4-(7,8-Dimethoxy-3-methyl-3*H*-pyrazolo[3,4-c]chinolin-1-yl)-2-hydroxymethyl-benzonitril (41 mg, 110 µmol) als Feststoff anfallen. MS: 375,2 (M+H⁺).

Unter Argon wird 4-(7,8-Dimethoxy-3-methyl-3*H*-pyrazolo[3,4-c]chinolin-1-yl)-2-hydroxymethyl-benzonitril (17mg, 44 µmol) in *N,N*-Dimethylformamid (2,1 ml) gelöst und anschließend Methyliodid (8,2 µl, 131 µmol) und Natriumhydrid (95%ig, 2,4 mg, 96 µmol) zugegeben. Die Reaktionslösung wird für 2 h bei 0°C gerührt. Nach Zugabe von wenig Wasser wird zur Aufarbeitung mit Essigsäureethylester und halbgesättigter Kochsalzlösung extrahiert. Die organische Phase wird über Na₂SO₄ getrocknet, abgesaugt und im Vakuum bei 40°C zur Trockne eingeengt. Der Rückstand wird chromatograpisch (pHPLC, Lösungsmittelgradient Wasser / 1-30 vol.% Acetonitril, 0,1 vol.% Ameisensäure) aufgereinigt und die Produktfraktionen gefriergetrocknet, wobei 4-(7,8-Dimethoxy-3-methyl-3*H*-pyrazolo[3,4-c]chinolin-1-yl)-2-methoxymethyl-benzonitril (4 mg, 10,3 µmol) als farbloser Feststoff anfallen. MS: 389,2 (M+H⁺), DC (HPTLC): R_{f} = 0,41 (Essigsäureethylester / Ethanol 8:1, Volumenanteile).

Verbindungen, die entsprechend der Synthesevorschriften aus Beispiel 7A und 7B hergestellt werden, finden sich in der nachfolgenden Tabelle 8.

**Tab. 8 Verbindungen der Formeln (I), (IA) und (IE)**

| **Nr.** | **Strukturformel** | **Name** | **Analytik** | **IC₅₀ DNA-PK [µM]** |
|---|---|---|---|---|
| 70 | 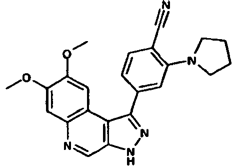 | 4-(7,8-Dimethoxy-3*H-*pyrazolo[3,4-c]chinolin-1-yl)-2-pyrrolidin-1-yl-benzonitril | (Beispiel 7A) | < 0,1 |
| 71 | 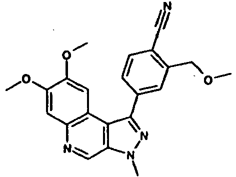 | 4-(7,8-Dimethoxy-3-methyl-3*H*-pyrazolo[3,4-c]chinolin-1-yl)-2-methoxymethyl-benzonitril | (Beispiel 7B) | < 0,1 |
| 72 | 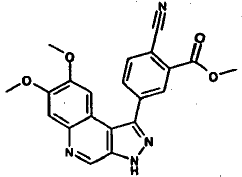 | 2-Cyano-5-(7,8-dimethoxy-3H-pyrazolo[3,4-c]chinolin-1-yl)-benzoic acid methyl ester | MS: 389,1 (M+H)⁺ | < 0,1 |
| | | | DC (HPTLC): R_{f} = 0,56 (Essigsäureethylester/Ethanol 5:1, Volumenanteile) | |
| 73 | 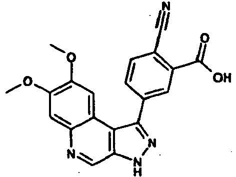 | 2-Cyano-5-(7,8-dimethoxy-3H-pyrazolo[3,4-c]chinolin-1-yl)-benzoic acid | MS: 375,1 (M+H)⁺ | < 0,1 |
| | | | DC (HPTLC): R_{f} = 0,30 (Essigsäureethylester/Ethanol 5:1, Volumenanteile) | |
| 74 | 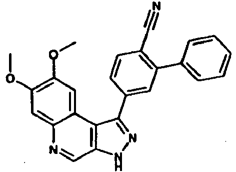 | 5-(7,8-Dimethoxy-3H-pyrazolo[3,4-c]chinolin-1-yl)-biphenyl-2-carbonitrile | MS: 407,4 (M+H)⁺ | > 0,5 |
| | | | DC (HPTLC): R_{f} = 0,24 (Essigsäureethylester) | |

### BEISPIEL 8: Synthese von 1-[4-(4-Ethyl-piperazin-1-yl)-phenyl]-7,8-dimethoxy-3-methyl-3H-pyrazolo[3,4-c]chinolin

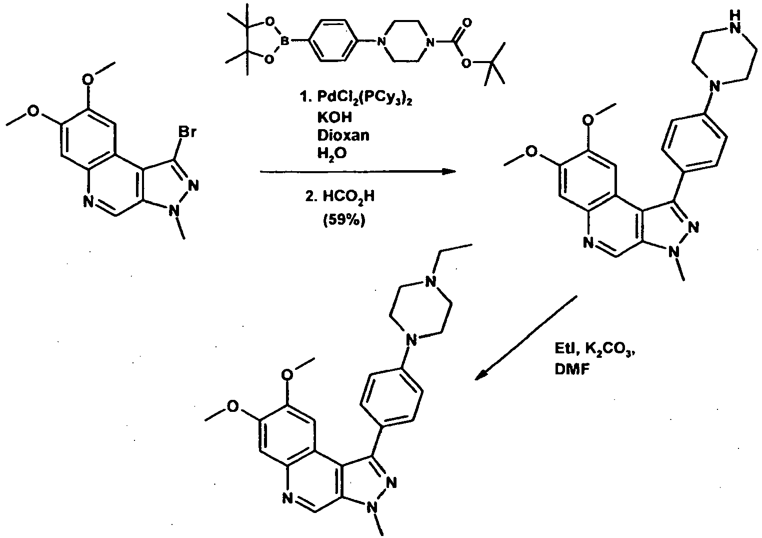

1-Brom-7,8-dimethoxy-3-methyl-3*H*-pyrazolo[3,4-c]chinolin (30 mg, 93 µmol) wird unter Argon in Dioxan (1,0 ml) gelöst. Anschließend werden KOH (5,2 mg, 93 µmol), 4-[4-(4,4,5,5-Tetramethyl-[1,3,2]dioxaborolan-2-yl)-phenyl]-piperazin-1-carbonsäure-tert-butylester (83 mg, 214 µmol), *trans*-Bis(tricyclohexylphosphan)-Palladium-(II)-dichlorid (10,4 mg, 14 µmol) und Wasser (50 µl) zugefügt. Die Reaktionssuspension wird anschließend 90 min auf 110°C erhitzt (Mikrowelle). Anschließend wird auf Wasser gegossen, 15 min gerührt und der erhaltene Niederschlag abgesaugt. Der Filterrückstand wird danach 18 h bei Raumtemperatur in Ameisensäure (2,5 ml) behandelt, anschließend mit Wasser verdünnt und zweimal mit Essigsäureethylester extrahiert. Die vereinigten organischen Phasen werden mit Wasser gewaschen, über Na₂SO₄ getrocknet, abgesaugt und im Vakuum zur Trockne eingeengt. Der Rückstand wird chromatograpisch (pHPLC, Lösungsmittelgradient Wasser / 1-30 vol.% Acetonitril, 0,1 vol.% Ameisensäure) aufgereinigt und die Produktfraktionen gefriergetrocknet, wobei 7,8-Dimethoxy-3-methyl-1-(4-piperazin-1-yl)-phenyl)-3*H*-pyrazolo[3,4-c]chinolin (22 mg, 55 µmol) als Feststoff anfallen. MS: 404,1 (M+H⁺). DC (HPTLC): R_{f} = 0,19 (Methanol/Hünigsbase 99:1, Volumenanteile).

7,8-Dimethoxy-3-methyl-1-(4-piperazin-1-yl)-phenyl)-3*H*-pyrazolo[3,4-c]chinolin (28 mg, 70 µmol) wird in wasserfreiem *N,N*-Dimethylformamid (3,0 ml) wird Kaliumcarbonat (19 mg, 136 µmol) und Ethyliodid (6 µl, 74 µmol) bei Raumtemperatur zugefügt. Die Reaktionsmischung wird anschließend 18 h bei Raumtemperatur gerührt. Nach Beendigung der Reaktion wird auf Wasser gegossen und zweimal mit Essigsäureethylester extrahiert. Die vereinigten organischen Phasen werden über Na₂SO₄ getrocknet, filtriert und im Vakuum zur Trockne eingeengt. Der Rückstand wird aus Wasser/Acetonitril gefriergetrocknet, wobei die Titelverbindung 1-[4-(4-Ethyl-piperazin-1-yl)-phenyl]-7,8-dimethoxy-3-methyl-3*H-*pyrazolo[3,4-c]chinolin (6,6 mg, 15,3 mmol) als Feststoff erhalten wird. MS: 432,2 (M+H⁺). DC (HPTLC): R_{f} = 0,35 (Methanol/Hünigsbase 99:1, Volumenanteile).

Verbindungen, die entsprechend der Synthesevorschrift aus Beispiel 8 hergestellt werden, finden sich in der nachfolgenden Tabelle 9.

**Tab. 9 Verbindungen der Formeln (I), (IA) und (IE)**

| **Nr.** | **Strukturformel** | **Name** | **Analytik** | **IC₅₀ DNA-PK [µM]** |
|---|---|---|---|---|
| 75 | 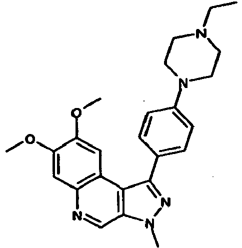 | 1-[4-(4-Ethyl-piperazin-1-yl)-phenyl]-7,8-dimethoxy-3-methyl-3*H-*pyrazolo[3,4-c]chinolin | (Beispiel 8) | 0,1 - 0,5 |
| 76 | 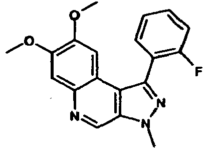 | 1-(2-Fluorphenyl)-7,8-dimethoxy-3-methyl-3*H-*pyrazolo[3,4-c]chinolin | MS: 338,2 (M+H)⁺ | < 0,1 |
| | | | DC (HPTLC): R_{f} = 0,48 (Essigsäure-ethylester/Ethanol 5:1, Volumenanteile) | |

Verbindungen der u.g. Strukturformeln (Tab. 9a) können methodisch nach den Synthesevorschriften zu Beispiel 8 - beginnend mit der Verbindung der Formel 3-2-hergestellt werden.

**Tab. 9a Verbindungen der Formeln (I) und (IE)**

| **Nr.** | **Strukturformel** | **Name** | **Analytik** |
|---|---|---|---|
| 80 | 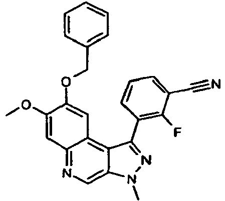 | 3-(8-Benzyloxy-7-methoxy-3-methyl-3H-pyrazolo[3,4-c]chinolin-1-yl)-2-fluor-benzonitril | MS: 439,1 (M+H)⁺ |
| | | | DC (HPTLC): R_{f} = 0,49 (Essigsäureethylester/Ethanol 8:1, Volumenanteile) |
| 81 | 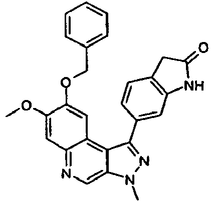 | 6-(8-Benzyloxy-7-methoxy-3-methyl-3H-pyrazolo[3,4-c]chinolin-1-yl)-1,3-dihydro-indol-2-on | MS: 451,1 (M+H)⁺ |
| | | | DC (HPTLC): R_{f} = 0,34 (Essigsäureethylester/Ethanol 8:1, Volumenanteile) |
| 82 | 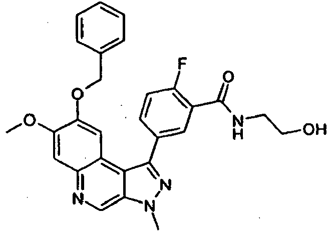 | 5-(8-Benzyloxy-7-methoxy-3-methyl-3H-pyrazolo[3,4-c]chinolin-1-yl)-2-fluor-N-(2-hydroxy-ethyl)-benzamid | MS: 501,2 (M+H)⁺ |
| | | | DC (HPTLC): R_{f} = 0,20 (Essigsäureethylester/Ethanol 8:1, Volumenanteile) |
| 83 | 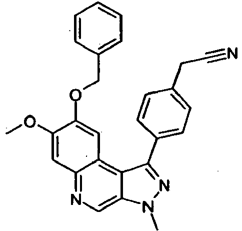 | [4-(8-Benzyloxy-7-methoxy-3-methyl-3H-pyrazolo[3,4-c]qchinolin-1-yl)-phenyl]-acetonitril | MS: 435,1 (M+H)⁺ |
| | | | DC (HPTLC): R_{f} = 0,44 (Essigsäure-ethylester/Ethanol 8:1, Volumenanteile) |
| 84 | 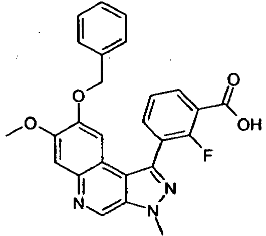 | 3-(8-Benzyloxy-7-methoxy-3-methyl-3H-pyrazolo[3,4-c]chinolin-1-yl)-2-fluor-benzoesäure | MS: 458,1 (M+H)⁺ |
| | | | DC (HPTLC): R_{f} = 0,44 (Essigsäureethylester/Ethanol 5:1, Volumenanteile) |
| 85 | 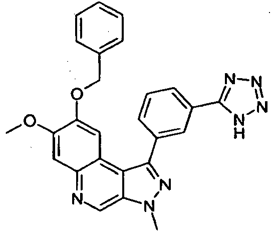 | 8-Benzyloxy-7-methoxy-3-methyl-1-[3-(1 H-tetrazol-5-yl)-phenyl]-3H-pyrazolo[3,4-c]chinolin | MS: 464,1 (M+H)⁺ |
| | | | DC (HPTLC): R_{f} = 0,20 (Dichlormethan/Ethanol 5:1, Volumenanteile) |
| 86 | 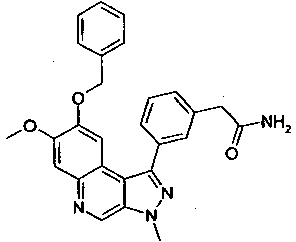 | 2-[3-(8-Benzyloxy-7-methoxy-3-methyl-3H-pyrazolo[3,4-c]chinolin-1-yl)-phenyl]-acetamid | MS: 453,1 (M+H)⁺ |
| | | | DC (HPTLC): R_{f} = 0,58 (Essigsäureethylester/Ethanol 8:1, Volumenanteile) |
| 87 | 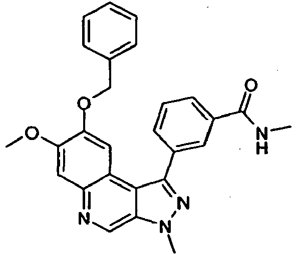 | 3-(8-Benzyloxy-7-methoxy-3-methyl-3H-pyrazolo[3,4-c]chinolin-1-yl)-N-methyl-benzamid | MS: 453,1 (M+H)⁺ |
| | | | DC (HPTLC): R_{f} = 0,42 (Essigsäureethylester/Ethanol 8:1, Volumenanteile) |

Die Verbindung der u.g. Strukturformel (Tab. 9b) kann methodisch nach den Synthesevorschriften zu den Beispielen 1, 8 und 9 - beginnend mit der Verbindung der Formel 3-2 - hergestellt werden.

**Tab. 9b Verbindung der Formel (I) und (IE)**

| **Nr.** | **Strukturformel** | **Name** | **Analytik** | **IC₅₀ DNA-PK [µM]** |
|---|---|---|---|---|
| 88 | 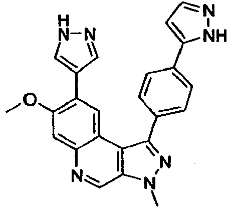 | 7-Methoxy-3-methyl-8-(1 H-pyrazol-4-yl)-1-[4-(2H-pyrazol-3-yl)-phenyl]-3H-pyrazolo[3,4-c]chinolin | MS: 422,1 (M+H)⁺ | < 0,1 |
| | | | DC (HPTLC): R_{f} = 0,40 (Dichlormethan/Ethanol 6:1, Volumenanteile) | |

### BEISPIEL 9: Synthese von 3-Fluor-4-(7-methoxy-3-methyl-8-chinolin-3-yl -3H-pyrazolo[3,4-c]chinolin-1-yl)-benzonitril

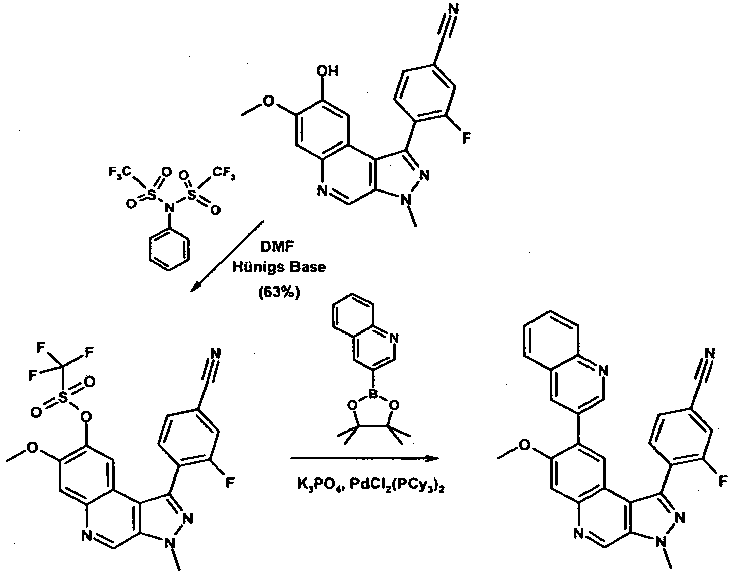

3-Fluor-4-(8-hydroxy-7-methoxy-3-methyl-3*H*-pyrazolo[3,4-c]chinolin-1-yl)-benzonitril (192 mg, 550 µmol), *N*-Phenyltrifluormethanesulfonimid (393 mg, 1,10 mmol) und Hünigsbase (374 µl, 2,20 mmol) werden in *N,N*-Dimethylformamid (22 ml) gelöst. Anschließend wird 30 min bei Raumtemperatur gerührt. Zur Aufarbeitung wird auf Wasser (100 ml) gegossen und weitere 30 min gerührt. Danach wird der entstandene Niederschlag abgesaugt und mit Wasser nachgewaschen. Der Nutschkuchen wird anschließend in wenig 2-Propanol suspendiert, erneut abgesaugt und im Hochvakuum über Nacht bei Raumtemperatur getrocknet, wobei Trifluormethansulfonsäure-[1-(4-cyano-2-fluor-phenyl)-7-methoxy-3-methyl-3*H*-pyrazolo[3,4-c]chinolin-8-yl]-ester (195 mg, 406 µmol) als Feststoff erhalten wird. MS: 481,0 (M+H⁺), DC (HPTLC): R_{f} = 0,56 (Essigsäureethylester / Ethanol 2:1, Volumenanteile).

Trifluormethansulfonsäure-[1-(4-cyano-2-fluor-phenyl)-7-methoxy-3-methyl-3*H-*pyrazolo[3,4-c]chinolin-8-yl]-ester (64 mg, 133 µmol), 3-(4,4,5,5-Tetramethyl-[1,3,2]dioxaborolan-2-yl)-chinolin (272 mg, 1,07 mmol), Trikaliumphosphat (58 mg, 267 µmol) und *trans*-Bis(tricyclohexylphosphan)-Palladium-(II)-dichlorid (30 mg, 41 µmol) werden in sauerstofffreiem *N,N*-Dimethylformamid (3,9 ml) gelöst. Anschließend wird 90 min auf 130°C erhitzt (Mikrowelle). Danach wird das Reaktionsgemisch abgesaugt, das Filtrat mit Wasser verdünnt und 30 min bei Raumtemperatur gerührt. Der entstandene Niederschlag wird abgesaugt und mit Wasser nachgewaschen. Der Rückstand wird in einem Gemisch von Dimethylsulfoxid und Tetrahydrofuran gelöst und chromatographiert (pHPLC, Lösungsmittelgradient Wasser / 1-30 vol.% Acetonitril, 0,1 vol.% Ameisensäure), wobei nach dem Gefriertrocknen die Titelverbindung 3-Fluor-4-(7-methoxy-3-methyl-8-chinolin-3-yl -3*H*-pyrazolo[3,4-c]chinolin-1-yl)-benzonitril (28 mg, 61 µmol) als Lyophilisat erhalten wird. MS: 460,1 (M+H⁺), DC (HPTLC): R_{f} = 0,32 (Essigsäureethylester / Ethanol 8:1, Volumenanteile).

Verbindungen, die entsprechend der Synthesevorschrift aus Beispiel 9 hergestellt werden, finden sich in der nachfolgenden Tabelle 10.

**Tab. 10 Verbindungen der Formeln (I) und (IA)**

| **Nr.** | **Strukturformel** | **Name** | **Analytik** | **IC₅₀ DNA-PK [µM]** |
|---|---|---|---|---|
| 77 | 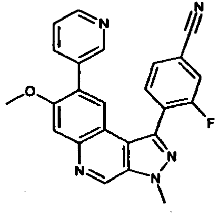 | 3-Fluor-4-(7-methoxy-3-methyl-8-pyridin-3-yl-3*H-*pyrazolo[3,4-c]chinolin-1-yl)-benzonitril | ¹H NMR (400 MHz, DMSO) δ = 9.49 (s, 1H), 8.65 (s, 1H), 8.56 (d, *J*=4.1, 1H), 8.16 (dd, *J*=9.9, 1.4, 1H), 7.98 (dd, *J*=7.6, 7.6, 1H), 7.93 - 7.84 (m, 2H), 7.81 (s, 1H), 7.65 (d, *J*=3.1, 1H), 7.47 (dd, *J*=7.7, 4.7, 1H), 4.38 (s, 3H), 3.96 (s, 3H). | < 0,1 |
| | | | MS: 410,1 (M+H)⁺ | |
| | | | DC (HPTLC): R_{f} = 0,30 (Essigsäure-ethylester/Ethanol 8:1, Volumenanteile) | |
| 78 | 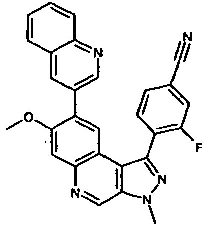 | 3-Fluor-4-(7-methoxy-3-methyl-8-chinolin-3-yl-3*H-*pyrazolo[3,4-c]chinolin-1-yl)-benzonitril | (Beispiel 9) | < 0,1 |
| 89 | 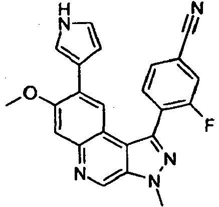 | 3-Fluoro-4-[7-methoxy-3-methyl-8-(1H-pyrrol-3-yl)-3H-pyrazolo[3,4-c]chinolin-1-yl]-benzonitril | MS: 398,1 (M+H)⁺ | < 0,1 |
| | | | DC (HPTLC): R_{f} = 0,49 (Essigsäure-ethylester) | |

### BEISPIEL 10: DNA-PK / Biochemischer Assay

Der Kinase-Assay erfolgte in mit Streptavidin beschichteten 348-Well-Mikrotiter-FlashPlates. Dazu wurden 1,5 µg DNA-PK-Proteinkomplex und 100 ng biotinyliertes Substrat, wie z.B. PESQEAFADLWKK-Biotin-NH2 ("Biotin-DNA-PK-Peptid"), in einem Gesamtvolumen von 36,5 µl (34,25 mM HEPES/KOH; 7,85 mM Tris-HCl; 68,5 mM KCl; 5 µM ATP; 6,85 mM MgCl₂; 0,5 mM EDTA; 0,14 mM EGTA; 0,69 mM DTT; pH 7,4) mit 500 ng DNA aus Kälberthymus, 0,1 µCi 33P-ATP und 1,8 % DMSO pro Well mit bzw. ohne die Testverbindung 90 min bei Raumtemperatur inkubiert. Die Reaktion wurde mit 50 µl/Well 200 mM EDTA gestoppt. Nach Inkubieren für weitere 30 min bei Raumtemperatur wurde die Flüssigkeit entfernt. Jedes Well wurde dreimal mit 100 µl 0,9 %-iger Kochsalzlösung gewaschen. Eine unspezifische Reaktion (Leerwert) wurde mit 10 µM eines eigenen Kinase-Inhibitors ermittelt. Die Radioaktivitätsmessung erfolgte mit einem TopCount. IC₅₀-Werte wurden in RS1 berechnet.
Literatur: Kashishian et al. (2003) Molecular Cancer Therapeutics 1257.

### BEISPIEL 11: Zelluläre DNA-PK Phosphorylierung an Serin 2056

HCT116 Zellen wurden in MEM alpha Medium mit 10% fötalem Kälberserum, 1 mM Natriumpyruvat und 2 mM Glutamin bei 37°C und 10% CO₂ kultiviert. Die Zellen wurden mithilfe von Trypsin/EDTA vom Boden der Kulturgefäße abgelöst, in Zentrifugenröhrchen abzentrifugiert und in frischem Medium aufgenommen. Anschließend wurde die Zelldichte ermittelt. 200.000 Zellen wurden in 1 ml Kulturmedium pro Kavität einer 12-Well Zellkulturplatte ausgesät und über Nacht kultiviert. Am nächsten Tag wurde 10 µM Bleomycin und Testsubstanzen in frischem Kulturmedium zu den Zellen hinzugefügt und diese für weitere sechs Stunden kultiviert. Im Anschluss erfolgte eine Zelllyse. Die Zelllysate wurden nach SDS-Polyacrylamidgelelektrophorese mittels DNA-PK spezifischerm Antikörper (Abcam ab13852: Gesamt-DNA-PK; ab18192: phospho-Serin 2056 DNA-PK) und Western Blotting untersucht. Die Entwicklung der enzymatischen Reaktion erfolgte mithilfe eines Chemiluminiszenzreagenz. Die Chemiluminiszenz wurde mithilfe eines Dokumentationssystems (VersaDoc^{™}, Bio-Rad, USA) aufgezeichnet und unter Zuhilfenahme der gerätespezifischen Software (Quantity One) densitometrisch ausgewertet. Die Signale mit phospho-DNA-PK spezifischen Antikörper wurden auf das Signal mit dem Antikörper gegen das Gesamtprotein DNA-PK normalisiert. Die Ermittlung von IC₅₀ Werten sowie prozentualer Inhibitionsdaten erfolgte durch Referenzierung auf das Signalniveau der Bleomycin behandelten Vehikelkontrollgruppe.

### BEISPIEL 12: Zellulärer Koloniewachstumstest

Die kolorektale Karzinomzelllinie HCT116 wurde in MEM alpha Medium mit 10% fötalem Kälberserum, 1 mM Natriumpyruvat und 2 mM Glutamin bei 37°C und 10% CO₂ kultiviert. Die Zellen wurden mithilfe von Trypsin/EDTA vom Boden der Kulturgefäße abgelöst, in Zentrifugenröhrchen abzentrifugiert und in frischem Medium aufgenommen. Anschließend wurde die Zelldichte ermittelt. 300 Zellen wurden in 2 ml Kulturmedium in 6-Well Zellkulturplatten ausgesät und über Nacht kultiviert. Am nächsten Tag wurden die Zellen mit Testsubstanzen für eine Stunde behandelt, bevor die Zellkulturplatten mit definierten Dosen Röntgenstrahlung (in der Regel 0, 2.4, 4.8, 12 Gray; Bestrahlungsgerät: Faxitron RX-650; Faxitron X-Ray LLC, USA) behandelt wurden. Zur Ermittlung von Dosiswirkungsbeziehungen wurden die Zellen mit verschiedenen Konzentrationen einer Testsubstanz behandelt. Nach Bestrahlung erfolgt eine weitere 24-stündige Kultivierung in Gegenwart der Testsubstanz, das Kulturmedium wurde dann gegen Kulturmedium ohne Testsubstanz ausgetauscht und die Zellen für weitere 6-8 Tage kultiviert. Im Anschluss wurden die gebildeten Zellkolonien mithilfe von Kristallviolett angefärbt und in einem Koloniezählgerät (Gelcount, Oxford Optronics, UK) ausgezählt. Die Ermittlung von Dosiswirkungskurven, insbesondere von IC₅₀-Werten, erfolgte über Anwendung einer Kurvenanpassungsfunktion für nichtlineare Dosiswirkungsbeziehungen.

| **Nr.** | **Strukturformel** | **Name** | **IC₅₀ [µM] (4,8 Gy)** | **Quotient aus IC₅₀-Werten ohne und mit Bestrahlung** |
|---|---|---|---|---|
| 90 | 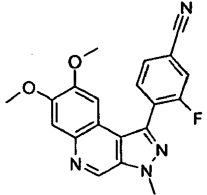 | 4-(7,8-Dimethoxy-3-methyl-3H-pyrazolo[3,4-c]chinolin-1-yl)-3-fluor-benzonitril | 0,1 - 0,5 | >75 |

### BEISPIEL 13: Zelluläre CHK2 Phosphorylierung an Threonin 68

HCT116 Zellen wurden in MEM alpha Medium mit 10% fötalem Kälberserum, 1 mM Natriumpyruvat und 2 mM Glutamin bei 37°C und 10% CO2 kultiviert. Die Zellen wurden mithilfe von Trypsin/EDTA vom Boden der Kulturgefäße abgelöst, in Zentrifugenröhrchen abzentrifugiert und in frischem Medium aufgenommen. Anschließend wurde die Zelldichte ermittelt. 50.000 Zellen wurden in 0,1 ml Kulturmedium pro Kavität einer 96-well Zellkulturplatte ausgesät und über Nacht kultiviert. Am nächsten Tag wurden 10 µM Bleomycin und Testsubstanzen in frischem Kulturmedium zu den Zellen hinzugefügt und diese für weitere sechs Stunden kultiviert. Nach Lyse der Zellen erfolgte die Detektion von phospho-Threonin 68 der CHK2 Kinase in den Lysaten mithilfe eines phospho-CHK2 (Thr68) spezifischen ELISA Detektionsystems (Katalognr. 7037, Cell Signaling Technologies, USA). Die ELISA Farbreaktion wurde spektralphotometrisch bei 450 nm gemessen. Die Extinktion der unstimulierten Kontrollen (Vehikelkontrolle ohne Bleomycin) wurde von den Extinktionswerten der Behandlungsgruppen subtrahiert. Die Kontrollen, die mit Bleomycin behandelt wurden, wurden gleich 100% gesetzt und alle anderen Extinktionswerte hierzu in Beziehung gesetzt. Die Bestimmung von IC₅₀-Werten erfolgte mithilfe der Statistikprogramme GraphPad Prism (GraphPad Software, USA) oder Assay Explorer (Symyx Technologies Inc., USA).

| **Nr.** | **Strukturformell** | **Name** | **IC₅₀ [µM]** |
|---|---|---|---|
| 91 | 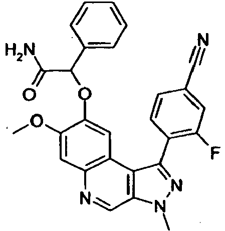 | 2-[1-(4-Cyano-2-fluorphenyl)-7-methoxy-3-methyl-3H-pyrazolo[3,4-c]chinolin-8-yloxy]-2-phenyl-acetamid | 0,1-0,5 |

### BEISPIEL 14: Pharmazeutische Zubereitungen

### Beispiel A: Iniektionsgläser

Eine Lösung von 100 g erfindungsgemäßem Wirkstoff und 5 g Dinatriumhydrogenphosphat wurde in 3 I zweifach destilliertem Wasser mit 2 N Salzsäure auf pH 6,8 eingestellt, steril filtriert, in Injektionsgläser abgefüllt, unter sterilen Bedingungen lyophilisiert und steril verschlossen. Jedes Injektionsglas enthielt 5 mg erfindungsgemäßen Wirkstoff.

### Beispiel B: Suppositorien

Man schmolz ein Gemisch von 20 g erfindungsgemäßem Wirkstoff mit 100 g Sojalecithin und 1400 g Kakaobutter, goss es in Formen und lies es erkalten. Jedes Suppositorium enthielt 20 mg erfindungsgemäßen Wirkstoff.

### Beispiel C: Lösung

Man bereitete eine Lösung aus 1 g erfindungsgemäßem Wirkstoff, 9,38 g NaH₂PO₄ *2 H₂O, 28,48 g Na₂HPO₄ *12 H₂O und 0,1 g Benzalkoniumchlorid in 940 ml zweifach destilliertem Wasser. Man stellte auf pH 6,8 ein, füllte auf 1 I auf und sterilisierte durch Bestrahlung. Diese Lösung konnte in Form von Augentropfen verwendet werden.

### Beispiel D: Salbe

Man mischte 500 mg erfindungsgemäßen Wirkstoff mit 99,5 g Vaseline unter aseptischen Bedingungen.

### Beispiel E: Tabletten

Ein Gemisch von 1 kg erfindungsgemäßem Wirkstoff, 4 kg Laktose, 1,2 kg Kartoffelstärke, 0,2 kg Talk und 0,1 kg Magnesiumstearat wurde in üblicher Weise zu Tabletten verpresst, derart, dass jede Tablette 10 mg erfindungsgemäßen Wirkstoff enthielt.

### Beispiel F: Dragees

Analog Beispiel E wurden Tabletten gepresst, die danach in üblicher Weise mit einem Überzug aus Saccharose, Kartoffelstärke, Talk, Tragant und Farbstoff überzogen wurden.

### Beispiel G: Kapseln

2 kg erfindungsgemäßer Wirkstoff wurden in üblicher Weise in Hartgelatinekapseln gefüllt, so dass jede Kapsel 20 mg erfindungsgemäßen Wirkstoff enthielt.

### Beispiel H: Ampullen

Eine Lösung von 1 kg erfindungsgemäßem Wirkstoff in 60 I zweifach destilliertem Wasser wurde steril filtriert, in Ampullen abgefüllt, unter sterilen Bedingungen lyophilisiert und steril verschlossen. Jede Ampulle enthielt 10 mg erfindungsgemäßen Wirkstoff.

### Beispiel I: Inhalations-Spray

Man löste 14 g erfindungsgemäßen Wirkstoff in 10 I isotonischer NaCl-Lösung und füllte die Lösung in handelsübliche Sprühgefäße mit Pump-Mechanismus. Die Lösung konnte in Mund oder Nase gesprüht werden. Ein Sprühstoß (etwa 0,1 ml) entsprach einer Dosis von etwa 0,14 mg.

## Patentansprüche

1. Verbindungen der Formel (I) 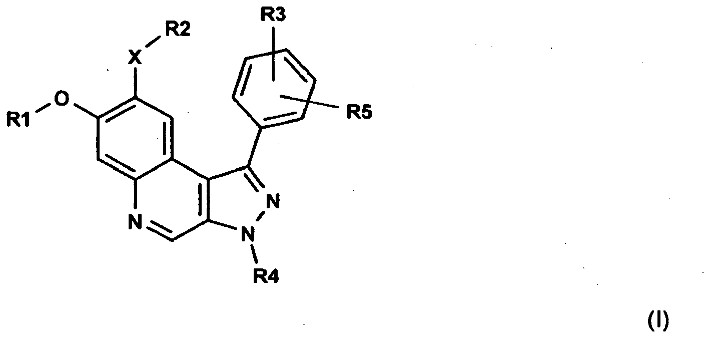 worin
R1 Y, -Alk-OY, -Alk-NYY oder -Alk-Ar,
R2 Y, -Alk-OY, -Alk-NYY, -C(Y)(R6)(R7), -C(Hal)(R6)(R7), -SO₂A, -SO₂-Ar oder -POOH-Ar,
R3 H, Hal, CN, -Alk-CN, -Alk-NYY, Het¹ oder Het²,
R4 Hal, Y, Cyc, CN, -Alk-CN, -Alk-COOY, -Alk-CO-NYY oder Het¹,
R5 Hal, Y, OY, NYY, -NY-COY, COOY, -CO-NYY, -CO-NY-Alk-OY, -Alk-CO-NYY, -Alk-OY, -Alk-NYY, Ar, Het¹ oder Het²,
R3, R5 zusammen auch -Alk-CO-NY-,
R6 Hal, Y, -COOY, -CO-NYY, -CO-NY-OY, -CO-NY-C(=NH)-NYY, -CO-NY-Alk-OY, -CO-NY-Alk-NYY, -CO-NY-Alk-SO₂-NYY, -CO-NY-Alk-Ar, -CO-NY-Alk-Het² oder -CO-NY-O-Alk-CN,
R7 Ar, Het¹ oder -Het¹-Het¹,
X CH₂, O, S oder Het¹,
Y H oder A,
A unverzweigtes oder verzweigtes Alkyl mit 1-10 C-Atomen, wobei unabhängig voneinander 1-7 H-Atome durch Hal und/oder unabhängig voneinander ein oder zwei benachbarte CH₂-Gruppen durch eine -CH=CH- und/oder -CΞC- Gruppe ersetzt sein können,
Alk Alkylen mit 1-6 C-Atomen, wobei unabhängig voneinander 1-4 H-Atome durch Hal und/oder OY ersetzt sein können,
Cyc zyklisches Alkyl mit 3-7 C-Atomen, wobei unabhängig voneinander 1-4 H-Atome durch Hal und/oder OY ersetzt sein können,
Ar unsubstituiertes oder einfach durch Hal, A, CN, OY, NYY, -NY-COY, COOY, Het¹, Het², -Alk-OY, -Alk-NYY, -Alk-Het¹ oder Alk-Het² substituiertes Phenyl,
Het¹ ein- oder zweikerniges Heteroaryl mit 2-9 C-Atomen und 1-4 N-, O- und/oder S-Atomen, das unsubstituiert oder einfach durch Hal, A, CN, OY, NYY, -NY-COY, COOY, -Alk-OY oder -Alk-NYY substituiert sein kann,
Het² einen einkernigen gesättigten Heterozyklus mit 2-7 C-Atomen und 1-4 N-, O- und/oder S-Atomen, der unsubstituiert oder einfach durch A substituiert sein kann, und
Hal F, Cl, Br oder I bedeuten,
und/oder ihre physiologisch unbedenklichen Salze, Tautomere und/oder Stereoisomere, einschließlich deren Mischungen in allen Verhältnissen.

2. Verbindungen nach Anspruch 1 mit der Teilformel (IA) 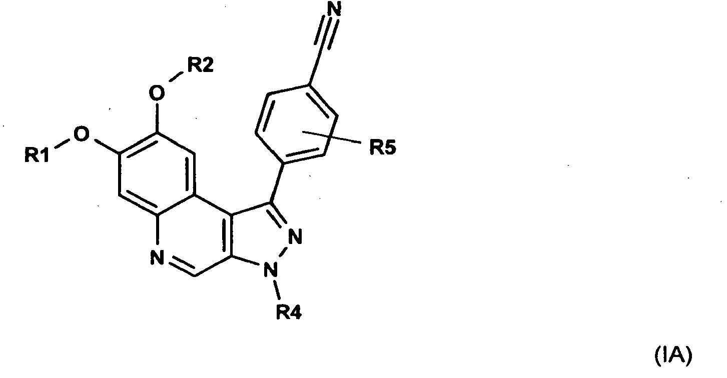 worin
R1, R4 Y,
R2 Y oder -CH(R6)(R7),
R5 Hal, Y, COOY, Alk-OA oder Het²,
R6 -CO-NYY, -CO-NY-OY, -CO-NY-C(=NH)-NYY oder -CO-NY-Alk-OY,
R7 Ar oder Het¹,
Y H oder A,
A unverzweigtes oder verzweigtes Alkyl mit 1-4 C-Atomen, wobei unabhängig voneinander 1-3 H-Atome durch Hal ersetzt sein können,
Alk Alkylen mit 1-3 C-Atomen, wobei 1-2 H-Atome durch Hal und/oder OH ersetzt sein können,
Ar unsubstituiertes oder einfach durch Hal substituiertes Phenyl,
Het¹ ein- oder zweikerniges Heteroaryl mit 2-9 C-Atomen und 1-3 N- und/oder S-Atomen, das unsubstituiert oder einfach durch Hal, A, CN oder NYY substituiert sein kann,
Het² einen einkernigen gesättigten Heterozyklus mit 3-5 C-Atomen und 1-2 N- und/oder O-Atomen, der unsubstituiert oder einfach durch A substituiert sein kann, und
Hal F, Cl, Br oder I bedeuten,
und/oder ihre physiologisch unbedenklichen Salze, Tautomere und/oder Stereoisomere, einschließlich deren Mischungen in allen Verhältnissen.

3. Verbindungen nach Anspruch 1 oder 2, ausgewählt aus der Gruppe:
| | |
|---|---|
| 1 | 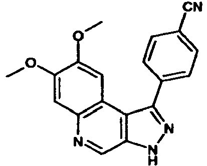 |
| 2 | 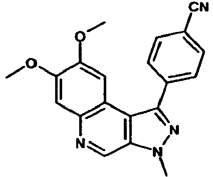 |
| 3 | 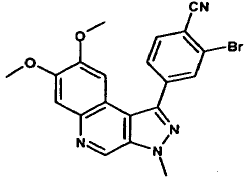 |
| 4 | 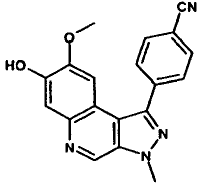 |
| 5 | 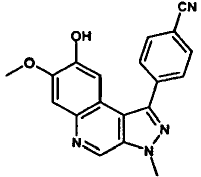 |
| 6 | 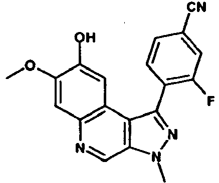 |
| 7 | 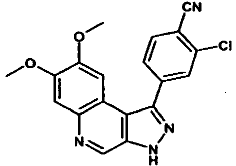 |
| 8 | 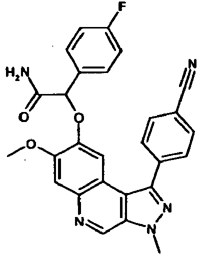 |
| 9 | 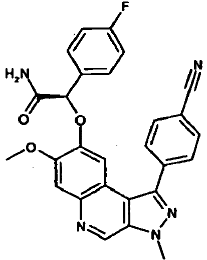 |
| 10 | 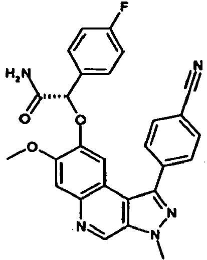 |
| 11 | 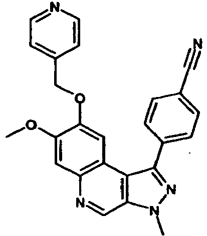 |
| 12 | 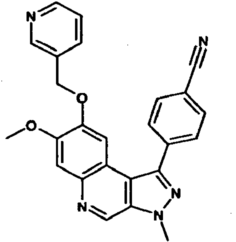 |
| 13 | 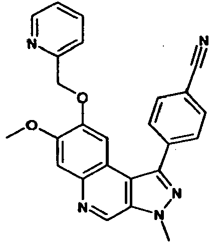 |
| 14 | 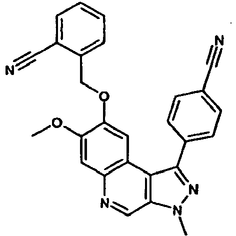 |
| 15 | 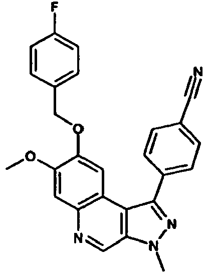 |
| 16 | 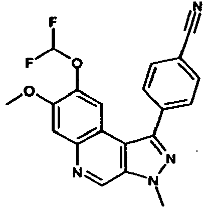 |
| 17 | 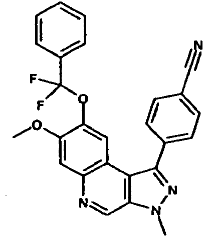 |
| 18 | 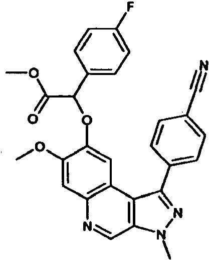 |
| 19 | 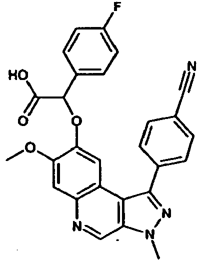 |
| 20 | 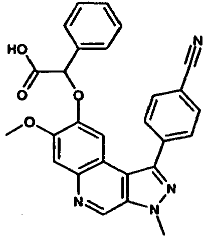 |
| 21 | 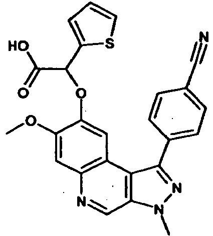 |
| 22 | 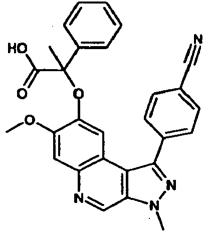 |
| 23 | 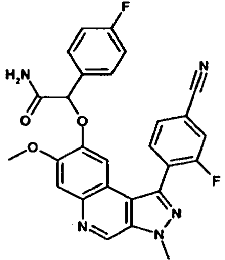 |
| 24 | 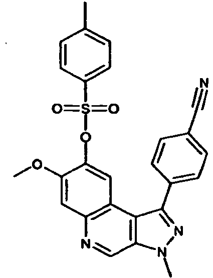 |
| 25 | 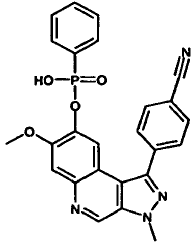 |
| 26 | 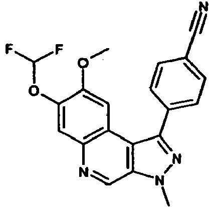 |
| 27 | 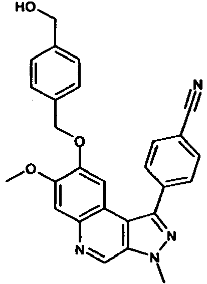 |
| 28 | 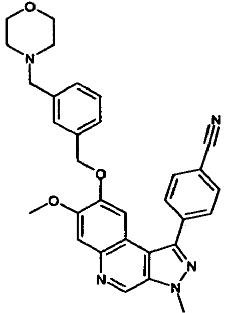 |
| 29 | 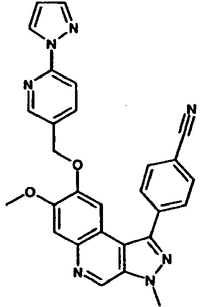 |
| 30 | 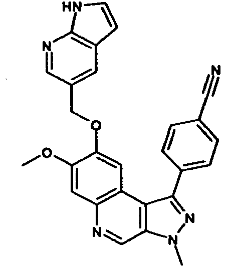 |
| 31 | 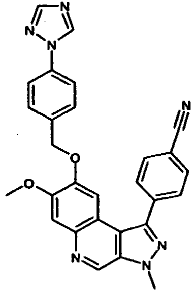 |
| 32 | 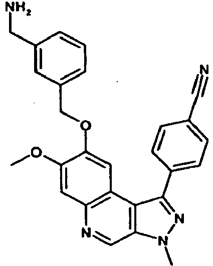 |
| 33 | 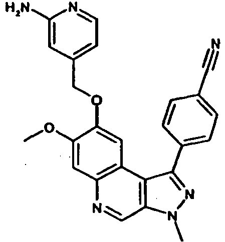 |
| 34 | 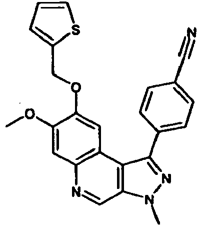 |
| 35 | 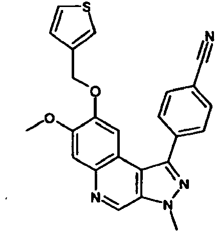 |
| 36 | 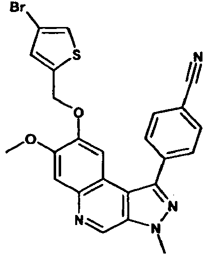 |
| 37 | 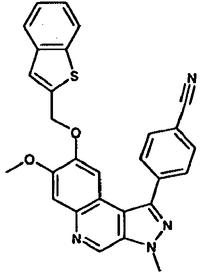 |
| 38 | 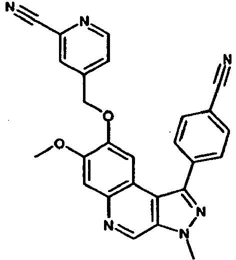 |
| 39 | 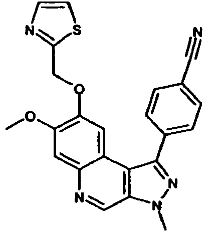 |
| 40 | 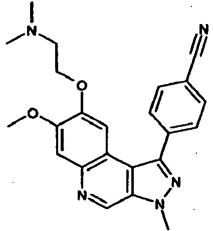 |
| 41 | 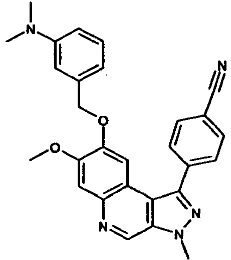 |
| 42 | 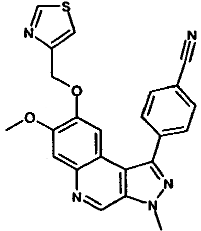 |
| 43 | 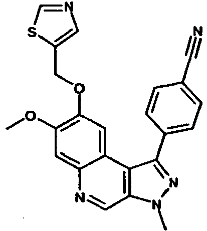 |
| 44 | 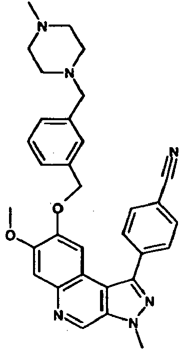 |
| 45 | 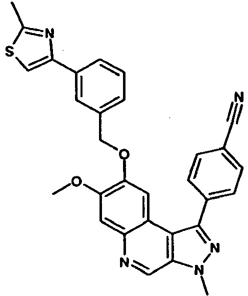 |
| 46 | 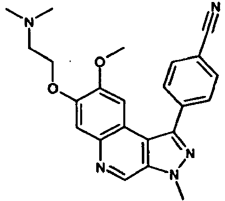 |
| 47 | 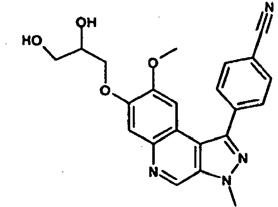 |
| 48 | 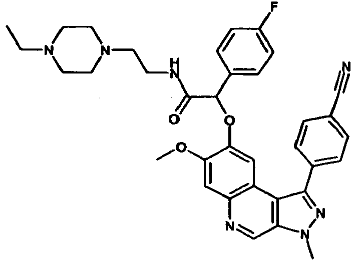 |
| 49 | 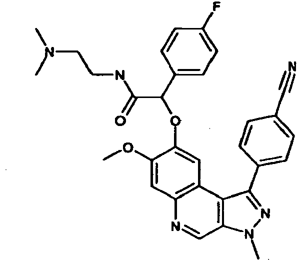 |
| 50 | 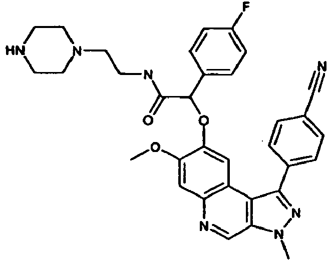 |
| 51 | 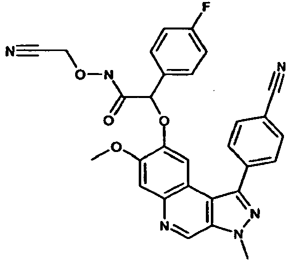 |
| 52 | 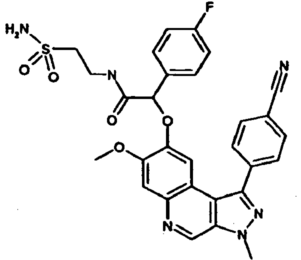 |
| 53 | 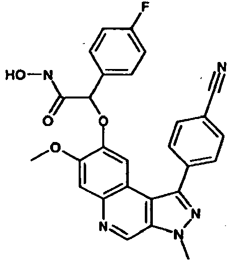 |
| 54 | 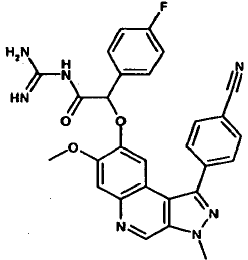 |
| 55 | 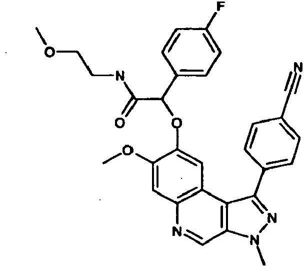 |
| 56 | 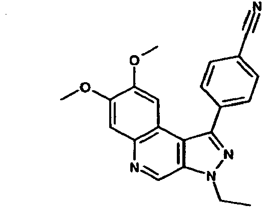 |
| 57 | 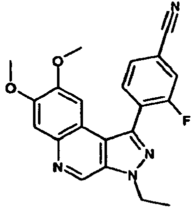 |
| 58 | 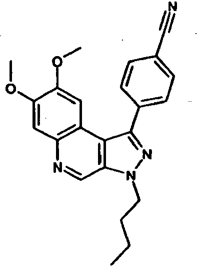 |
| 59 | 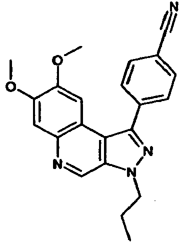 |
| 60 | 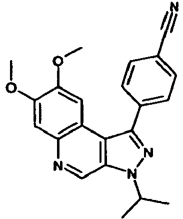 |
| 61 | 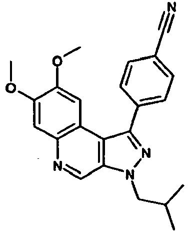 |
| 62 | 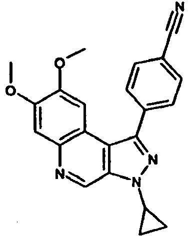 |
| 63 | 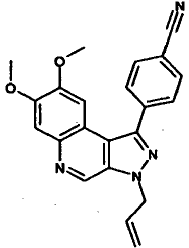 |
| 64 | 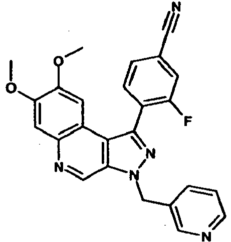 |
| 65 | 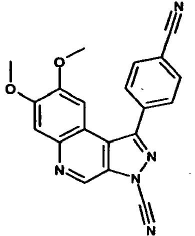 |
| 66 | 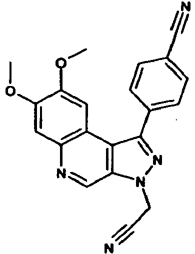 |
| 67 | 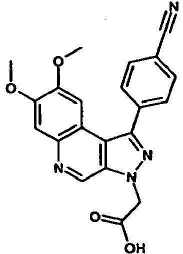 |
| 68 | 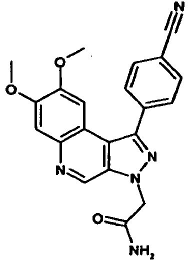 |
| 69 | 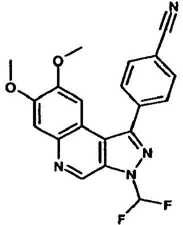 |
| 70 | 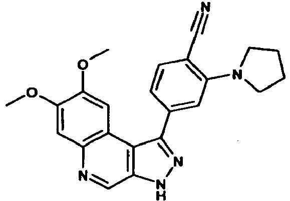 |
| 71 | 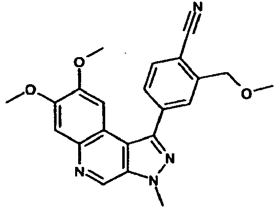 |
| 72 | 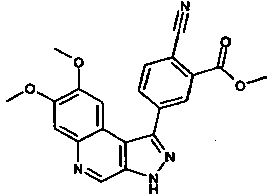 |
| 73 | 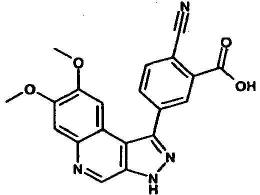 |
| 74 | 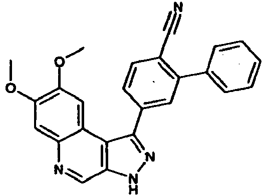 |
| 75 | 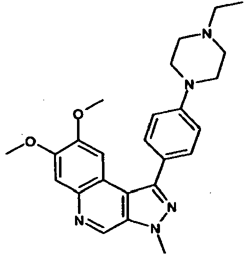 |
| 76 | 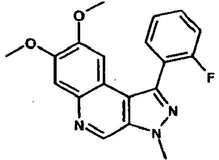 |
| 77 | 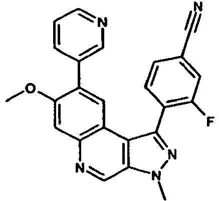 |
| 78 | 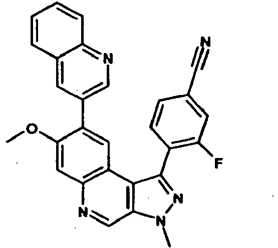 |
| 79 | 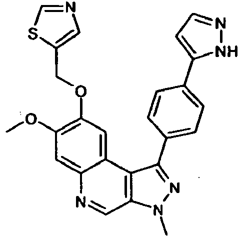 |
| 80 | 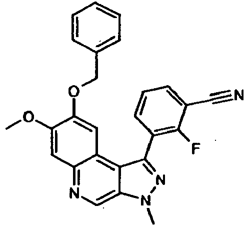 |
| 81 | 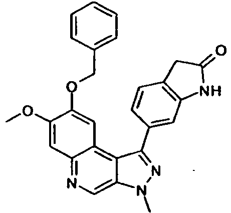 |
| 82 | 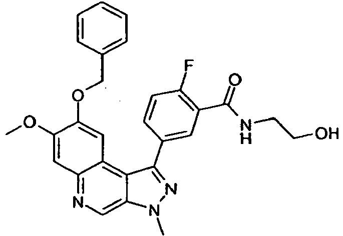 |
| 83 | 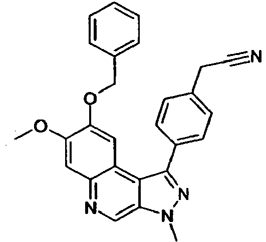 |
| 84 | 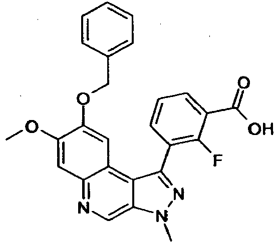 |
| 85 | 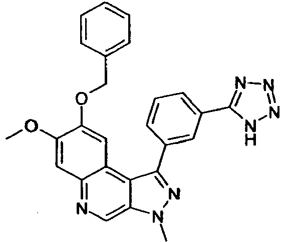 |
| 86 | 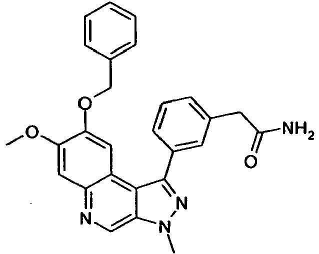 |
| 87 | 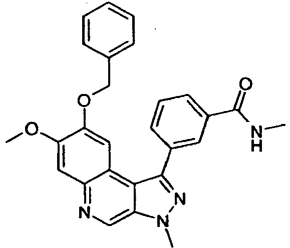 |
| 88 | 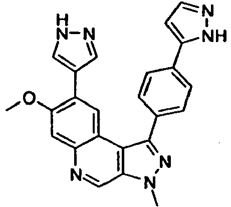 |
| 89 | 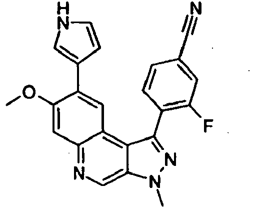 |
| 90 | 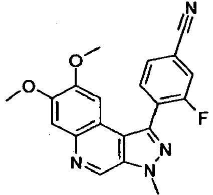 |
| 91 | 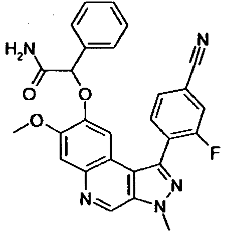 |
und/oder ihre physiologisch unbedenklichen Salze, Tautomere und/oder Stereoisomere, einschließlich deren Mischungen in allen Verhältnissen.

4. Zwischenverbindungen der Formel (II) 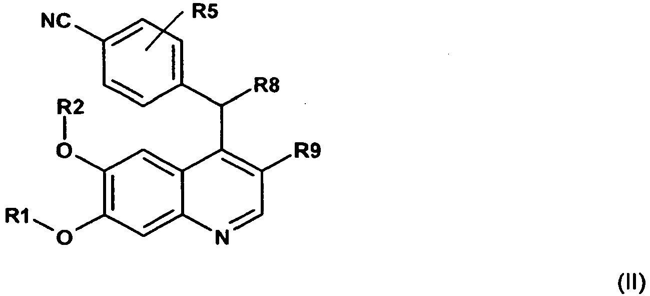 worin
R8 CN oder =O, und
R9 NO₂ oder NYY bedeuten, und
R1, R2, R5 und Y die in Anspruch 1 angegebene Bedeutung aufweisen,
und/oder ihre Salze, Tautomere und/oder Stereoisomere, einschließlich deren Mischungen in allen Verhältnissen.

5. Zwischenverbindungen nach Anspruch 4 mit der Teilformel (IIA) 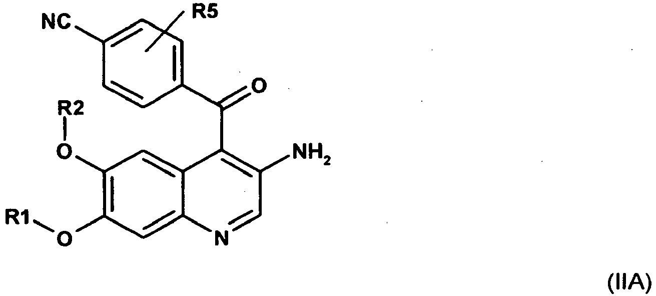 worin
R1, R2 unabhängig voneinander A oder -Alk-Ar, und
Alk Alkylen mit 1-3 C-Atomen, wobei 1-2 H-Atome durch Hal ersetzt sein können, bedeuten, und
R5, A, Ar und Hal die in Anspruch 2 angegebene Bedeutung aufweisen,
und/oder ihre Salze, Tautomere und/oder Stereoisomere, einschließlich deren Mischungen in allen Verhältnissen.

6. Zwischenverbindungen der Formel (III) 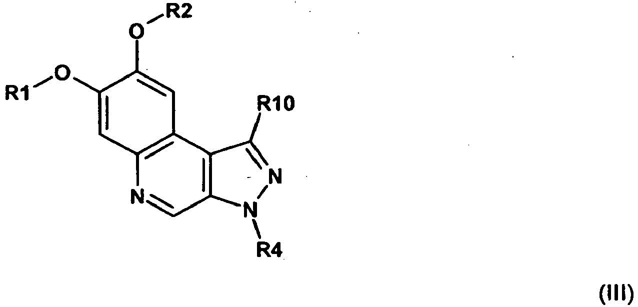 worin
R10 H oder Hal bedeutet, und
R1, R2, R4 und Hal die in Anspruch 1 angegebene Bedeutung aufweisen,
und/oder ihre Salze, Tautomere und/oder Stereoisomere, einschließlich deren Mischungen in allen Verhältnissen.

7. Zwischenverbindungen nach Anspruch 6, worin
R1, R2 unabhängig voneinander A oder -Alk-Ar,
R10 Hal, und
Alk Alkylen mit 1-3 C-Atomen, wobei 1-2 H-Atome durch Hal ersetzt sein können, bedeuten, und
R4, A, Ar und Hal die in Anspruch 2 angegebene Bedeutung aufweisen,
und/oder ihre physiologisch unbedenklichen Salze, Tautomere und/oder Stereoisomere, einschließlich deren Mischungen in allen Verhältnissen.

8. Verfahren zum Herstellen von Verbindungen der Formel (I) nach Anspruch 1, Teilformeln davon und/oder ihrer physiologisch unbedenklichen Salze, Tautomere und/oder Stereoisomere, einschließlich deren Mischungen in allen Verhältnissen, mit den folgenden Schritten:
(a) Umsetzen einer Verbindung der Teilformel (IIA) 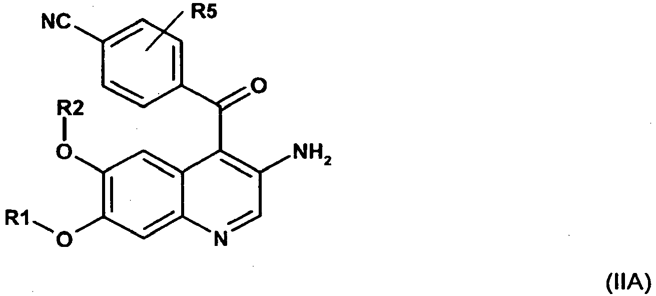 worin R1, R2 und R5 die in Anspruch 5 angegebene Bedeutung aufweisen,
im sauren Milieu mit einem Reduktionsmittel und mit einer Verbindung E-NO₂, worin E ein Element der 1. Hauptgruppe bedeutet,
unter Erhalt von Verbindungen der Teilformel (IB) 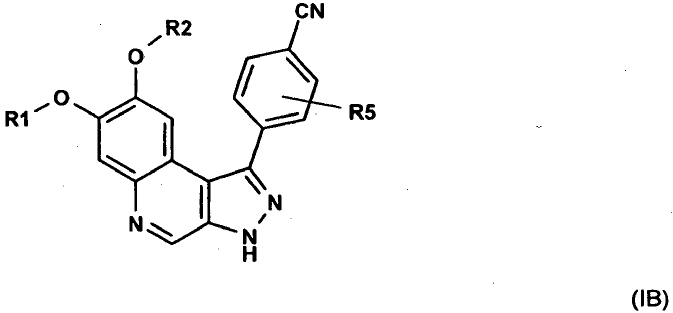 worin R1, R2 und R5 die in Anspruch 5 angegebene Bedeutung aufweisen,
und gegebenenfalls
(b') Umsetzen der Verbindungen der Teilformel (IB) mit einer Verbindung Hal-R4, worin R4 und Hal die in Anspruch 1 angegebene Bedeutung aufweisen,
unter Erhalt von Verbindungen der Teilformel (IC) 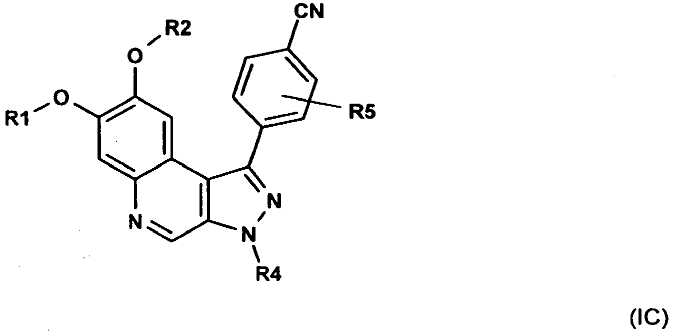 worin R1, R2 und R5 die in Anspruch 5 angegebene Bedeutung aufweisen, und R4 die in Anspruch 1 angegebene Bedeutung aufweist,
(b") Umwandeln von R1, -O-R2, R4, R5 und/oder der CN-Gruppe der Verbindungen der Teilformel (IC) unter Erhalt von Verbindungen der Teilformel (IE) 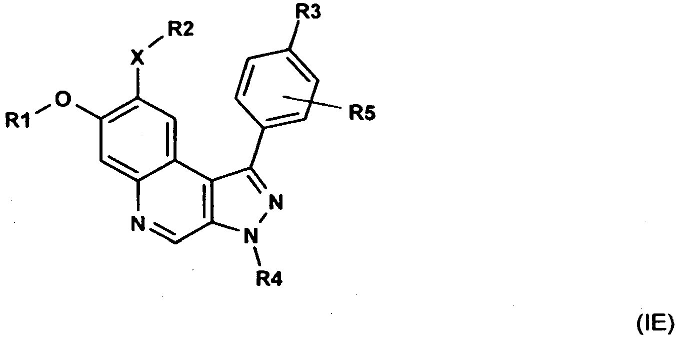 worin R1, R2, R3, R4, R5 und X die in Anspruch 1 angegebene Bedeutung aufweisen,
und/oder
(b"') Umwandeln einer Base oder Säure der Verbindungen der Teilformel (IE) oder Teilformeln (IB) oder (IC) in eines ihrer physiologisch unbedenklichen Salze,
oder
(a) Umsetzen einer Verbindung der Formel (III) 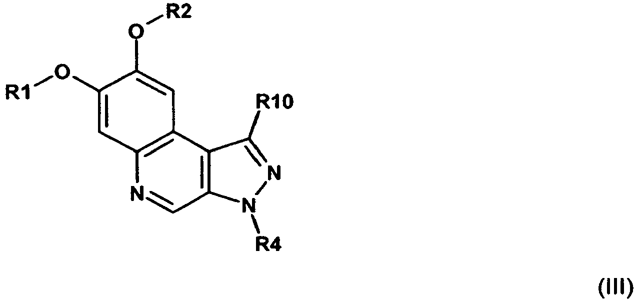 worin R1, R2, R4 und R10 die in Anspruch 7 angegebene Bedeutung aufweisen,
mit einer Verbindung der Formel (IV) 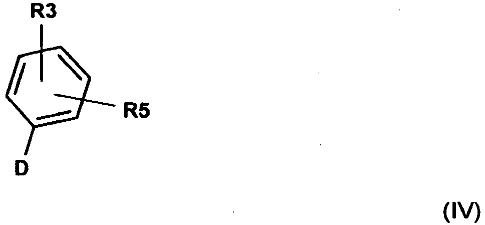 worin
D Borsäure, Borester, organische Zinnverbindung oder Bortrifluormethansulfonat bedeutet, und
R3 und R5 die in Anspruch 1 angegebene Bedeutung aufweisen,
unter Erhalt von Verbindungen der Teilformel (ID) 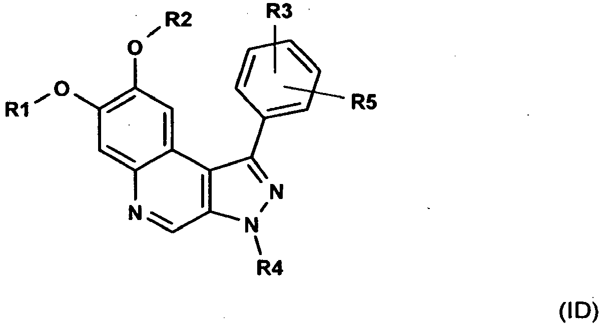 worin R1, R2 und R4 die in Anspruch 7 angegebene Bedeutung aufweisen, und R3 und R5 die in Anspruch 1 angegebene Bedeutung aufweisen,
und gegebenenfalls
(b') Umwandeln von R1, -O-R2, R3, R4 und/oder R5 der Verbindungen der Teilformel (ID) unter Erhalt von Verbindungen der Formel (I) 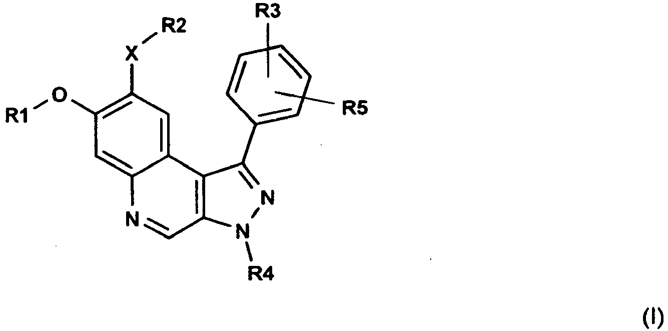 worin R1, R2, R3, R4, R5 und X die in Anspruch 1 angegebene Bedeutung aufweisen,
und/oder
(b") Umwandeln einer Base oder Säure der Verbindungen der Formel (I) oder Teilformel (ID) in eines ihrer physiologisch unbedenklichen Salze.

9. Verfahren zum Herstellen von Zwischenverbindungen der Formel (II) nach Anspruch 4 und/oder ihrer Salze, Tautomere und/oder Stereoisomere, einschließlich deren Mischungen in allen Verhältnissen, mit den folgenden Schritten:
(a) Umsetzen einer Verbindung der Formel (V) 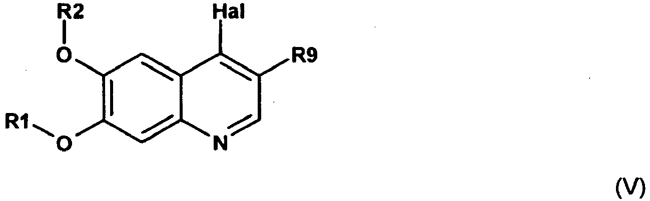 worin
Hal F, Cl, Br oder I bedeutet, und
R1, R2 und R9 die in Anspruch 4 angegebene Bedeutung aufweisen,
mit einer Verbindung der Formel (VI) 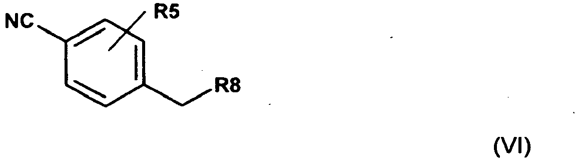 worin R5 und R8 die in Anspruch 4 angegebene Bedeutung aufweisen,
unter Erhalt von Verbindungen der Formel (II) 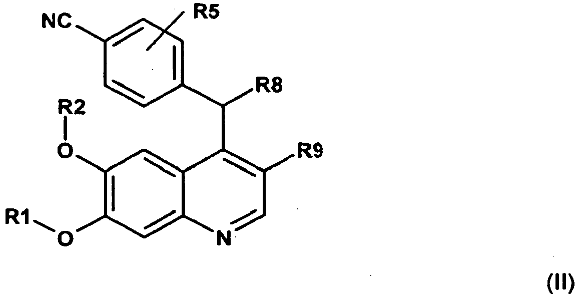 worin R1, R2, R5, R8 und R9 die in Anspruch 4 angegebene Bedeutung aufweisen,
und gegebenenfalls
(b) Umwandeln einer Base oder Säure der Verbindungen der Formel (II) in eines ihrer Salze.

10. Verfahren zum Herstellen von Zwischenverbindungen der Formel (III) nach Anspruch 6, Teilformeln davon und/oder ihrer Salze, Tautomere und/oder Stereoisomere, einschließlich deren Mischungen in allen Verhältnissen, mit den folgenden Schritten:
(a) Umsetzen einer Verbindung der Formel (VII) 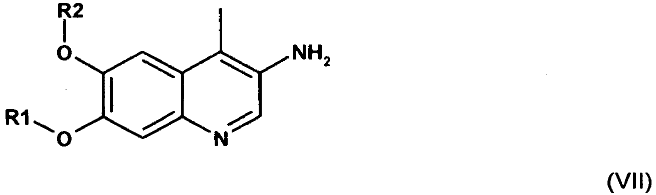 worin R1 und R2 die in Anspruch 6 angegebene Bedeutung aufweisen,
im sauren Milieu mit einer Verbindung E-NO₂, worin E ein Element der 1. Hauptgruppe bedeutet,
unter Erhalt von Verbindungen der Teilformel (IIIA) 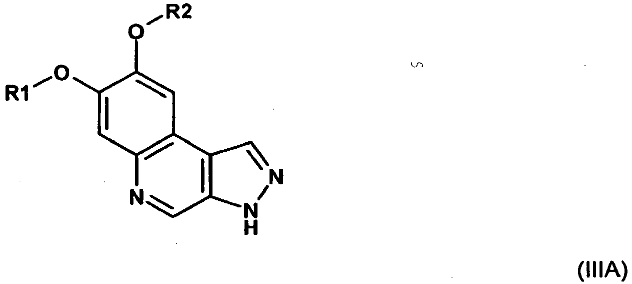 worin R1 und R2 die in Anspruch 6 angegebene Bedeutung aufweisen,
und gegebenenfalls
(b') Halogenieren der Verbindungen der Teilformel (IIIA) unter Erhalt von Verbindungen der Teilformel (IIIB) 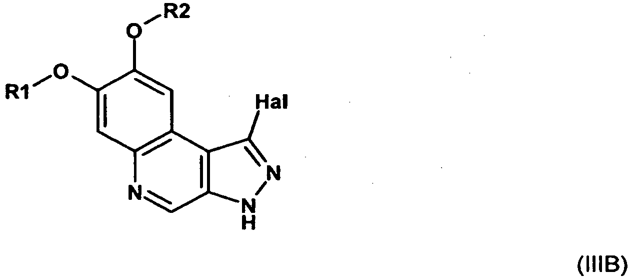 worin R1, R2 und Hal die in Anspruch 6 angegebene Bedeutung aufweisen,
(b") Umsetzen der Verbindungen der Formel (IIIA) oder (IIIB) mit einer Verbindung Hal-R4, worin R4 und Hal die in Anspruch 6 angegebene Bedeutung aufweisen,
unter Erhalt von Verbindungen der Formel (III) 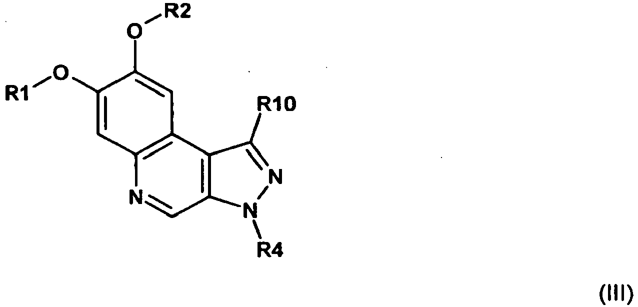 worin R1, R2, R4 und R10 die in Anspruch 6 angegebene Bedeutung aufweisen,
und/oder
(b"') Umwandeln einer Base oder Säure der Verbindungen der Formel (III) in eines ihrer Salze.

11. Verwendung von Verbindungen nach einem der Ansprüche 1 bis 3 und/oder ihrer physiologisch unbedenklichen Salze, Tautomere und/oder Stereoisomere, einschließlich deren Mischungen in allen Verhältnissen, zur Herstellung eines Arzneimittels zur Hemmung von Serin-Threonin-Proteinkinasen, vorzugsweise PIKK, besonders bevorzugt DNA-PK.

12. Verwendung von mindestens einer Verbindung nach einem der Ansprüche 1 bis 3 und/oder ihrer physiologisch unbedenklichen Salze, Tautomere und/oder Stereoisomere, einschließlich deren Mischungen in allen Verhältnissen, zur ensibilisierung von Krebszellen gegenüber Antikrebsmittel und/oder ionisierender Strahlung unter der Maßgabe, dass die Sensibilisierung in-vivo nicht am menschlichen oder tierischen Körper erfolgt.

13. Arzneimittel umfassend mindestens eine Verbindung nach einem der Ansprüche 1 bis 3 und/oder ihre physiologisch unbedenklichen Salze, Tautomere und/oder Stereoisomere, einschließlich deren Mischungen in allen Verhältnissen.

14. Pharmazeutische Zusammensetzung umfassend als Wirkstoff eine wirksame Menge von mindestens einer Verbindung nach einem der Ansprüche 1 bis 3 und/oder ihrer physiologisch unbedenklichen Salze, Tautomere und/oder Stereoisomere, einschließlich deren Mischungen in allen Verhältnissen, zusammen mit pharmazeutisch verträglichen Hilfsstoffen, in Kombination mit mindestens einem Antikrebsmittel.

15. Verbindungen nach einem der Ansprüche 1 bis 3 und/oder ihre physiologisch unbedenklichen Salze, Tautomere und/oder Stereoisomere, einschließlich deren Mischungen in allen Verhältnissen, zur Verwendung in der Prophylaxe, Therapie und/oder Verlaufskontrolle von Krebs, Tumoren, Metastasen und/oder Störungen der Angiogenese, in Kombination mit Radiotherapie und/oder mit mindestens einem Antikrebsmittel.

## Claims

1. Compounds of the formula (I) 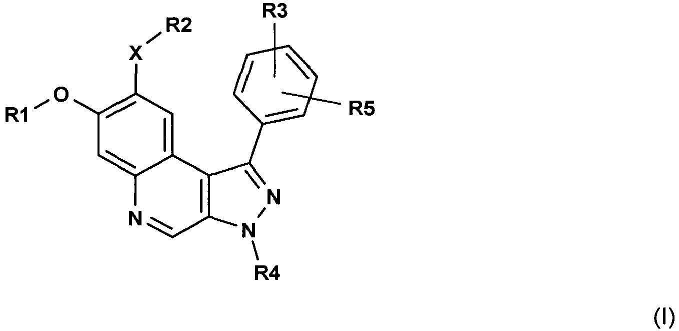 in which
R1 denotes Y, -Alk-OY, -Alk-NYY or -Alk-Ar,
R2 denotes Y, -Alk-OY, -Alk-NYY, -C(Y)(R6)(R7), -C(Hal)(R6)(R7), -SO₂A, -SO₂-Ar or -POOH-Ar,
R3 denotes H, Hal, CN, -Alk-CN, -Alk-NYY, Het¹ or Het²,
R4 denotes Hal, Y, Cyc, CN, -Alk-CN, -Alk-COOY, -Alk-CO-NYY or Het¹,
R5 denotes Hal, Y, OY, NYY, -NY-COY, COOY, -CO-NYY, -CO-NY-Alk-OY, -Alk-CO-NYY, -Alk-OY, -Alk-NYY, Ar, Het¹ or Het²,
R3, R5 together also denote -Alk-CO-NY,
R6 denotes Hal, Y, -COOY, -CO-NYY, -CO-NY-OY, -CO-NY-C(=NH)-NYY, -CO-NY-Alk-OY, -CO-NY-Alk-NYY, -CO-NY-Alk-SO₂-NYY, -CO-NY-Alk-Ar, -CO-NY-Alk-Het² or -CO-NY-O-Alk-CN,
R7 denotes Ar, Het¹ or -Het¹-Het¹,
X denotes CH₂, O, S or Het¹,
Y denotes H or A,
A denotes unbranched or branched alkyl having 1-10 C atoms, where, independently of one another, 1-7 H atoms may be replaced by Hal and/or, independently of one another, one or two adjacent CH₂ groups may be replaced by a -CH=CH- and/or -CΞC- group,
Alk denotes alkylene having 1-6 C atoms, where, independently of one another, 1-4 H atoms may be replaced by Hal and/or OY,
Cyc denotes cyclic alkyl having 3-7 C atoms, where, independently of one another, 1-4 H atoms may be replaced by Hal and/or OY,
Ar denotes phenyl which is unsubstituted or monosubstituted by Hal, A, CN, OY, NYY, -NY-COY, COOY, Het¹, Het², -Alk-OY, -Alk-NYY, -Alk-Het¹ or Alk-Het²,
Het¹ denotes mono- or bicyclic heteroaryl having 2-9 C atoms and 1-4 N, O and/or S atoms, which may be unsubstituted or monosubstituted by Hal, A, CN, OY, NYY, -NY-COY, COOY, -Alk-OY or -Alk-NYY,
Het² denotes a monocyclic saturated heterocycle having 2-7 C atoms and 1-4 N, O and/or S atoms, which may be unsubstituted or monosubstituted by A, and
Hal denotes F, Cl, Br or I,
and/or physiologically acceptable salts, tautomers and/or stereoisomers thereof, including mixtures thereof in all ratios.

2. Compounds according to Claim 1 having the part-formula (IA) 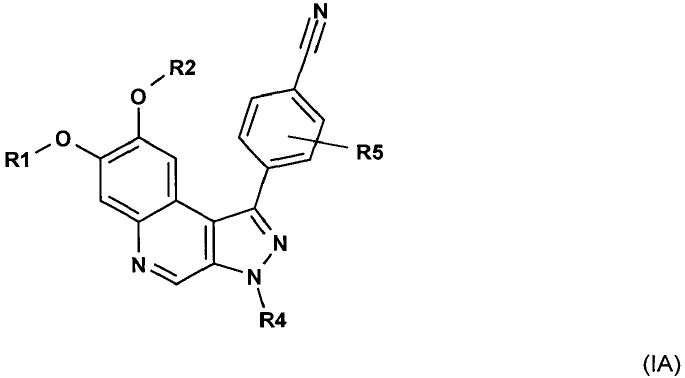 in which
R1, R4 denote Y,
R2 denotes Y or -CH(R6)(R7),
R5 denotes Hal, Y, COOY, Alk-OA or Het²,
R6 denotes -CO-NYY, -CO-NY-OY, -CO-NY-C(=NH)-NYY or -CO-NY-Alk-OY,
R7 denotes Ar or Het¹,
Y denotes H or A,
A denotes unbranched or branched alkyl having 1-4 C atoms, where, independently of one another, 1-3 H atoms may be replaced by Hal,
Alk denotes alkylene having 1-3 C atoms, where 1-2 H atoms may be replaced by Hal and/or OH,
Ar denotes phenyl which is unsubstituted or monosubstituted by Hal,
Het¹ denotes mono- or bicyclic heteroaryl having 2-9 C atoms and 1-3 N and/or S atoms, which may be unsubstituted or monosubstituted by Hal, A, CN or NYY,
Het² denotes a monocyclic saturated heterocycle having 3-5 C atoms and 1-2 N and/or O atoms, which may be unsubstituted or monosubstituted by A, and
Hal denotes F, Cl, Br or I,
and/or physiologically acceptable salts, tautomers and/or stereoisomers thereof, including mixtures thereof in all ratios.

3. Compounds according to Claim 1 or 2, selected from the group:
| | |
|---|---|
| 1 | 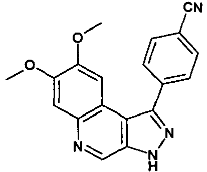 |
| 2 | 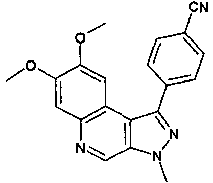 |
| 3 | 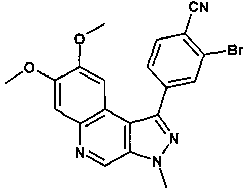 |
| 4 | 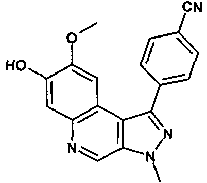 |
| 5 | 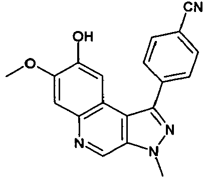 |
| 6 | 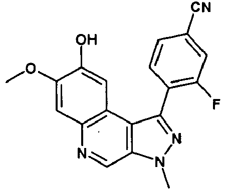 |
| 7 | 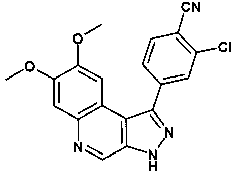 |
| 8 | 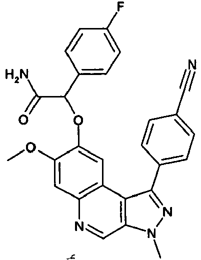 |
| 9 | 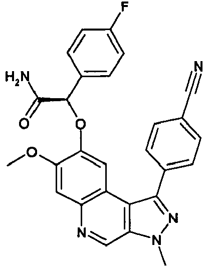 |
| 10 | 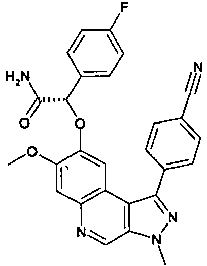 |
| 11 | 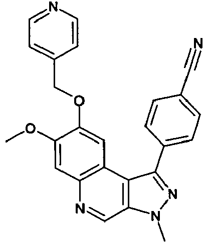 |
| 12 | 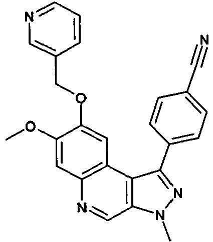 |
| 13 | 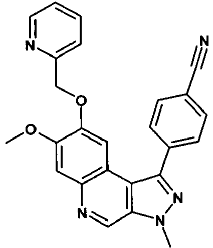 |
| 14 | 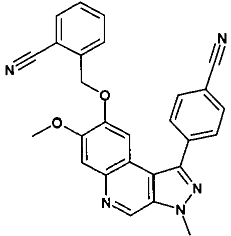 |
| 15 | 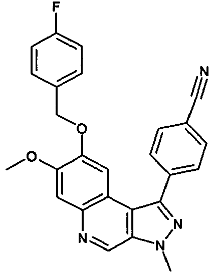 |
| 16 | 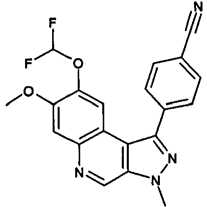 |
| 17 | 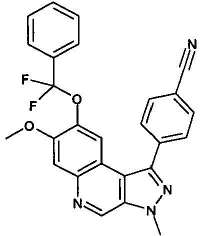 |
| 18 | 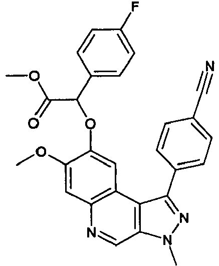 |
| 19 | 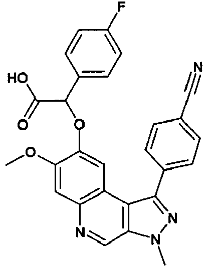 |
| 20 | 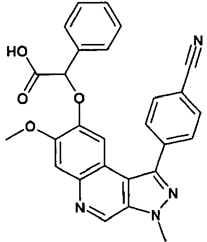 |
| 21 | 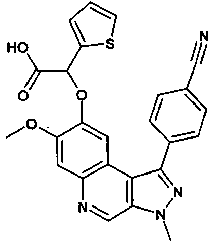 |
| 22 | 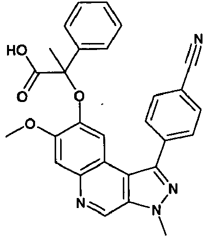 |
| 23 | 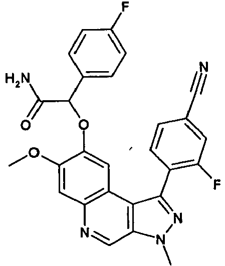 |
| 24 | 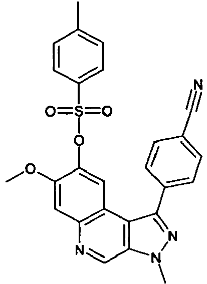 |
| 25 | 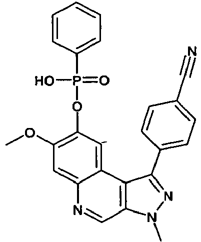 |
| 26 | 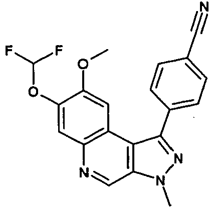 |
| 27 | 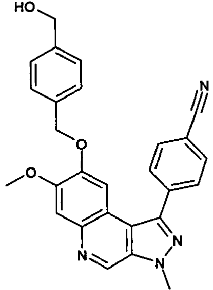 |
| 28 | 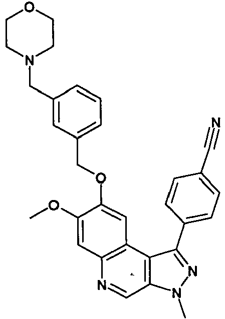 |
| 29 | 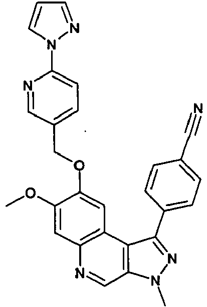 |
| 30 | 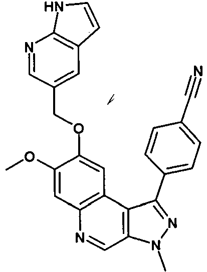 |
| 31 | 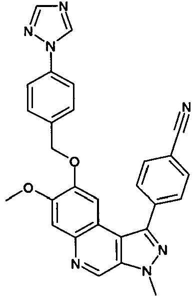 |
| 32 | 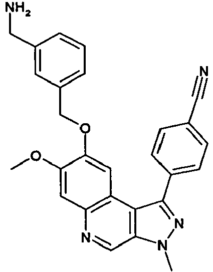 |
| 33 | 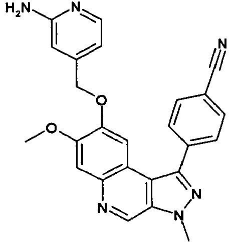 |
| 34 | 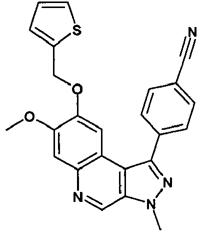 |
| 35 | 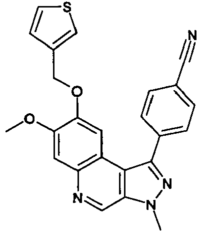 |
| 36 | 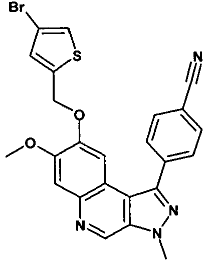 |
| 37 | 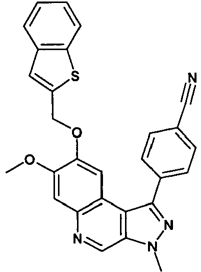 |
| 38 | 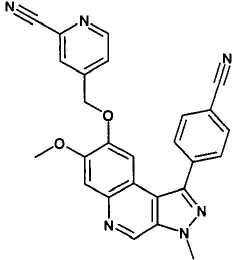 |
| 39 | 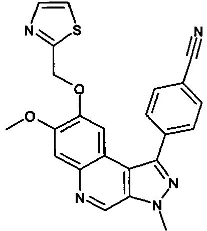 |
| 40 | 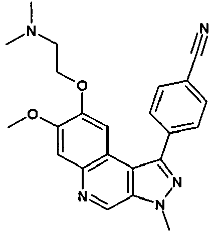 |
| 41 | 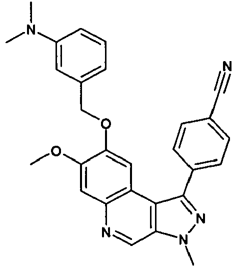 |
| 42 | 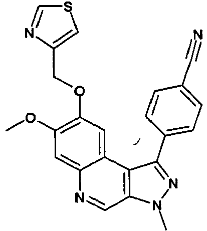 |
| 43 | 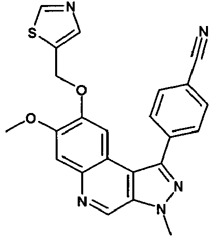 |
| 44 | 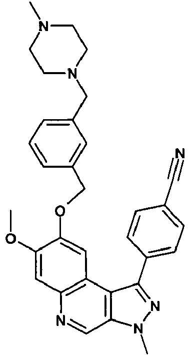 |
| 45 | 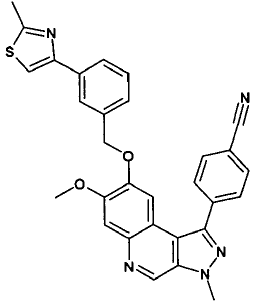 |
| 46 | 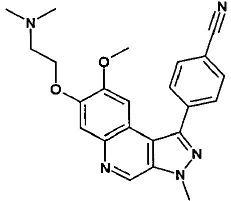 |
| 47 | 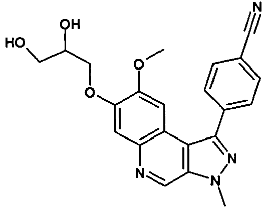 |
| 48 | 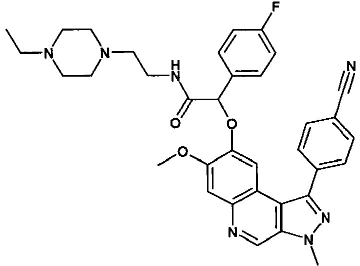 |
| 49 | 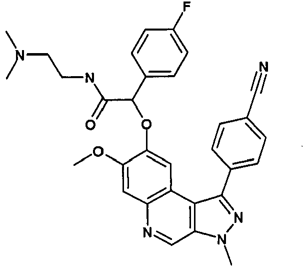 |
| 50 | 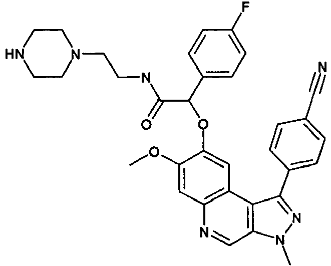 |
| 51 | 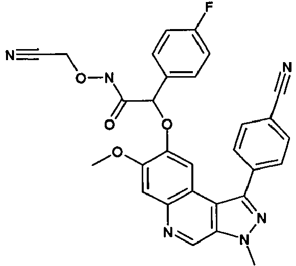 |
| 52 | 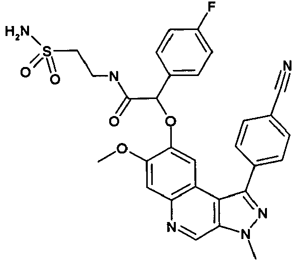 |
| 53 | 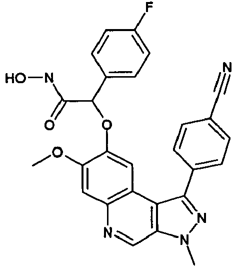 |
| 54 | 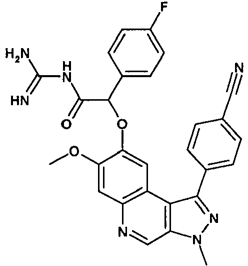 |
| 55 | 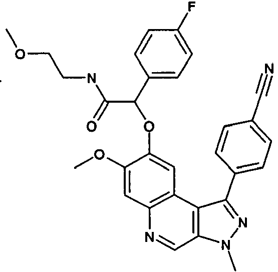 |
| 56 | 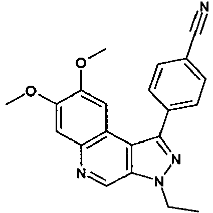 |
| 57 | 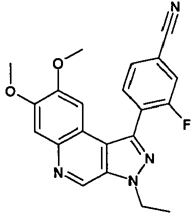 |
| 58 | 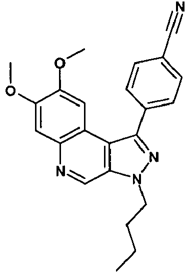 |
| 59 | 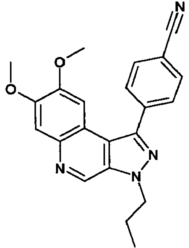 |
| 60 | 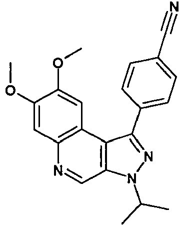 |
| 61 | 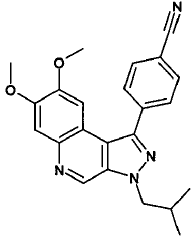 |
| 62 | 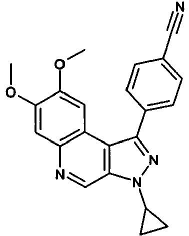 |
| 63 | 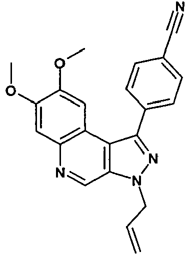 |
| 64 | 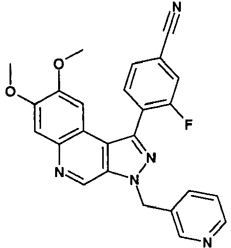 |
| 65 | 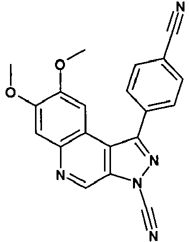 |
| 66 | 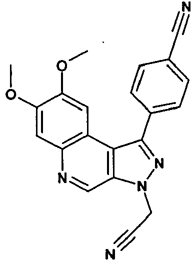 |
| 67 | 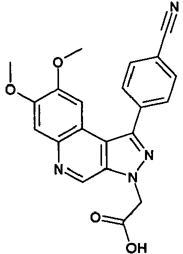 |
| 68 | 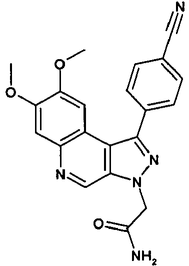 |
| 69 | 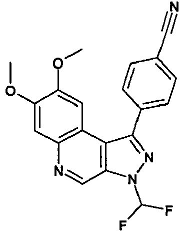 |
| 70 | 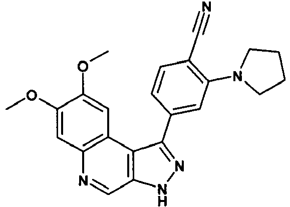 |
| 71 | 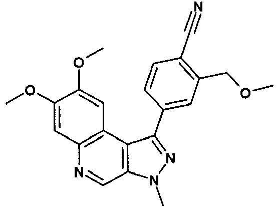 |
| 72 | 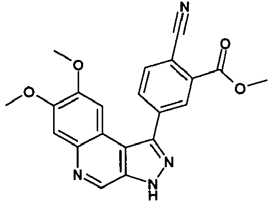 |
| 73 | 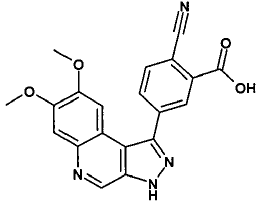 |
| 74 | 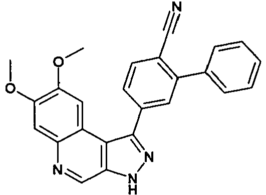 |
| 75 | 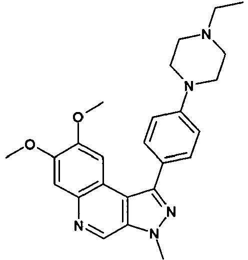 |
| 76 | 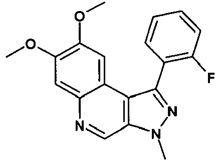 |
| 77 | 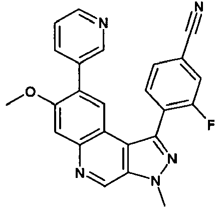 |
| 78 | 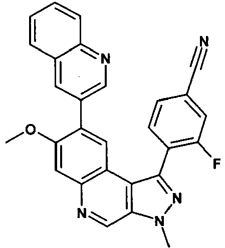 |
| 79 | 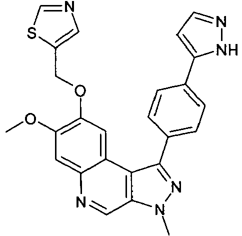 |
| 80 | 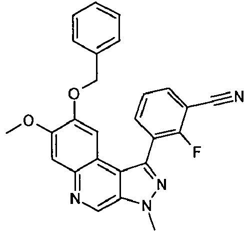 |
| 81 | 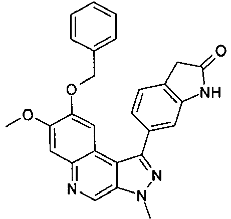 |
| 82 | 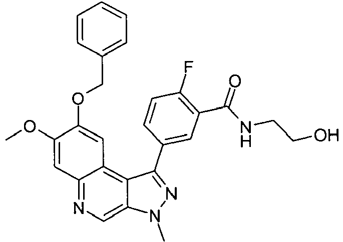 |
| 83 | 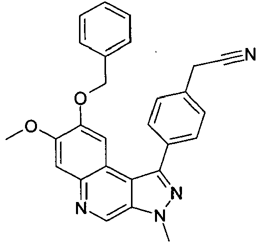 |
| 84 | 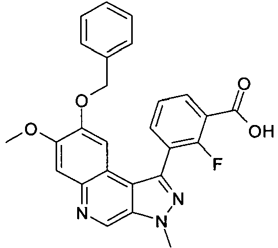 |
| 85 | 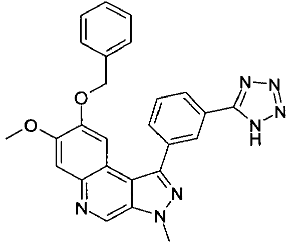 |
| 86 | 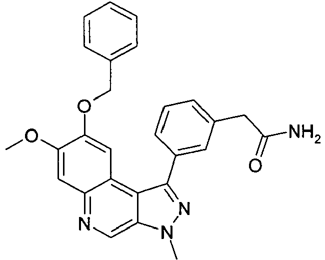 |
| 87 | 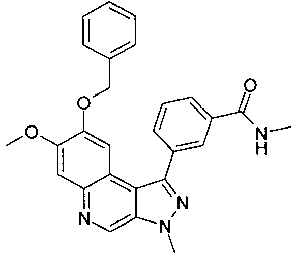 |
| 88 | 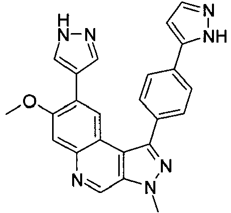 |
| 89 | 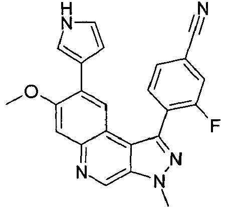 |
| 90 | 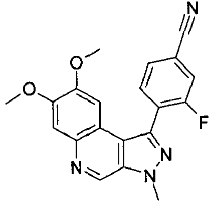 |
| 91 | 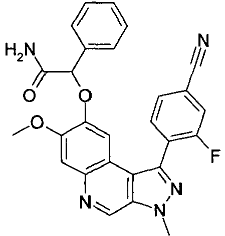 |
and/or physiologically acceptable salts, tautomers and/or stereoisomers thereof, including mixtures thereof in all ratios.

4. Intermediate compounds of the formula (II) 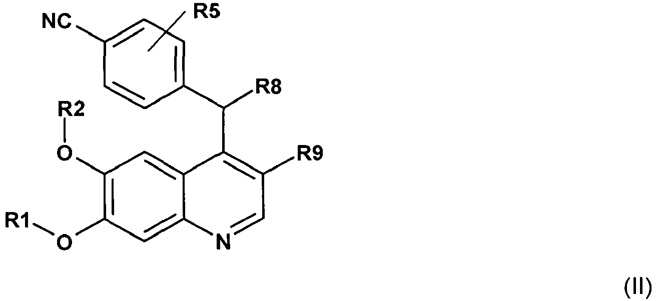 in which
R8 denotes CN or =O, and
R9 denotes NO₂ or NYY, and
R1, R2, R5 and Y have the meaning indicated in Claim 1,
and/or salts, tautomers and/or stereoisomers thereof, including mixtures thereof in all ratios.

5. Intermediate compounds according to Claim 4 having the part-formula (IIA) 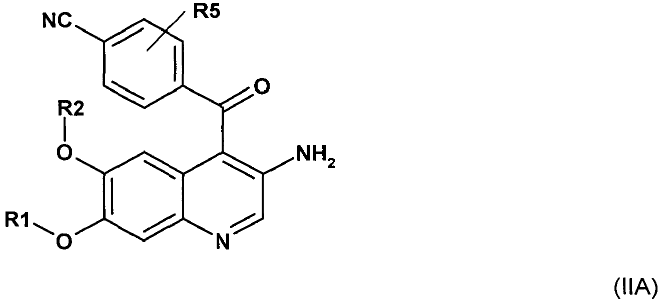 in which
R1, R2, independently of one another, denote A or -Alk-Ar, and
Alk denotes alkylene having 1-3 C atoms, where 1-2 H atoms may be replaced by Hal, and
R5, A, Ar and Hal have the meaning indicated in Claim 2,
and/or salts, tautomers and/or stereoisomers thereof, including mixtures thereof in all ratios.

6. Intermediate compounds of the formula (III) 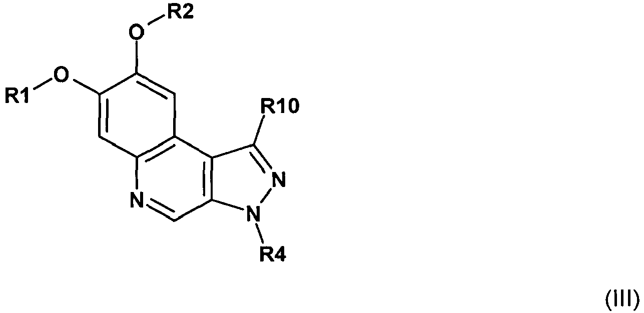 in which
R10 denotes H or Hal, and
R1, R2, R4 and Hal have the meaning indicated in Claim 1,
and/or salts, tautomers and/or stereoisomers thereof, including mixtures thereof in all ratios.

7. Intermediate compounds according to Claim 6 in which
R1, R2, independently of one another, denote A or -Alk-Ar,
R10 denotes Hal, and
Alk denotes alkylene having 1-3 C atoms, where 1-2 H atoms may be replaced by Hal, and
R4, A, Ar and Hal have the meaning indicated in Claim 2,
and/or physiologically acceptable salts, tautomers and/or stereoisomers thereof, including mixtures thereof in all ratios.

8. Process for the preparation of compounds of the formula (I) according to Claim 1, part-formulae thereof and/or physiologically acceptable salts, tautomers and/or stereoisomers thereof, including mixtures thereof in all ratios, having the following steps:
(a) reaction of a compound of the part-formula (IIA) 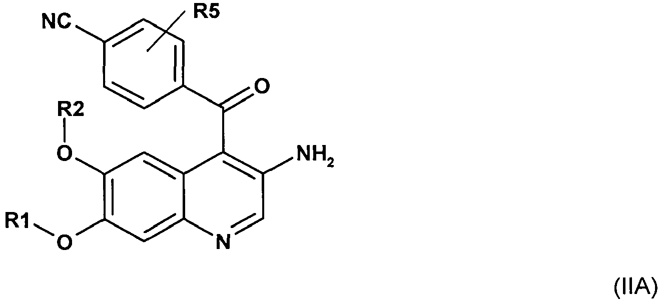 in which R1, R2 and R5 have the meaning indicated in Claim 5,
in acidic medium with a reducing agent and with a compound E-NO₂, in which E denotes an element from the 1 st main group,
to give compounds of the part-formula (IB) 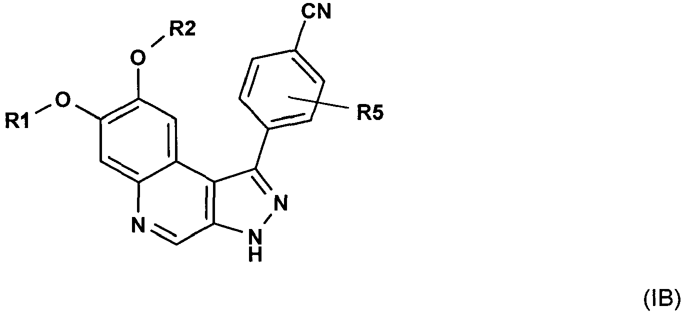 in which R1, R2 and R5 have the meaning indicated in Claim 5,
and optionally
(b') reaction of the compounds of the part-formula (IB) with a compound Hal-R4, in which R4 and Hal have the meaning indicated in Claim 1,
to give compounds of the part-formula (IC) 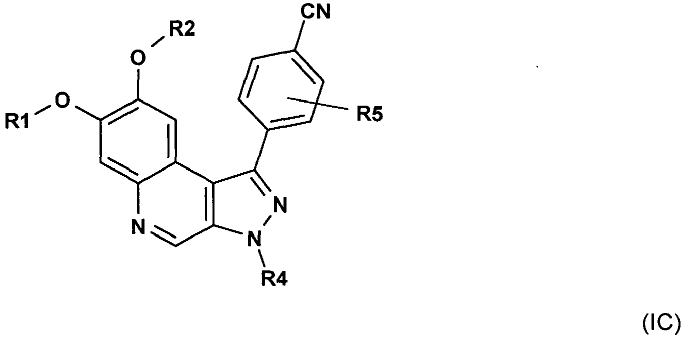 in which R1, R2 and R5 have the meaning indicated in Claim 5, and R4 has the meaning indicated in Claim 1,
(b") conversion of R1, -O-R2, R4, R5 and/or the CN group of the compounds of the part-formula (IC) to give compounds of the part-formula (IE) 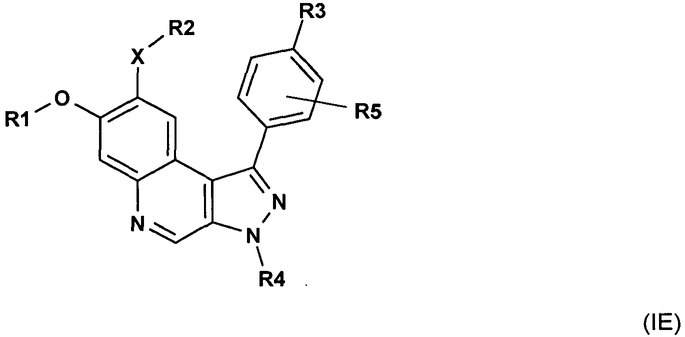 in which R1, R2, R3, R4, R5 and X have the meaning indicated in Claim 1, and/or
(b"') conversion of a base or acid of the compounds of the part-formula (IE) or part-formula (IB) or (IC) into one of its physiologically acceptable salts,
or
(a) reaction of a compound of the formula (III) 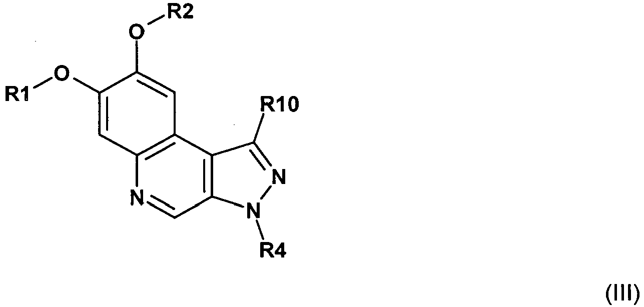 in which R1, R2, R4 and R10 have the meaning indicated in Claim 7,
with a compound of the formula (IV) 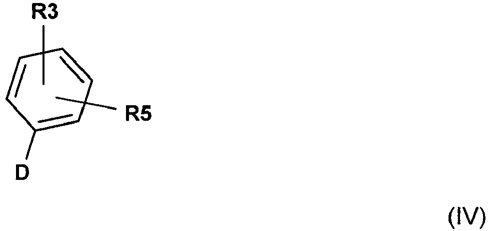 in which
D denotes boric acid, boric ester, organotin compound or boron trifluoromethanesulfonate, and
R3 and R5 have the meaning indicated in Claim 1,
to give compounds of the part-formula (ID) 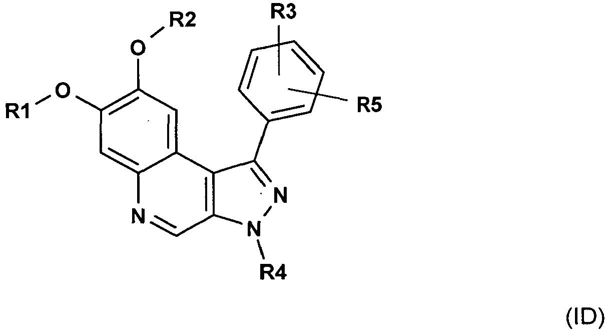 in which R1, R2 and R4 have the meaning indicated in Claim 7, and R3 and R5 have the meaning indicated in Claim 1,
and optionally
(b') conversion of R1, -O-R2, R3, R4 and/or R5 of the compounds of the part-formula (ID) to give compounds of the formula (I) 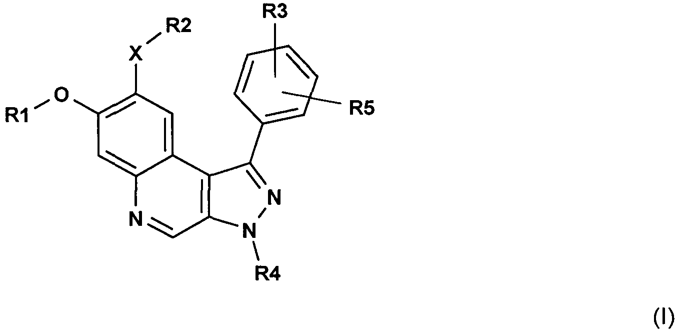 in which R1, R2, R3, R4, R5 and X have the meaning indicated in Claim 1,
and/or
(b") conversion of a base or acid of the compounds of the formula (I) or part-formula (ID) into one of its physiologically acceptable salts.

9. Process for the preparation of intermediate compounds of the formula (II) according to Claim 4 and/or salts, tautomers and/or stereoisomers thereof, including mixtures thereof in all ratios, having the following steps:
(a) reaction of a compound of the formula (V) 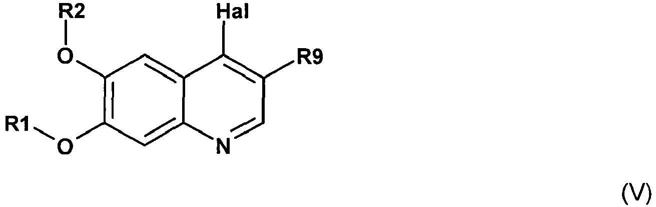 in which
Hal denotes F, Cl, Br or I, and
R1, R2 and R9 have the meaning indicated in Claim 4,
with a compound of the formula (VI) 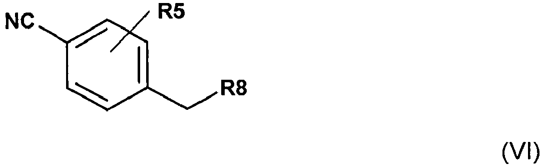 in which R5 and R8 have the meaning indicated in Claim 4,
to give compounds of the formula (II) 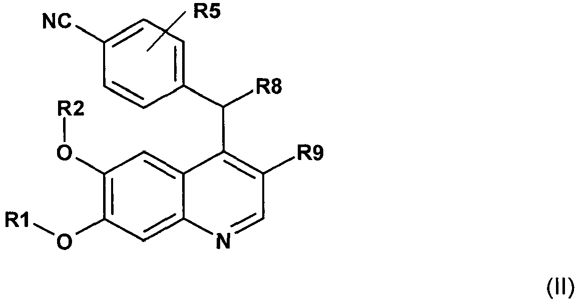 in which R1, R2, R5, R8 and R9 have the meaning indicated in Claim 4,
and optionally
(b) conversion of a base or acid of the compounds of the formula (II) into one of its salts.

10. Process for the preparation of intermediate compounds of the formula (III) according to Claim 6, part-formulae thereof and/or salts, tautomers and/or stereoisomers thereof, including mixtures thereof in all ratios, having the following steps:
(a) reaction of a compound of the formula (VII) 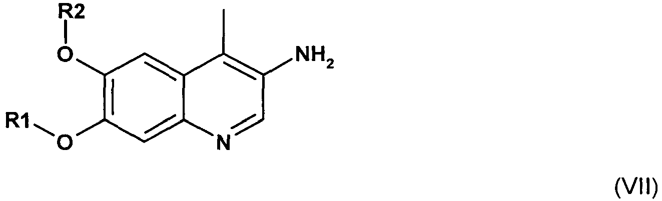 in which R1 and R2 have the meaning indicated in Claim 6,
in acidic medium with a compound E-NO₂, in which E denotes an element from the 1st main group,
to give compounds of the part-formula (IIIA) 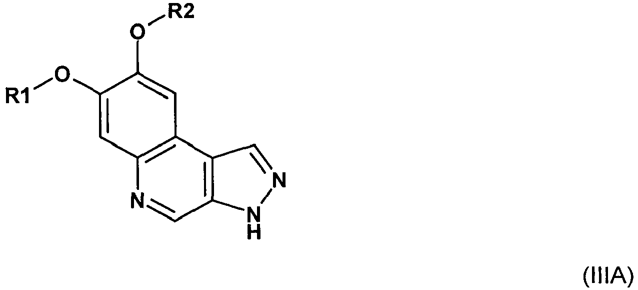 in which R1 and R2 have the meaning indicated in Claim 6,
and optionally
(b') halogenation of the compounds of the part-formula (IIIA) to give compounds of the part-formula (IIIB) 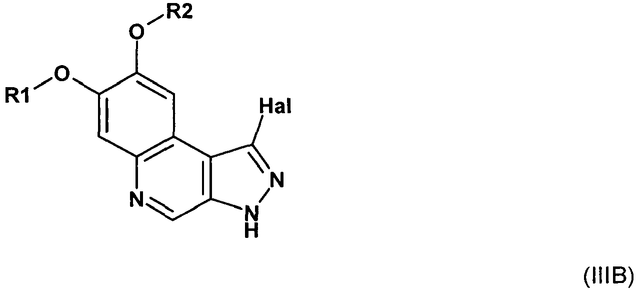 in which R1, R2 and Hal have the meaning indicated in Claim 6,
(b") reaction of the compounds of the formula (IIIA) or (IIIB) with a compound Hal-R4, in which R4 and Hal have the meaning indicated in Claim 6,
to give compounds of the formula (III) 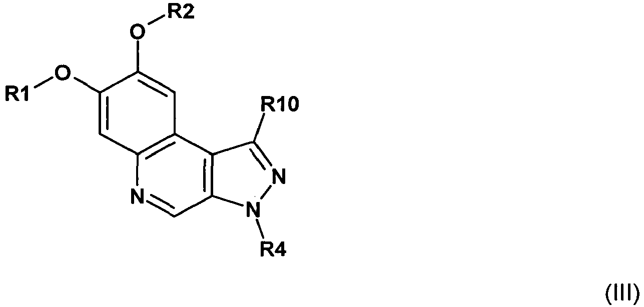 in which R1, R2, R4 and R10 have the meaning indicated in Claim 6,
and/or
(b'") conversion of a base or acid of the compounds of the formula (III) into one of its salts.

11. Use of compounds according to one of Claims 1 to 3 and/or physiologically acceptable salts, tautomers and/or stereoisomers thereof, including mixtures thereof in all ratios, for the preparation of a medicament for the inhibition of serine/threonine protein kinases, preferably PIKKs, particularly preferably DNA-PK.

12. Use of at least one compound according to one of Claims 1 to 3 and/or physiologically acceptable salts, tautomers and/or stereoisomers thereof, including mixtures thereof in all ratios, for the sensitisation of cancer cells to anticancer agents and/or ionising radiation, with the proviso that the sensitisation does not take place in vivo on the human or animal body.

13. Medicament comprising at least one compound according to one of Claims 1 to 3 and/or physiologically acceptable salts, tautomers and/or stereoisomers thereof, including mixtures thereof in all ratios.

14. Pharmaceutical composition comprising, as active compound, an effective amount of at least one compound according to one of Claims 1 to 3 and/or physiologically acceptable salts, tautomers and/or stereoisomers thereof, including mixtures thereof in all ratios, together with pharmaceutically tolerated assistants, in combination with at least one anticancer agent.

15. Compounds according to one of Claims 1 to 3 and/or physiologically acceptable salts, tautomers and/or stereoisomers thereof, including mixtures thereof in all ratios, for use in the prophylaxis, therapy and/or progress control of cancer, tumours, metastases and/or angiogenesis disorders, in combination with radiotherapy and/or with at least one anticancer agent.

## Revendications

1. Composés de formule (I) 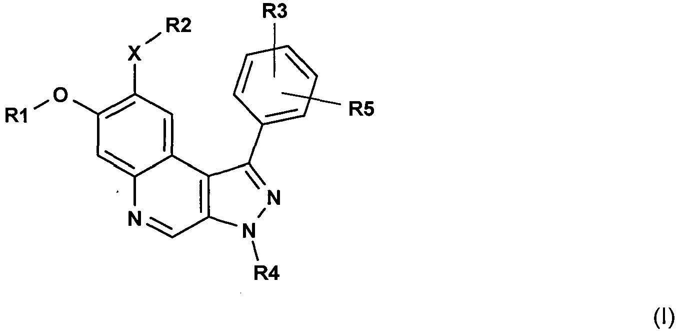 dans laquelle
R1 désigne Y, -Alk-OY, -Alk-NYY ou -Alk-Ar,
R2 désigne Y, -Alk-OY, -Alk-NYY, -C(Y)(R6)(R7), -C(Hal)(R6)(R7), -SO₂A, -SO₂-Ar ou -POOH-Ar,
R3 désigne H, Hal, CN, -Alk-CN, -Alk-NYY, Hét¹ ou Hét²,
R4 désigne Hal, Y, Cyc, CN, -Alk-CN, -Alk-COOY, -Alk-CO-NYY ou Hét¹,
R5 désigne Hal, Y, OY, NYY, -NY-COY, COOY, -CO-NYY, -CO-NY-Alk-OY, -Alk-CO-NYY, -Alk-OY, -Alk-NYY, Ar, Hét¹ ou Hét²,
R3, R5 désignent aussi ensemble -Alk-CO-NY,
R6 désigne Hal, Y, -COOY, -CO-NYY, -CO-NY-OY, -CO-NY-C(=NH)-NYY, -CO-NY-Alk-OY, -CO-NY-Alk-NYY, -CO-NY-Alk-SO₂-NYY, -CO-NY-Alk-Ar, -CO-NY-Alk-Hét² ou -CO-NY-O-Alk-CN,
R7 désigne Ar, Hét¹ ou -Hét¹-Hét¹,
X désigne CH₂, O, S ou Hét¹,
Y désigne H ou A,
A désigne alkyle non ramifié ou ramifié ayant 1-10 atomes de C, où, indépendamment les uns des autres, 1-7 atomes de H peuvent être remplacés par Hal et/ou, indépendamment les uns des autres, un ou deux groupements CH₂ adjacents peuvent être remplacés par un groupement - CH=CH- et/ou -C≡C-,
Alk désigne alkylène ayant 1-6 atomes de C, où, indépendamment les uns des autres, 1-4 atomes de H peuvent être remplacés par Hal et/ou OY,
Cyc désigne alkyle cyclique ayant 3-7 atomes de C, où, indépendamment les uns des autres, 1-4 atomes de H peuvent être remplacés par Hal et/ou OY,
Ar désigne phényle qui est non substitué ou monosubstitué par Hal, A, CN, OY, NYY, -NY-COY, COOY, Hét¹, Hét², -Alk-OY, -Alk-NYY, -Alk-Hét¹ ou Alk-Hét²,
Hét¹ désigne hétéroaryle mono- ou bicyclique ayant 2-9 atomes de C et 1-4 atomes de N, O et/ou S, qui peut être non substitué ou monosubstitué par Hal, A, CN, OY, NYY, -NY-COY, COOY, -Alk-OY ou -Alk-NYY,
Hét² désigne un hétérocycle monocyclique saturé ayant 2-7 atomes de C et 1-4 atomes de N, O et/ou S, qui peut être non substitué ou monosubstitué par A, et
Hal désigne F, Cl, Br ou I,
et/ou des sels, tautomères et/ou stéréoisomères physiologiquement acceptables de ceux-ci, y compris des mélanges de ceux-ci en toutes proportions.

2. Composés selon la revendication 1, répondant à la formule partielle (IA) 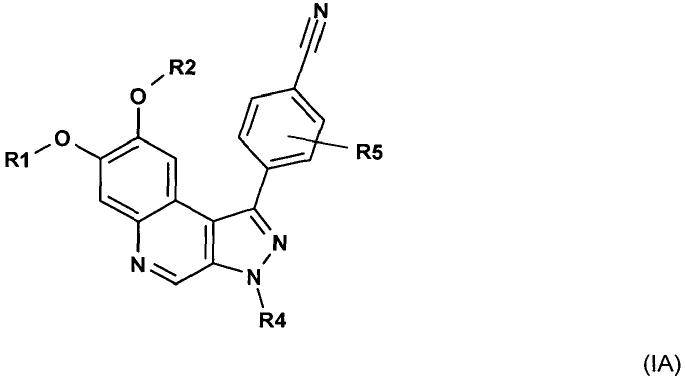 dans laquelle
R1, R4 désignent Y,
R2 désigne Y ou -CH(R6)(R7),
R5 désigne Hal, Y, COOY, Alk-OA ou Hét²,
R6 désigne -CO-NYY, -CO-NY-OY, -CO-NY-C(=NH)-NYY ou -CO-NY-Alk-OY,
R7 désigne Ar ou Hét¹,
Y désigne H ou A,
A désigne alkyle non ramifié ou ramifié ayant 1-4 atomes de C, où, indépendamment les uns des autres, 1-3 atomes de H peuvent être remplacés par Hal,
Alk désigne alkylène ayant 1-3 atomes de C, où 1-2 atomes de H peuvent être remplacés par Hal et/ou OH,
Ar désigne phényle qui est non substitué ou monosubstitué par Hal,
Hét¹ désigne hétéroaryle mono- ou bicyclique ayant 2-9 atomes de C et 1-3 atomes de N et/ou S, qui peut être non substitué ou monosubstitué par Hal, A, CN ou NYY,
Hét² désigne un hétérocycle monocyclique saturé ayant 3-5 atomes de C et 1-2 atomes de N et/ou O, qui peut être non substitué ou monosubstitué par A, et
Hal désigne F, CI, Br ou I, et/ou des sels, tautomères et/ou stéréoisomères physiologiquement acceptables de ceux-ci, y compris des mélanges de ceux-ci en toutes proportions.

3. Composés selon la revendication 1 ou 2, choisis dans le groupe constitué par :
| | |
|---|---|
| 1 | 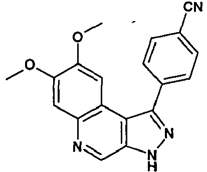 |
| 2 | 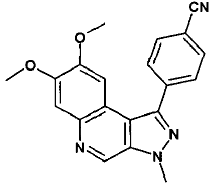 |
| 3 | 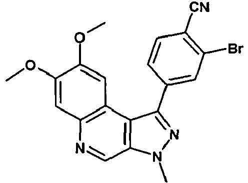 |
| 4 | 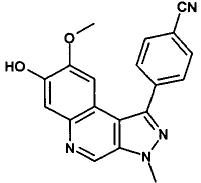 |
| 5 | 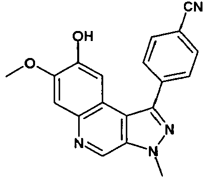 |
| 6 | 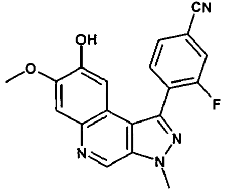 |
| 7 | 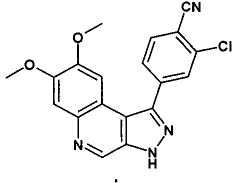 |
| 8 | 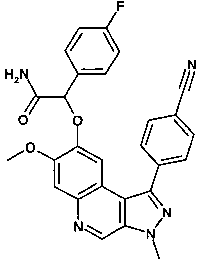 |
| 9 | 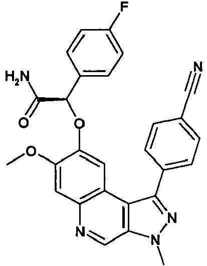 |
| 10 | 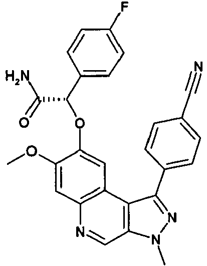 |
| 11 | 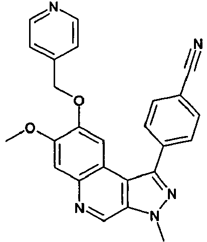 |
| 12 | 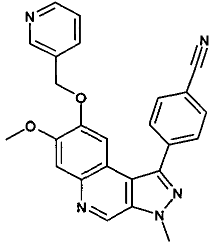 |
| 13 | 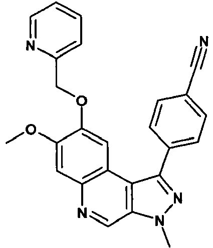 |
| 14 | 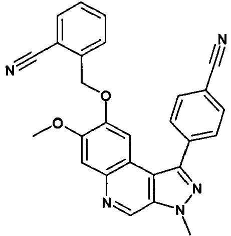 |
| 15 | 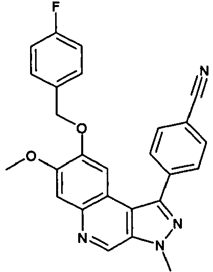 |
| 16 | 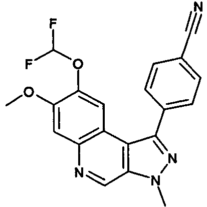 |
| 17 | 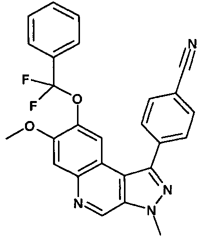 |
| 18 | 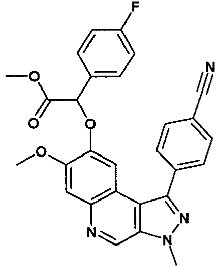 |
| 19 | 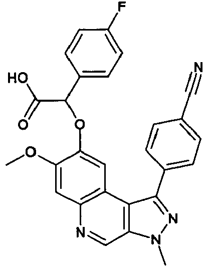 |
| 20 | 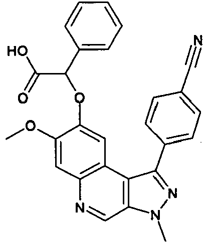 |
| 21 | 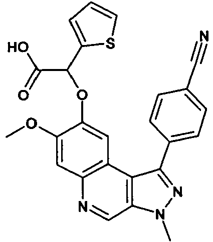 |
| 22 | 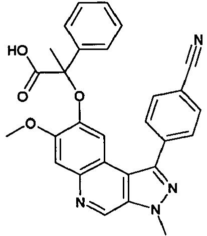 |
| 23 | 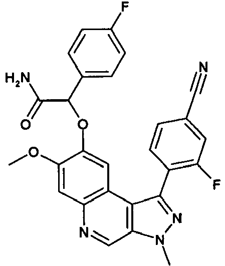 |
| 24 | 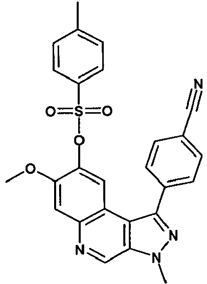 |
| 25 | 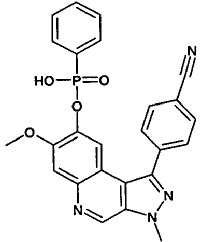 |
| 26 | 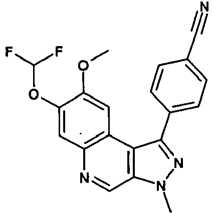 |
| 27 | 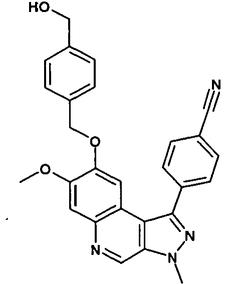 |
| 28 | 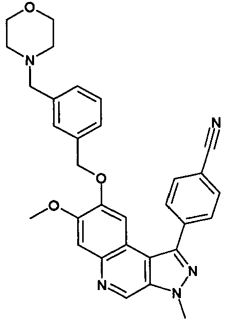 |
| 29 | 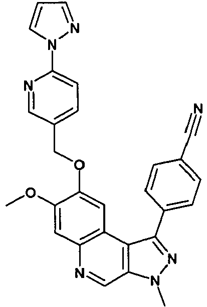 |
| 30 | 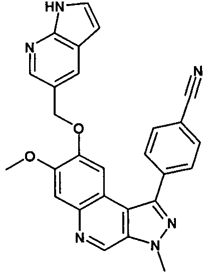 |
| 31 | 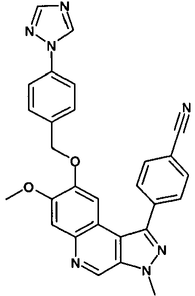 |
| 32 | 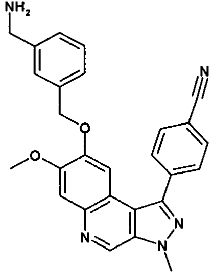 |
| 33 | 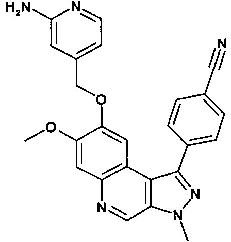 |
| 34 | 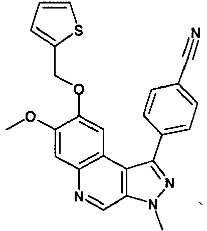 |
| 35 | 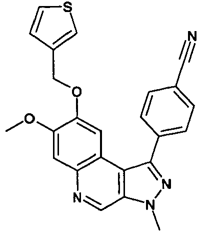 |
| 36 | 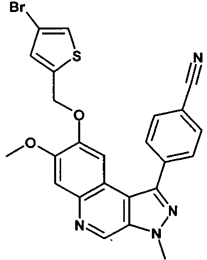 |
| 37 | 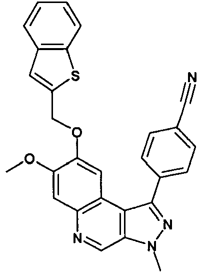 |
| 38 | 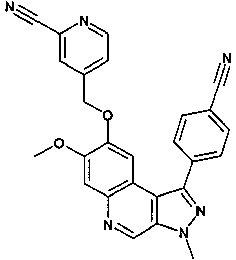 |
| 39 | 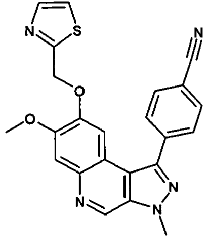 |
| 40 | 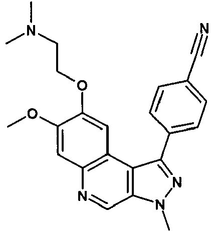 |
| 41 | 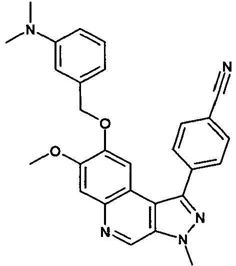 |
| 42 | 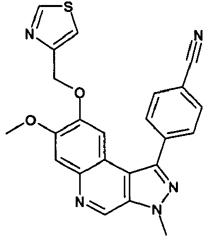 |
| 43 | 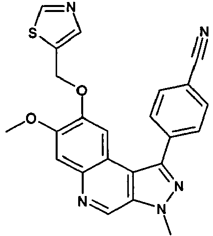 |
| 44 | 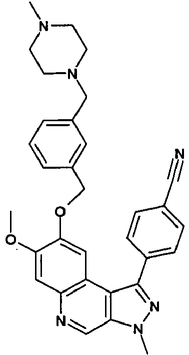 |
| 45 | 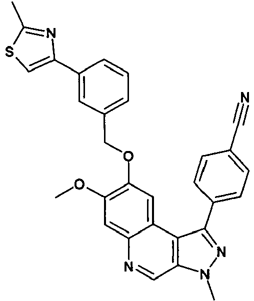 |
| 46 | 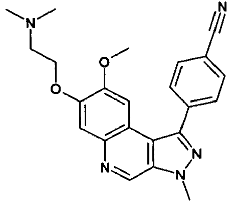 |
| 47 | 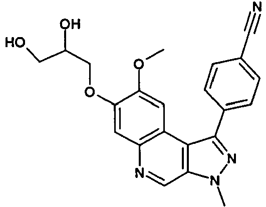 |
| 48 | 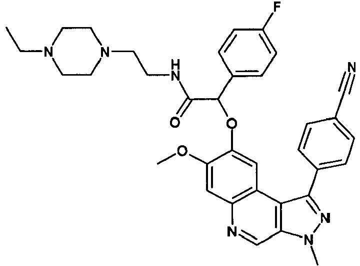 |
| 49 | 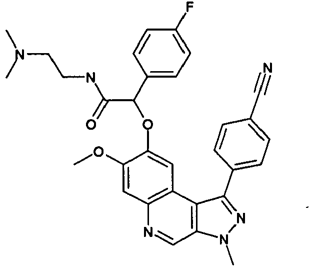 |
| 50 | 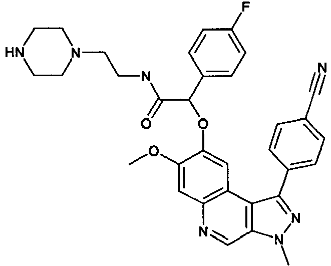 |
| 51 | 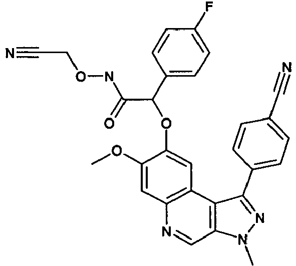 |
| 52 | 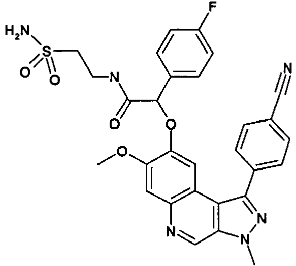 |
| 53 | 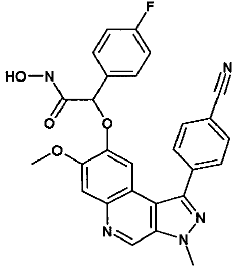 |
| 54 | 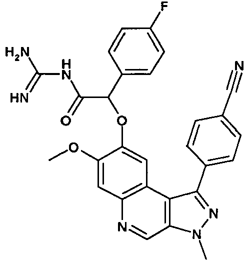 |
| 55 | 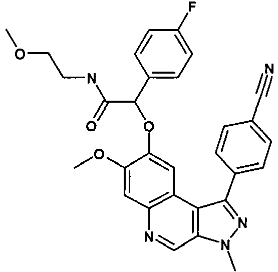 |
| 56 | 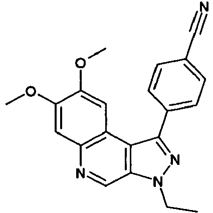 |
| 57 | 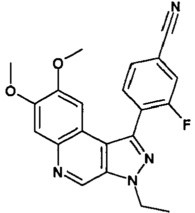 |
| 58 | 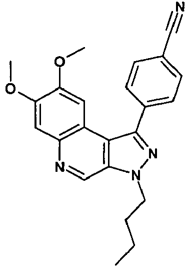 |
| 59 | 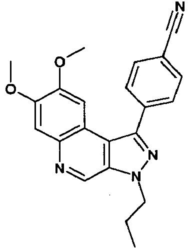 |
| 60 | 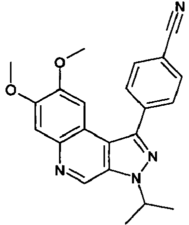 |
| 61 | 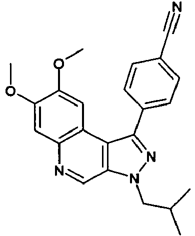 |
| 62 | 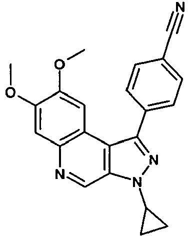 |
| 63 | 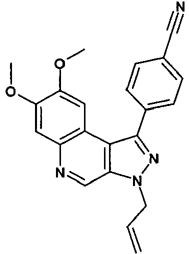 |
| 64 | 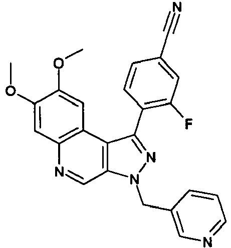 |
| 65 | 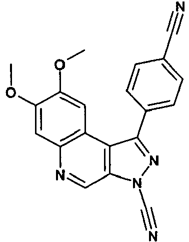 |
| 66 | 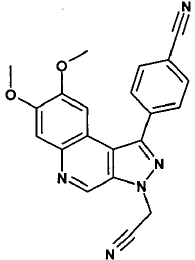 |
| 67 | 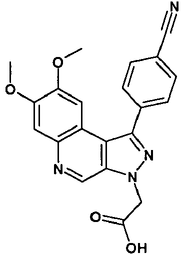 |
| 68 | 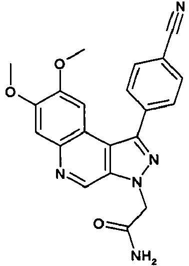 |
| 69 | 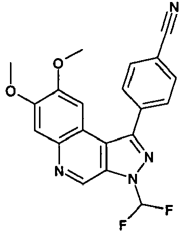 |
| 70 | 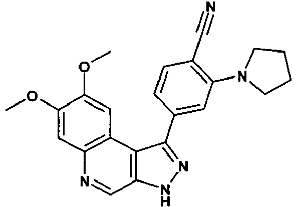 |
| 71 | 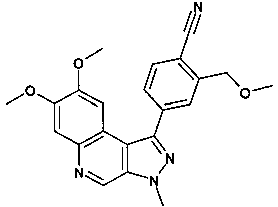 |
| 72 | 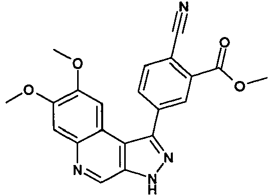 |
| 73 | 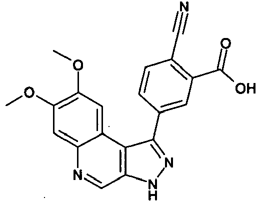 |
| 74 | 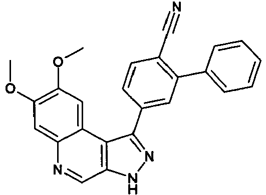 |
| 75 | 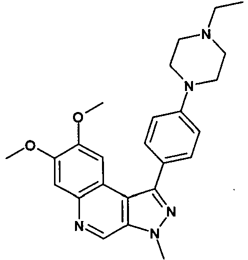 |
| 76 | 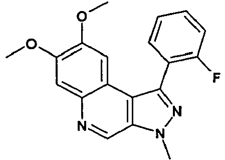 |
| 77 | 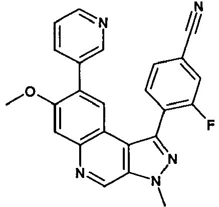 |
| 78 | 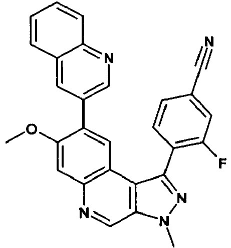 |
| 79 | 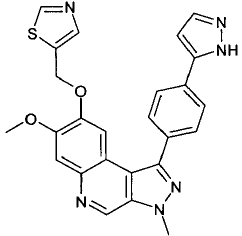 |
| 80 | 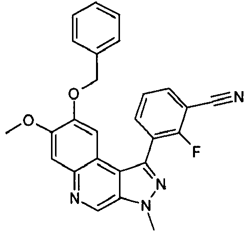 |
| 81 | 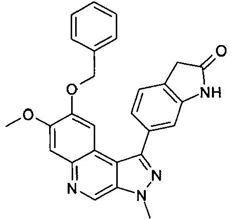 |
| 82 | 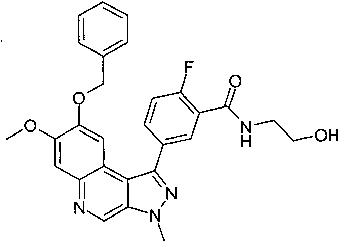 |
| 83 | 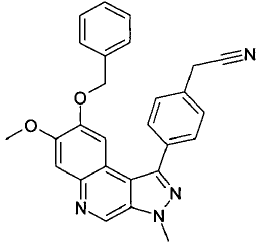 |
| 84 | 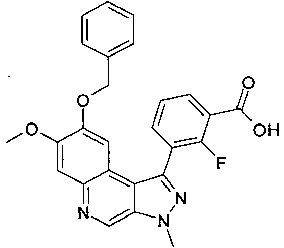 |
| 85 | 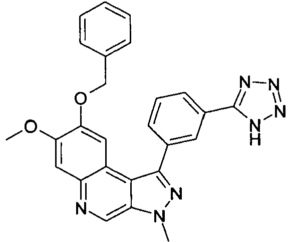 |
| 86 | 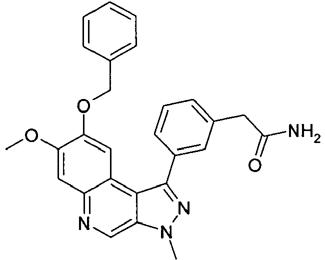 |
| 87 | 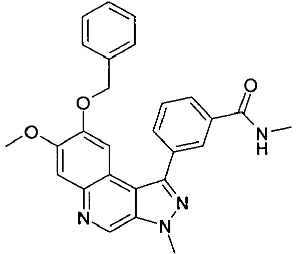 |
| 88 | 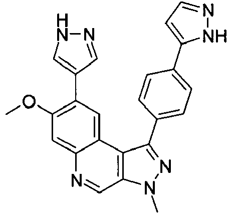 |
| 89 | 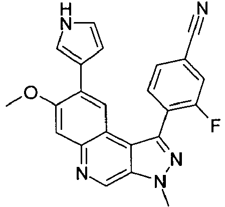 |
| 90 | 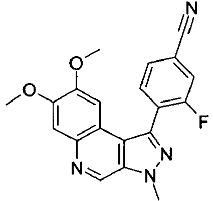 |
| 91 | 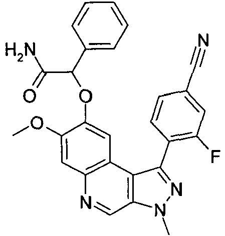 |
et/ou des sels, tautomères et/ou stéréoisomères physiologiquement acceptables de ceux-ci, y compris des mélanges de ceux-ci en toutes proportions.

4. Composés intermédiaires de formule (II) 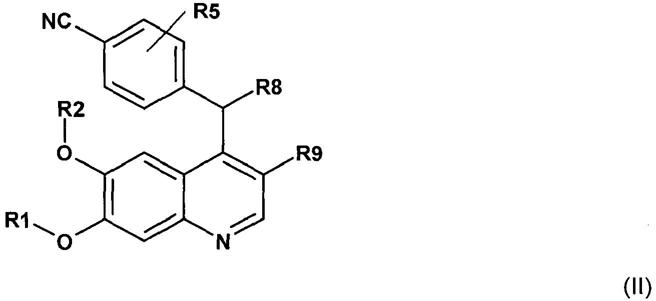 dans laquelle
R8 désigne CN ou =O, et
R9 désigne NO₂ ou NYY, et
R1, R2, R5 et Y revêtent la signification indiquée selon la revendication 1,
et/ou des sels, tautomères et/ou stéréoisomères de ceux-ci, y compris des mélanges de ceux-ci en toutes proportions.

5. Composés intermédiaires selon la revendication 4, répondant à la formule partielle (IIA) 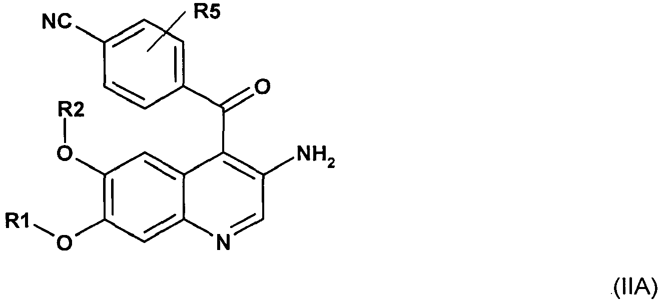 dans laquelle
R1, R2, indépendamment l'un de l'autre, désignent A ou -Alk-Ar, et
Alk désigne alkylène ayant 1-3 atomes de C, où 1-2 atomes de H peuvent être remplacés par Hal, et
R5, A, Ar et Hal revêtent la signification indiquée selon la revendication 2,
et/ou des sels, tautomères et/ou stéréoisomères de ceux-ci, y compris des mélanges de ceux-ci en toutes proportions.

6. Composés intermédiaires de formule (III) 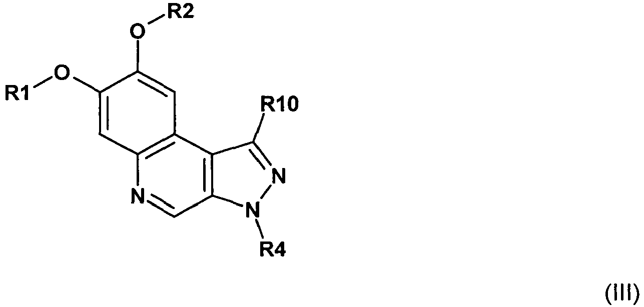 dans laquelle
R10 désigne H ou Hal, et
R1, R2, R4 et Hal revêtent la signification indiquée selon la revendication 1,
et/ou des sels, tautomères et/ou stéréoisomères de ceux-ci, y compris des mélanges de ceux-ci en toutes proportions.

7. Composés intermédiaires selon la revendication 6, dans lesquels
R1, R2, indépendamment l'un de l'autre, désignent A ou -Alk-Ar,
R10 désigne Hal, et
Alk désigne alkylène ayant 1-3 atomes de C, où 1-2 atomes de H peuvent être remplacés par Hal, et
R4, A, Ar et Hal revêtent la signification indiquée selon la revendication 2,
et/ou des sels, tautomères et/ou stéréoisomères physiologiquement acceptables de ceux-ci, y compris des mélanges de ceux-ci en toutes proportions.

8. Procédé de préparation de composés de formule (I) selon la revendication 1, de formules partielles de ceux-ci et/ou de sels, tautomères et/ou stéréoisomères physiologiquement acceptables de ceux-ci, y compris des mélanges de ceux-ci en toutes proportions, comportant les étapes suivantes :
(a) la réaction d'un composé de formule partielle (IIA) 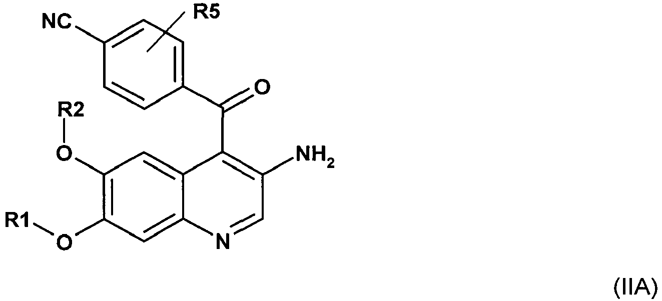 dans laquelle R1, R2 et R5 revêtent la signification indiquée selon la revendication 5,
en milieu acide avec un agent réducteur et avec un composé E-NO₂, où E désigne un élément issu du 1 er groupe principal,
pour donner les composés de formule partielle (IB) 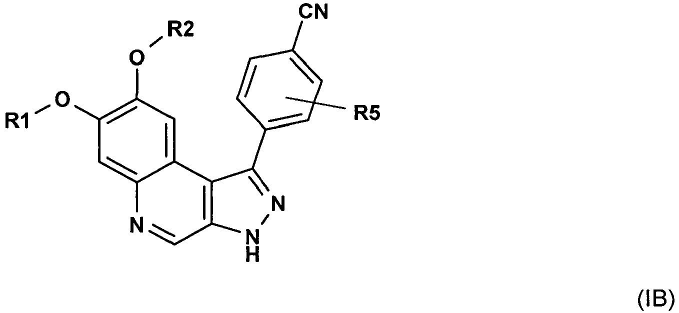 dans laquelle R1, R2 et R5 revêtent la signification indiquée selon la revendication 5,
et éventuellement
(b') la réaction des composés de formule partielle (IB) avec un composé Hal-R4, où R4 et Hal revêtent la signification indiquée selon la revendication 1,
pour donner les composés de formule partielle (IC) 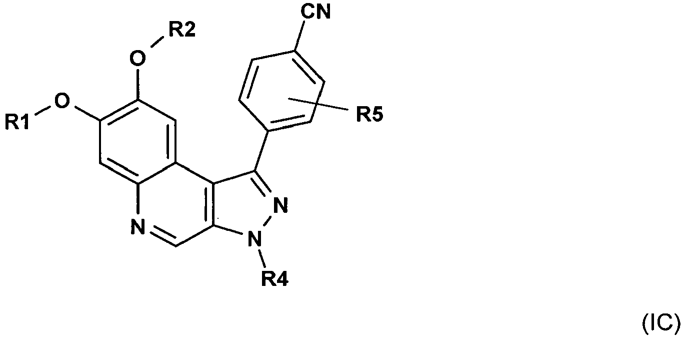 dans laquelle R1, R2 et R5 revêtent la signification indiquée selon la revendication 5, et R4 revêt la signification indiquée selon la revendication 1,
(b") la conversion de R1, -O-R2, R4, R5 et/ou du groupement CN des composés de formule partielle (IC) pour donner les composés de formule partielle (IE) 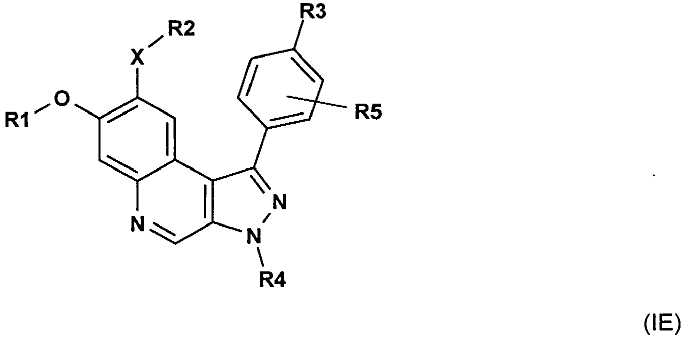 dans laquelle R1, R2, R3, R4, R5 et X revêtent la signification indiquée selon la revendication 1,
et/ou
(b"') la conversion d'une base ou d'un acide des composés de formule partielle (IE) ou de formule partielle (IB) ou (IC) en l'un de ses sels physiologiquement acceptables,
ou
(a) la réaction d'un composé de formule (III) 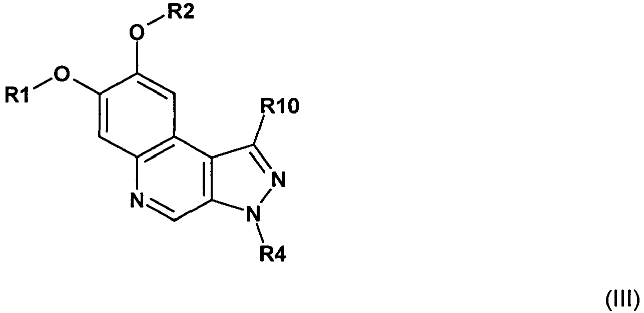 dans laquelle R1, R2, R4 et R10 revêtent la signification indiquée selon la revendication 7,
avec un composé de formule (IV) 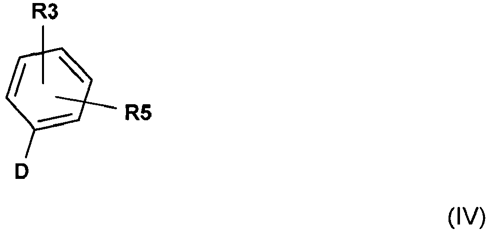 dans laquelle
D désigne l'acide borique, un ester borique, un composé organostannique ou le trifluorométhanesulfonate de bore, et
R3 et R5 revêtent la signification indiquée selon la revendication 1,
pour donner les composés de formule partielle (ID) 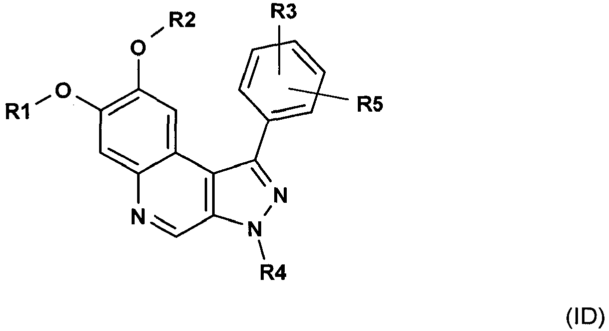 dans laquelle R1, R2 et R4 revêtent la signification indiquée selon la revendication 7,
et R3 et R5 revêtent la signification indiquée selon la revendication 1,
et éventuellement
(b') la conversion de R1, -O-R2, R3, R4 et/ou R5 des composés de formule partielle (ID) pour donner les composés de formule (I) 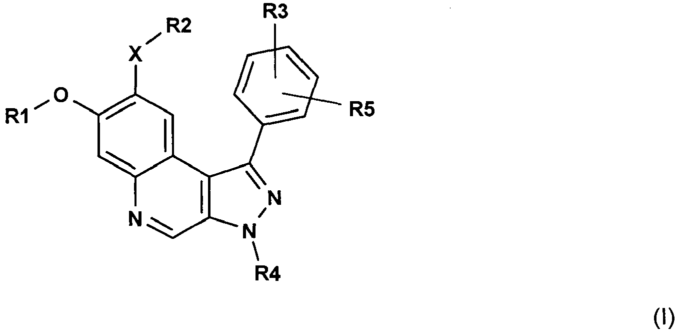 dans laquelle R1, R2, R3, R4, R5 et X revêtent la signification indiquée selon la revendication 1,
et/ou
(b") la conversion d'une base ou d'un acide des composés de formule (I) ou de formule partielle (ID) en l'un de ses sels physiologiquement acceptables.

9. Procédé de préparation de composés intermédiaires de formule (II) selon la revendication 4 et/ou de sels, tautomères et/ou stéréoisomères de ceux-ci, y compris des mélanges de ceux-ci en toutes proportions, comportant les étapes suivantes :
(a) la réaction d'un composé de formule (V) 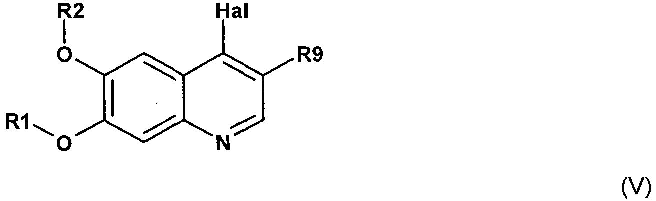 dans laquelle
Hal désigne F, Cl, Br ou I, et
R1, R2 et R9 revêtent la signification indiquée selon la revendication 4,
avec un composé de formule (VI) 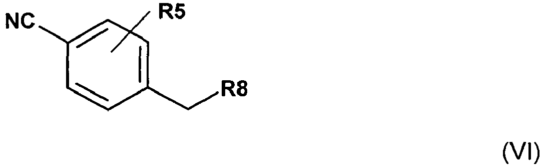 dans laquelle R5 et R8 revêtent la signification indiquée selon la revendication 4,
pour donner les composés de formule (II) 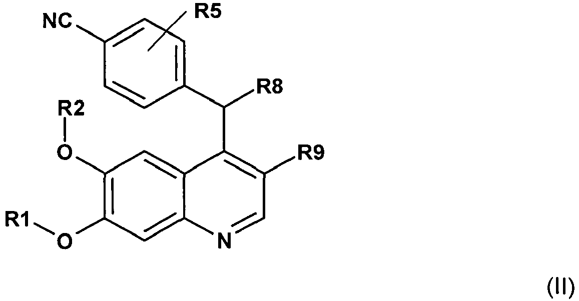 dans laquelle R1, R2, R5, R8 et R9 revêtent la signification indiquée selon la revendication 4,
et éventuellement
(b) la conversion d'une base ou d'un acide des composés de formule (II) en l'un de ses sels.

10. Procédé de préparation de composés intermédiaires de formule (III) selon la revendication 6, de formules partielles de ceux-ci et/ou de sels, tautomères et/ou stéréoisomères de ceux-ci, y compris des mélanges de ceux-ci en toutes proportions, comportant les étapes suivantes :
(a) la réaction d'un composé de formule (VII) 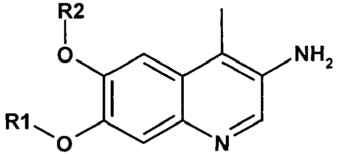 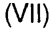 dans laquelle R1 et R2 revêtent la signification indiquée selon la revendication 6,
en milieu acide avec un composé E-NO₂, où E désigne un élément issu du 1 er groupe principal,
pour donner les composés de formule partielle (IIIA) 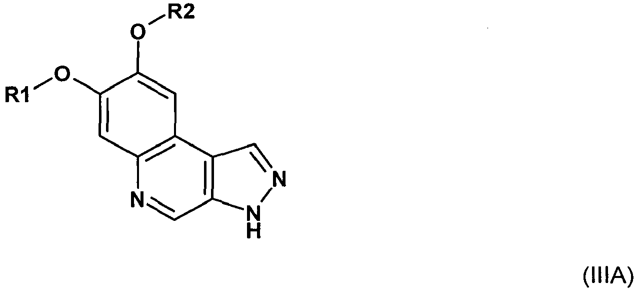 dans laquelle R1 et R2 revêtent la signification indiquée selon la revendication 6,
et éventuellement
(b') l'halogénation des composés de formule partielle (IIIA) pour donner les composés de formule partielle (IIIB) 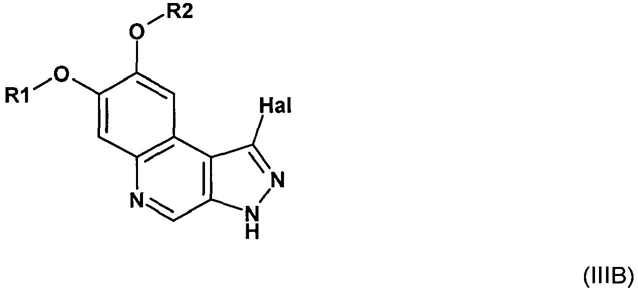 dans laquelle R1, R2 et Hal revêtent la signification indiquée selon la revendication 6,
(b") la réaction des composés de formule (IIIA) ou (IIIB) avec un composé Hal-R4, où R4 et Hal revêtent la signification indiquée selon la revendication 6, pour donner les composés de formule (III) 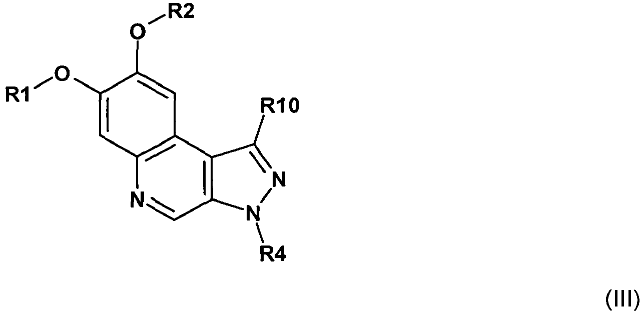 dans laquelle R1, R2, R4 et R10 revêtent la signification indiquée selon la revendication 6,
et/ou
(b"') la conversion d'une base ou d'un acide des composés de formule (III) en l'un de ses sels.

11. Utilisation de composés selon l'une des revendications 1 à 3 et/ou de sels, tautomères et/ou stéréoisomères physiologiquement acceptables de ceux-ci, y compris des mélanges de ceux-ci en toutes proportions, pour la préparation d'un médicament destiné à l'inhibition de sérine/thréonine protéine kinases, préférablement de PIKK, particulièrement préférablement d'ADN-PK.

12. Utilisation d'au moins un composé selon l'une des revendications 1 à 3 et/ou de sels, tautomères et/ou stéréoisomères physiologiquement acceptables de celui-ci, y compris des mélanges de ceux-ci en toutes proportions, pour la sensibilisation de cellules cancéreuses vis-à-vis d'agents anticancéreux et/ou d'un rayonnement ionisant, à condition que la sensibilisation n'ait pas lieu *in vivo* sur le corps humain ou animal.

13. Médicament comprenant au moins un composé selon l'une des revendications 1 à 3 et/ou des sels, tautomères et/ou stéréoisomères physiologiquement acceptables de celui-ci, y compris des mélanges de ceux-ci en toutes proportions.

14. Composition pharmaceutique comprenant, comme composé actif, une quantité efficace d'au moins un composé selon l'une des revendications 1 à 3 et/ou de sels, tautomères et/ou stéréoisomères physiologiquement acceptables de celui-ci, y compris des mélanges de ceux-ci en toutes proportions, conjointement avec des auxiliaires pharmaceutiquement tolérés, en combinaison avec au moins un agent anticancéreux.

15. Composés selon l'une des revendications 1 à 3 et/ou des sels, tautomères et/ou stéréoisomères physiologiquement acceptables de ceux-ci, y compris des mélanges de ceux-ci en toutes proportions, pour une utilisation dans la prophylaxie, la thérapie et/ou le contrôle de la progression du cancer, des tumeurs, des métastases et/ou des troubles d'angiogenèse, en combinaison avec une radiothérapie et/ou avec au moins un agent anticancéreux.
